# EUROPEAN PATENT APPLICATION

(11) **EP 4 741 398 A1**
(43) Date of publication of application: **13.05.2026**
(21) Application number: 24839453.8
(22) Date of filing: 21.06.2024
(51) Int. Cl.: C07D 491/22, C07D 495/22, C09K 11/06, H10K 50/12, H10K 59/10, H10K 59/60, H10K 59/95, H10K 85/60, H10K 101/30, H10K 101/40

(54) **ORGANIC COMPOUND AND ORGANIC LIGHT-EMITTING ELEMENT**

(30) Priority: 07.07.2023 JP 2023112256; 23.05.2024 JP 2024083867
(71) Applicant: Canon Kabushiki Kaisha, Tokyo, 146-8501 (JP)
(72) Inventor: SEKIGUCHI, Takeshi, Tokyo 146-8501 (JP); TANAKA, Akane, Tokyo 146-8501 (JP)
(74) Representative: TBK
(86) International application number: PCT/JP2024/022485
(87) International publication number: WO 2025/013557

(57) **Abstract**

The present disclosure provides an organic compound represented by general formula (1-1) or (1-2).

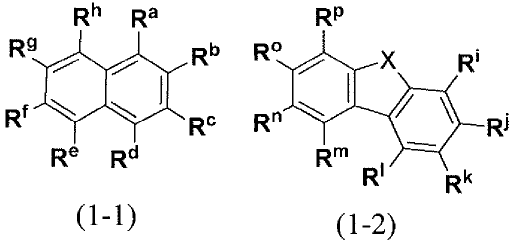

In general formulas (1-1) and (1-2), at least four of bonding sites R^{a} to R^{h} and at least four of bonding sites Rⁱ to R^{p} are bonded to a skeleton selected from Structure A group. Among the bonding sites R^{a} to R^{p}, bonding sites not bonded to Structure A group are each independently selected from a hydrogen atom or a substituent.

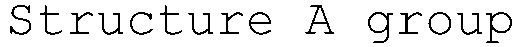

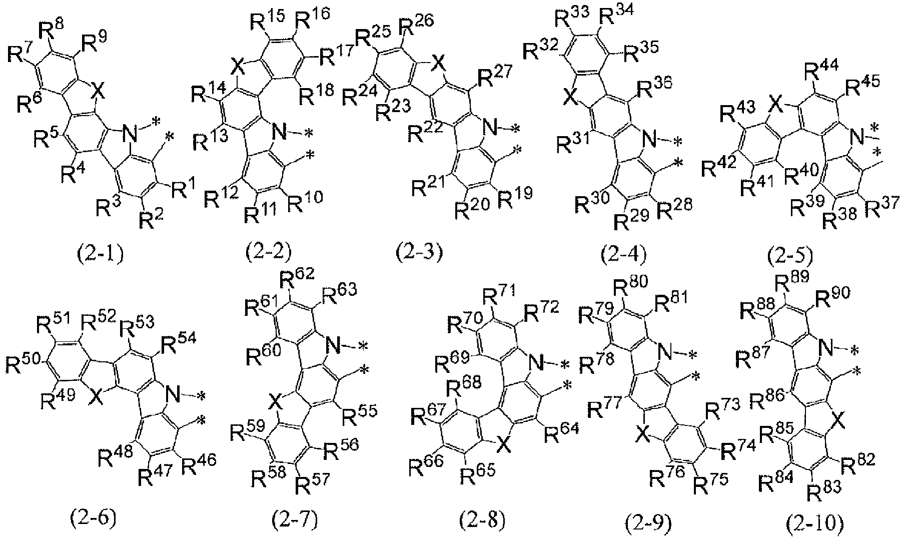

In general formulas (2-1) to (2-10), R¹ to R⁹⁰ are each independently selected from a hydrogen atom, a deuterium atom, and various substituents. X is an oxygen atom, a sulfur atom, NR⁹¹, CR⁹²R⁹³, or SiR⁹⁴R⁹⁵. R⁹¹ to R⁹⁵ are each independently selected from a hydrogen atom or a substituent. Bonding positions * represent positions of bonding to the bonding sites R^{a} to R^{p}.

## Description

### Technical Field

The present invention relates to an organic compound and an organic light-emitting element including the organic compound.

### Background Art

An organic light-emitting element (hereinafter also referred to as an "organic electroluminescent element" or an "organic EL element") is an electronic element including a pair of electrodes and an organic compound layer disposed between the electrodes. By injecting electrons and holes into the organic compound layer through the pair of electrodes, excitons of a luminescent organic compound in the organic compound layer are generated, and the organic light-emitting element emits light when the excitons return to their ground state.

Compounds suitable for organic light-emitting elements have been actively created to date. PTL 1 discloses compound Ref-1 and compound Ref-2 as compounds having an indolocarbazole skeleton.

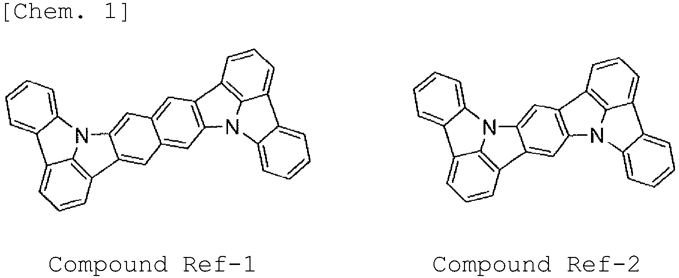

### Citation List

### Patent Literature

PTL 1: International Publication No. 2019/111971

### Summary of Invention

### Technical Problem

However, the above compounds each have room for improvement in terms of thermal stability.

The present invention has been made in view of the above problem, and an object thereof is to provide an organic compound having high thermal stability.

### Solution to Problem

An organic compound according to the present invention is represented by general formula (1-1) or (1-2).

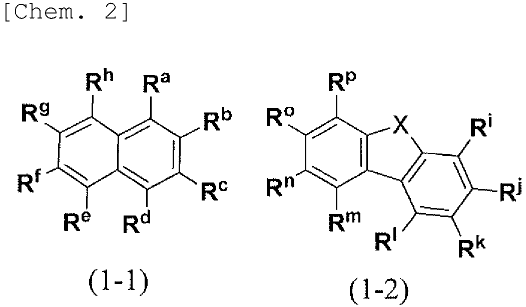

In general formula (1-1), at least four of bonding sites R^{a} to R^{h} are bonded to a skeleton selected from Structure A group. Among the bonding sites R^{a} to R^{h}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group.

In general formula (1-2), at least four of bonding sites Rⁱ to R^{p} are bonded to a skeleton selected from Structure A group. Among the bonding sites Rⁱ to R^{p}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group.

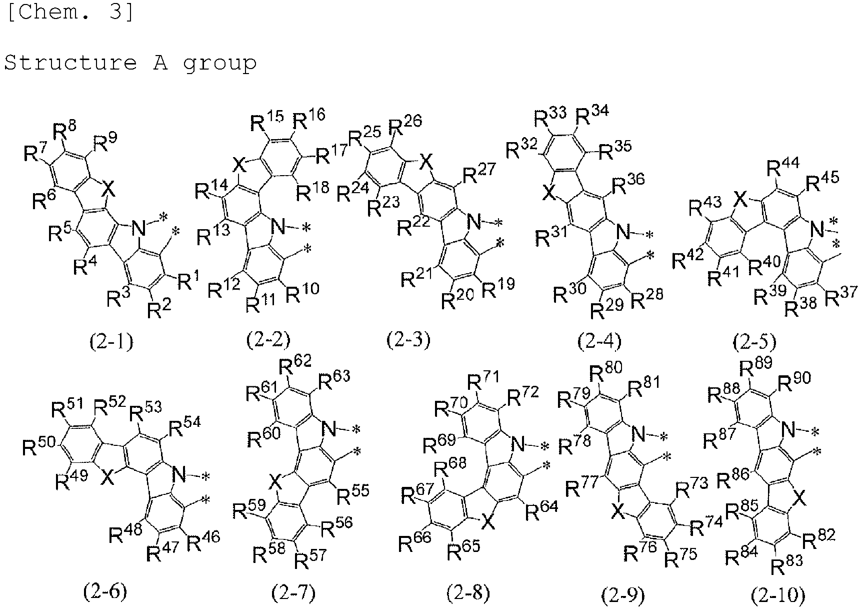

In general formulas (2-1) to (2-10), R¹ to R⁹⁰ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group.

X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group. Bonding positions * represent positions of bonding to the bonding sites R^{a} to R^{p}.

### Advantageous Effects of Invention

According to the present invention, an organic compound having high thermal stability can be provided.

### Brief Description of Drawings

[Fig. 1A] Fig. 1A is a schematic sectional view showing an example of a pixel of a display apparatus according to an embodiment of the present invention.
[Fig. 1B] Fig. 1B is a schematic sectional view of an example of a display apparatus including an organic EL element according to an embodiment of the present invention.
[Fig. 2] Fig. 2 is a schematic view showing an example of a display apparatus according to an embodiment of the present invention.
[Fig. 3A] Fig. 3A is a schematic view showing an example of an image pickup apparatus according to an embodiment of the present invention.
[Fig. 3B] Fig. 3B is a schematic view showing an example of an electronic apparatus according to an embodiment of the present invention.
[Fig. 4A] Fig. 4A is a schematic view showing an example of a display apparatus according to an embodiment of the present invention.
[Fig. 4B] Fig. 4B is a schematic view showing an example of a foldable display apparatus.
[Fig. 5A] Fig. 5A is a schematic view showing an example of a lighting apparatus according to an embodiment of the present invention.
[Fig. 5B] Fig. 5B is a schematic view showing an example of an automobile including a vehicle lighting fixture according to an embodiment of the present invention.
[Fig. 6A] Fig. 6A is a schematic view showing an example of a wearable device according to an embodiment of the present invention.
[Fig. 6B] Fig. 6B is a schematic view of an example of a wearable device according to an embodiment of the present invention, the wearable device including an image pickup apparatus.
[Fig. 7A] Fig. 7A is a schematic view showing an example of an image-forming apparatus according to an embodiment of the present invention.
[Fig. 7B] Fig. 7B is a schematic view showing an example of an exposure light source of an image-forming apparatus according to an embodiment of the present invention.
[Fig. 7C] Fig. 7C is a schematic view showing an example of an exposure light source of an image-forming apparatus according to an embodiment of the present invention.
[Fig. 8] Fig. 8 is a crystal structure of intermediate 7 obtained by single-crystal X-ray structure analysis.
[Fig. 9] Fig. 9 is a normalized emission spectrum of compound A-7 in a toluene solution.
[Fig. 10] Fig. 10 is a normalized emission spectrum of compound D-1 in a toluene solution.
[Fig. 11] Fig. 11 is a normalized emission spectrum of compound D-2 in a toluene solution.

### Description of Embodiments

In this specification, examples of halogen atoms include a fluorine atom, a chlorine atom, a bromine atom, and an iodine atom, but are not limited thereto.

Alkyl groups may have 1 to 20 carbon atoms, 1 to 10 carbon atoms, or 1 to 4 carbon atoms. Specific examples include a methyl group, an ethyl group, a normal propyl group, an isopropyl group, a normal butyl group, a tertiary butyl group, a secondary butyl group, an octyl group, a cyclohexyl group, a tertiary pentyl group, a 3-methylpentan-3-yl group, a 1-adamantyl group, and a 2-adamantyl group, but are not limited thereto.

Aryl groups may have 6 to 20 carbon atoms or 6 to 12 carbon atoms. Specific examples include a phenyl group, a naphthyl group, an indenyl group, a biphenyl group, a terphenyl group, a fluorenyl group, a phenanthryl group, a triphenylenyl group, a pyrenyl group, an anthranil group, a perylenyl group, a chrysenyl group, and a fluoranthenyl group, but are not limited thereto.

Heterocyclic groups may have 3 to 24 carbon atoms, 3 to 18 carbon atoms, or 3 to 12 carbon atoms. Specific examples include a pyridyl group, a pyrimidyl group, a pyrazyl group, a triazyl group, a benzofuranyl group, a benzothiophenyl group, a dibenzofuranyl group, a dibenzothiophenyl group, an oxazolyl group, an oxadiazolyl group, a thiazolyl group, a thiadiazolyl group, a carbazolyl group, an acridinyl group, and a phenanthrolyl group, but are not limited thereto.

Amino groups may be substituted amino groups substituted with an alkyl group or an aryl group, or substituted amino groups substituted with an alkyl group having 1 to 4 carbon atoms or an aryl group having 6 to 12 carbon atoms. Specific examples include an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, N,N-dimesitylamino group, an N-phenyl-N-(4-tertiary butylphenyl)amino group, an N-phenyl-N-(4-trifluoromethylphenyl)amino group, and an N-piperidyl group, but are not limited thereto.

Alkoxy groups may have 1 to 10 carbon atoms or 1 to 4 carbon atoms. Specific examples include a methoxy group, an ethoxy group, a propoxy group, a 2-ethyl-octyloxy group, and a benzyloxy group, but are not limited thereto.

Specific examples of aryloxy groups include a phenoxy group, but are not limited thereto.

Specific examples of heteroaryloxy groups include a thienyloxy group, but are not limited thereto.

Specific examples of silyl groups include a trimethylsilyl group and a triphenylsilyl group, but are not limited thereto.

Examples of substituents that the above alkyl groups, alkoxy groups, amino groups, aryloxy groups, silyl groups, aryl groups, and heterocyclic groups may further have include halogen atoms such as fluorine, chlorine, bromine, and iodine, alkyl groups such as a methyl group, an ethyl group, a normal propyl group, an isopropyl group, a normal butyl group, and a tertiary butyl group, alkoxy groups such as a methoxy group, an ethoxy group, and a propoxy group, amino groups such as a dimethylamino group, a diethylamino group, a dibenzylamino group, a diphenylamino group, and a ditolylamino group, aryloxy groups such as a phenoxy group, aryl groups such as a phenyl group and a biphenyl group, heterocyclic groups such as a pyridyl group and a pyrrolyl group, and a cyano group, but are not limited thereto.

### (1) Organic Compound

First, an organic compound according to the present invention will be described.

The organic compound according to the present invention is a compound represented by general formula (1-1) or (1-2).

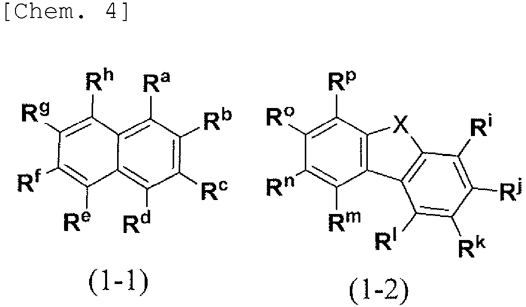

In general formula (1-1), at least four of bonding sites R^{a} to R^{h} are bonded to a skeleton selected from Structure A group. Among the bonding sites R^{a} to R^{h}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group.

In general formula (1-1), the bonding sites R^{d}, R^{c}, R^{f}, and R^{g} may be bonded to a skeleton selected from Structure A group.

In general formula (1-2), at least four of bonding sites Rⁱ to R^{p} are bonded to a skeleton selected from Structure A group. Among the bonding sites Rⁱ to R^{p}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

In general formula (1-2), the bonding sites R^{j}, R^{k}, Rⁿ, and R^{o} may be bonded to a skeleton selected from Structure A group.

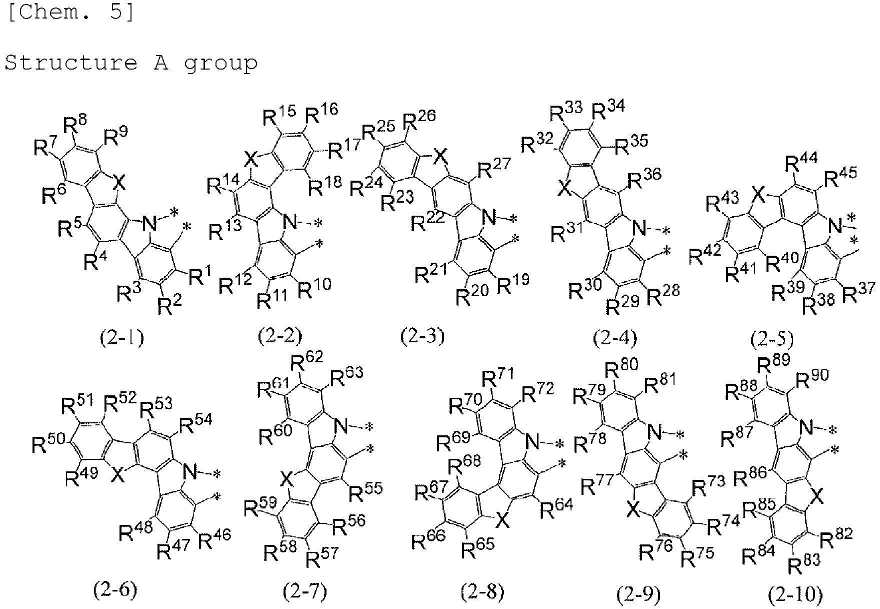

In general formulas (2-1) to (2-10), R¹ to R⁹⁰ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group. Bonding positions * represent positions of bonding to the bonding sites R^{a} to R^{p}.

The organic compound according to the present invention has a central skeleton that is a naphthalene skeleton or a skeleton in which six-membered rings are fused on both sides of a five-membered ring structure (e.g., a fluorene skeleton) and has an indolocarbazole skeleton further having a fused-ring structure, and thus is a compound having high thermal stability. The term "central skeleton" refers to a skeleton represented by general formula (1-1) or (1-2). Specifically, the organic compound according to the present invention differs from compound Ref-1 and compound Ref-2 in that the organic compound is structured to have a central skeleton that is a naphthalene skeleton or a skeleton in which six-membered rings are fused on both sides of a five-membered ring structure (e.g., a fluorene skeleton) and have a carbazole skeleton to which benzofuran, benzothiophene, or the like is fused. Accordingly, the organic compound according to the present invention has a larger molecular weight than compound Ref-1 and compound Ref-2. As a result, the organic compound according to the present invention has strong intermolecular forces, and thus is an organic compound having high thermal stability.

Table 1 shows the results of the TG-DTA (thermogravimetry-differential thermal analysis) measurement of the organic compound according to the present invention and compound Ref-1, which is a comparative example.

### [Table 1]

**Table 1**

| Compound | Compound A-6 | Compound A-7 | Compound Ref-1 |
|---|---|---|---|
| Structural formula | 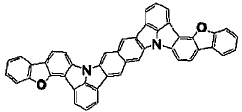 | 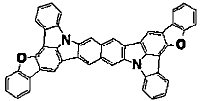 | 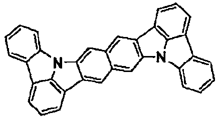 |
| 5% weight-loss temperature | 596°C | 573°C | 452°C |

| Compound | Compound D-1 | Compound D-2 | |
|---|---|---|---|
| Structural formula | 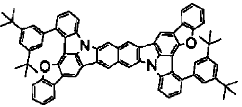 | 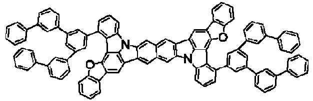 | |
| 5% weight-loss temperature | 540°C | 553°C | |

Table 1 shows that the 5% weight-loss temperatures of compounds A-6, A-7, D-1, and D-2, which are examples of the organic compound according to the present invention, are 596°C, 573°C, 540°C, and 553°C, respectively, and are sufficiently higher than the 5% weight-loss temperature of compound Ref-1, which is a comparative example. In particular, it is shown that the 5% weight-loss temperatures of compounds A-6, A-7, and D-2 are higher than the 5% weight-loss temperature of compound Ref-1 by 100°C or more.

As described above, the organic compound according to the present invention has a large molecular weight because it is structured to have a central skeleton that is a naphthalene skeleton or a skeleton in which six-membered rings are fused on both sides of a five-membered ring structure (e.g., a fluorene skeleton) and have a carbazole skeleton to which benzofuran, benzothiophene, or the like is fused. Therefore, the organic compound according to the present invention is considered to have high thermal stability.

Furthermore, the fusion of benzofuran or benzothiophene to a carbazole skeleton tends to decrease the HOMO (Highest Occupied Molecular Orbital) energy level. Therefore, among the organic compounds according to the present invention, compounds having a carbazole skeleton to which benzofuran or benzothiophene is fused are considered to have high stability against oxidation.

Because of having the above features, the organic compound according to the present invention, when used in an organic light-emitting element, can provide an organic light-emitting element having high durability.

Hereinafter, specific examples of the organic compound according to the present invention will be shown below. However, the present invention is not limited thereto.

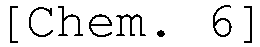

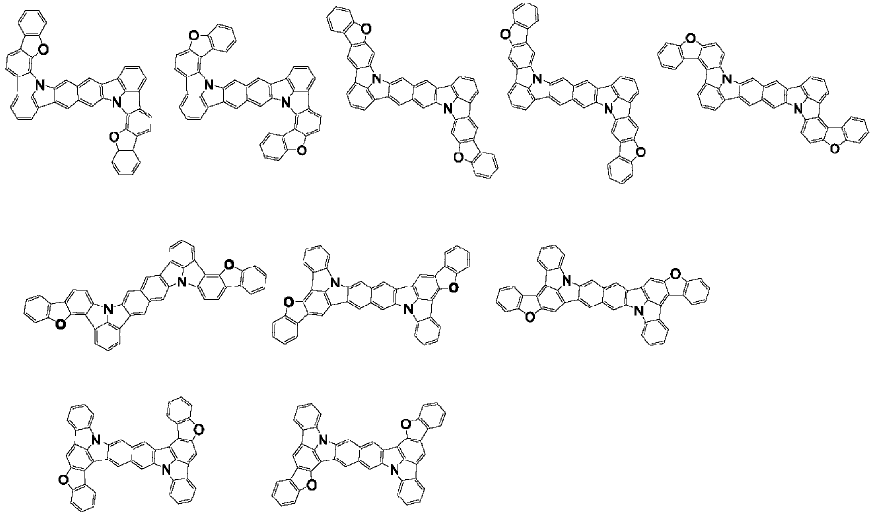

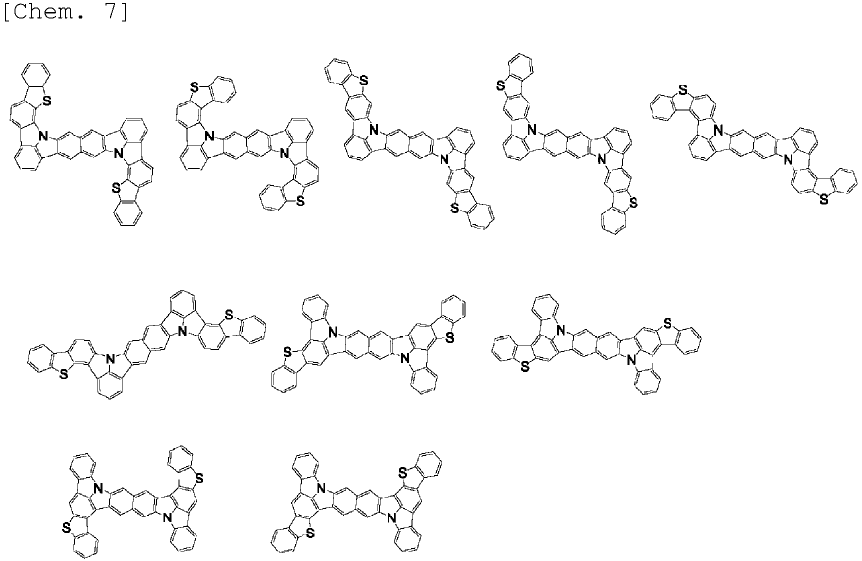

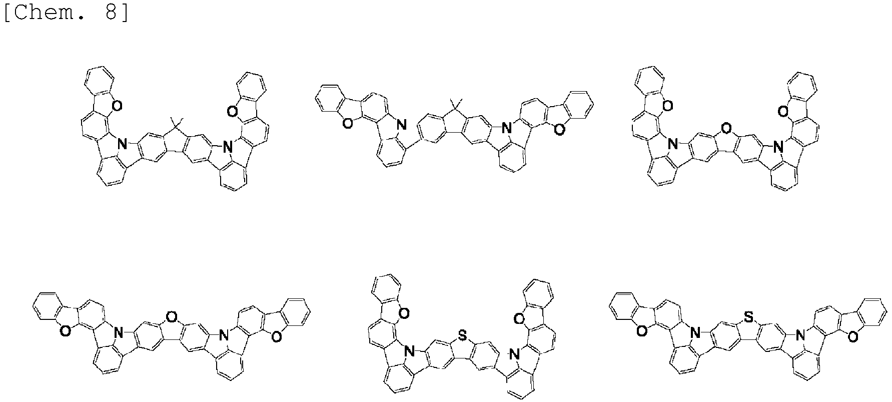

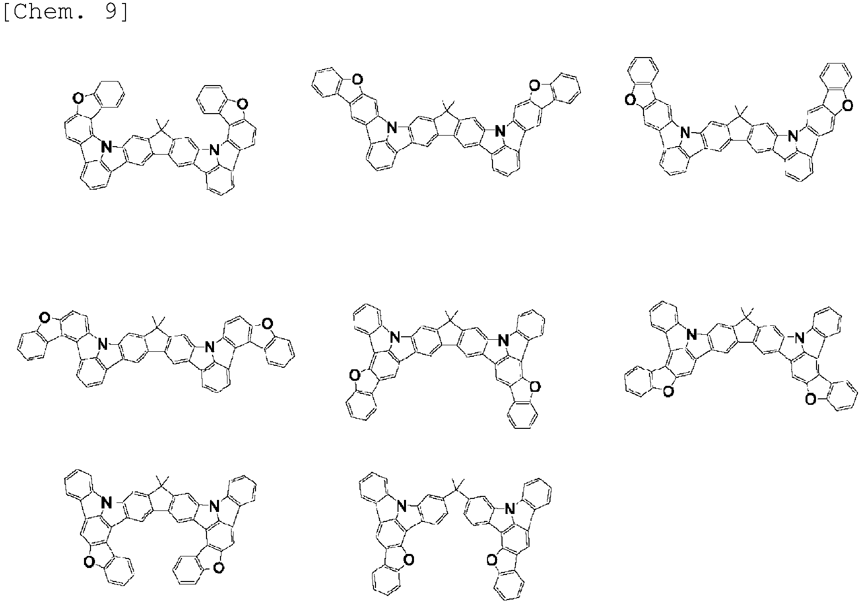

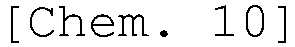

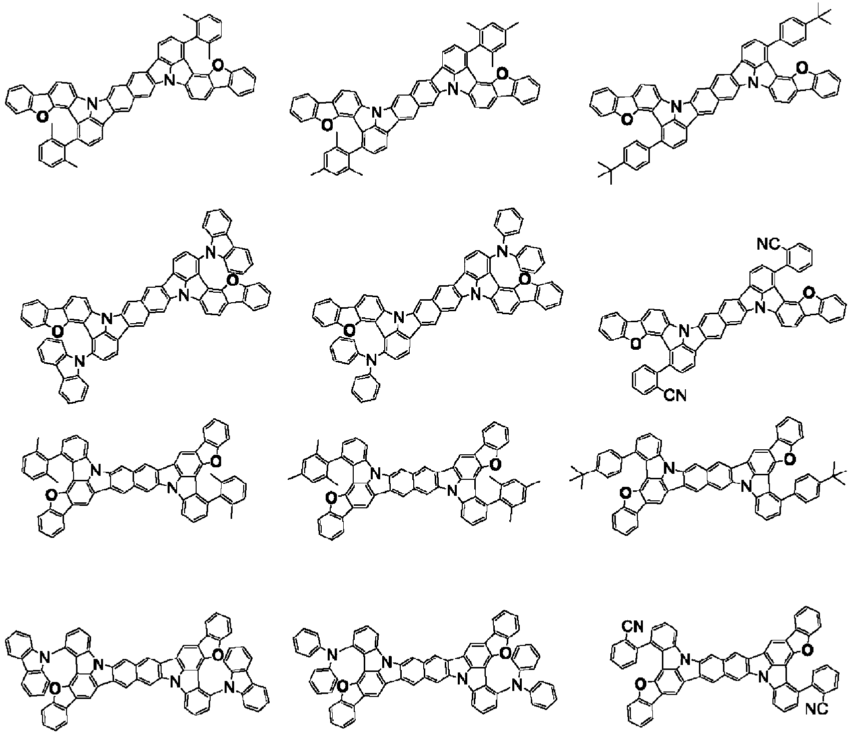

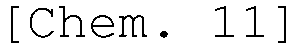

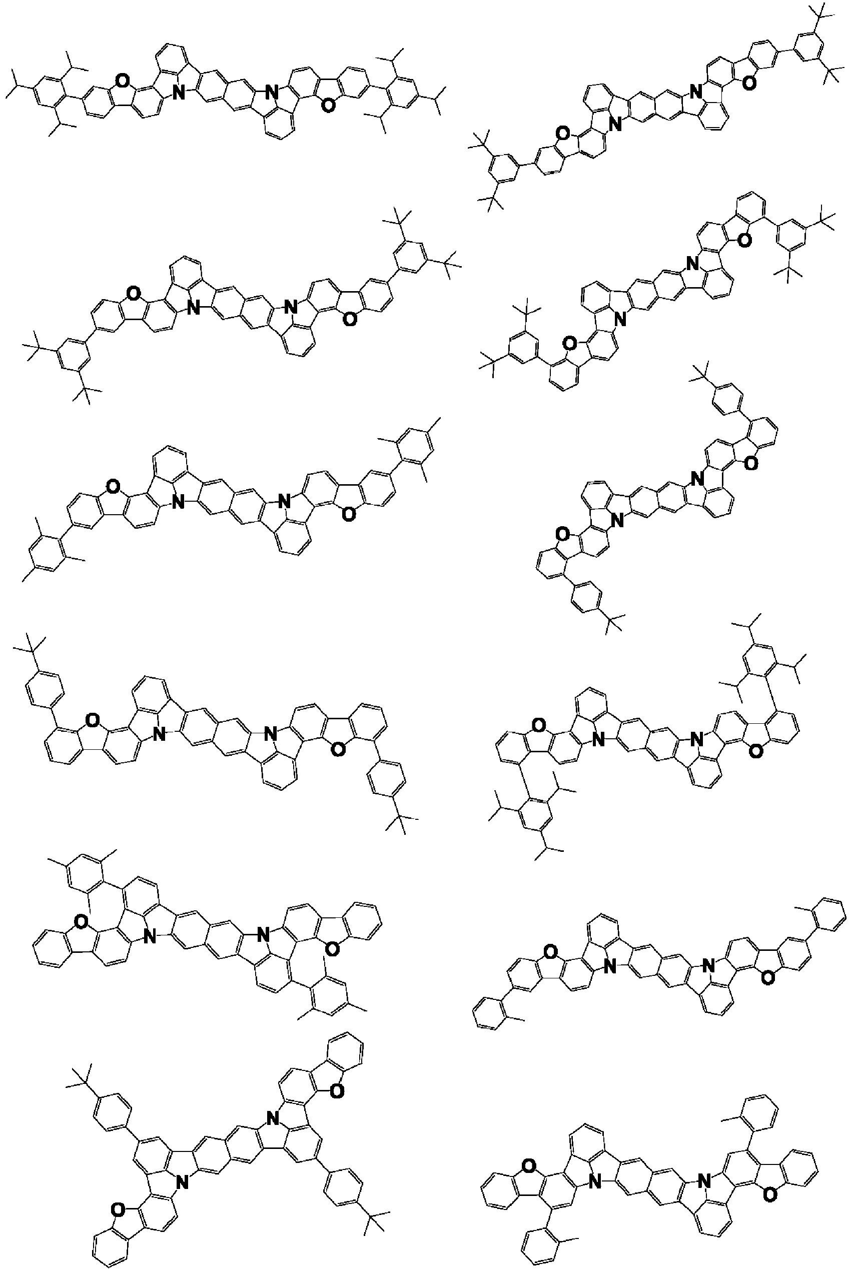

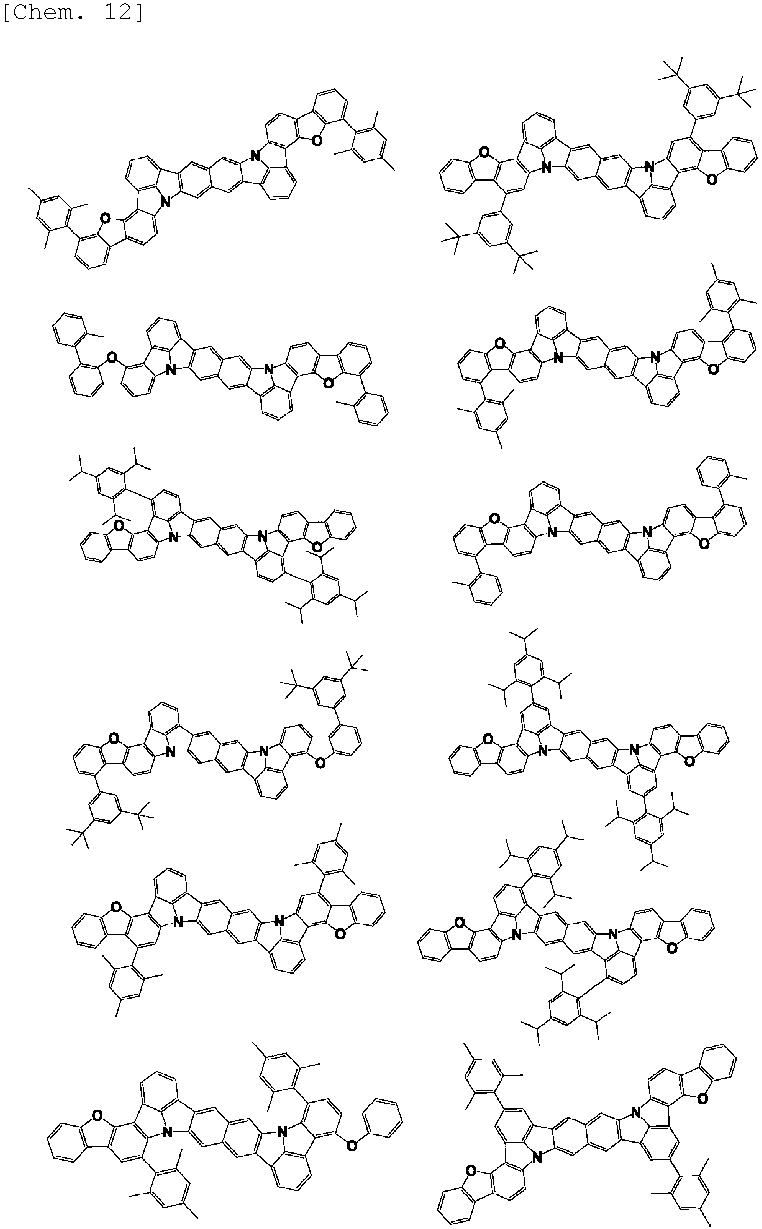

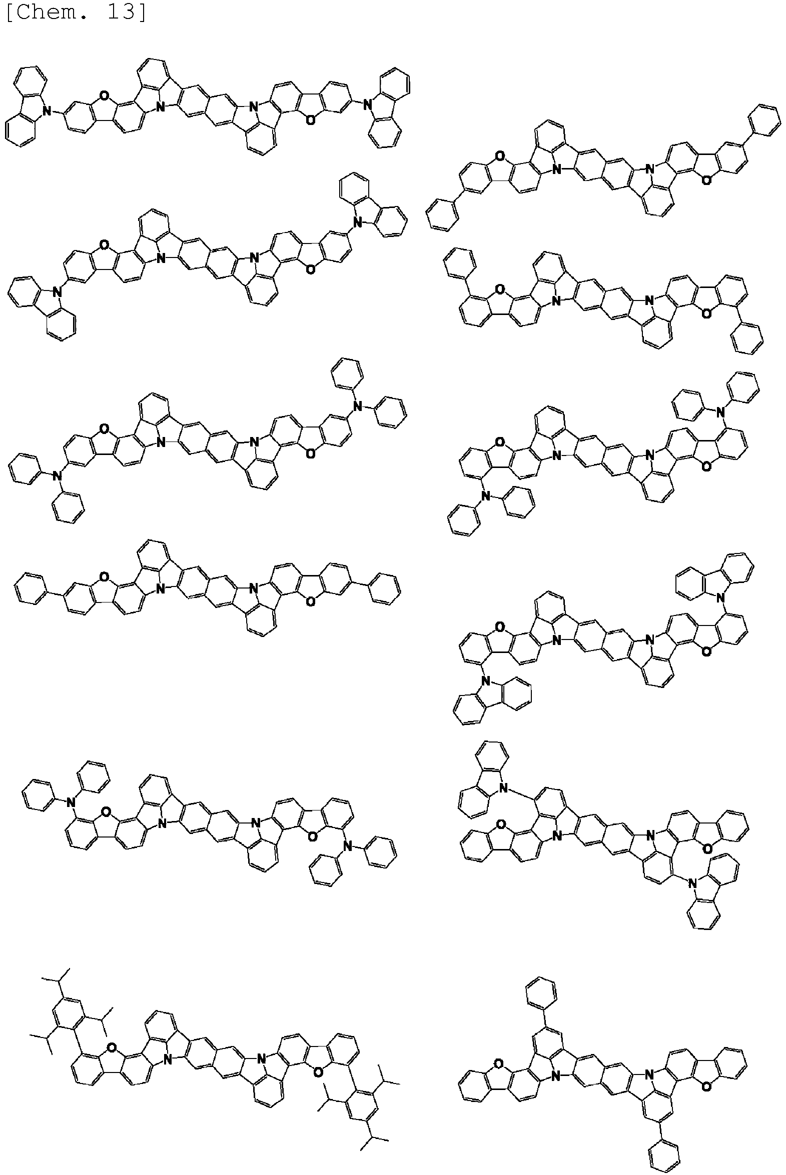

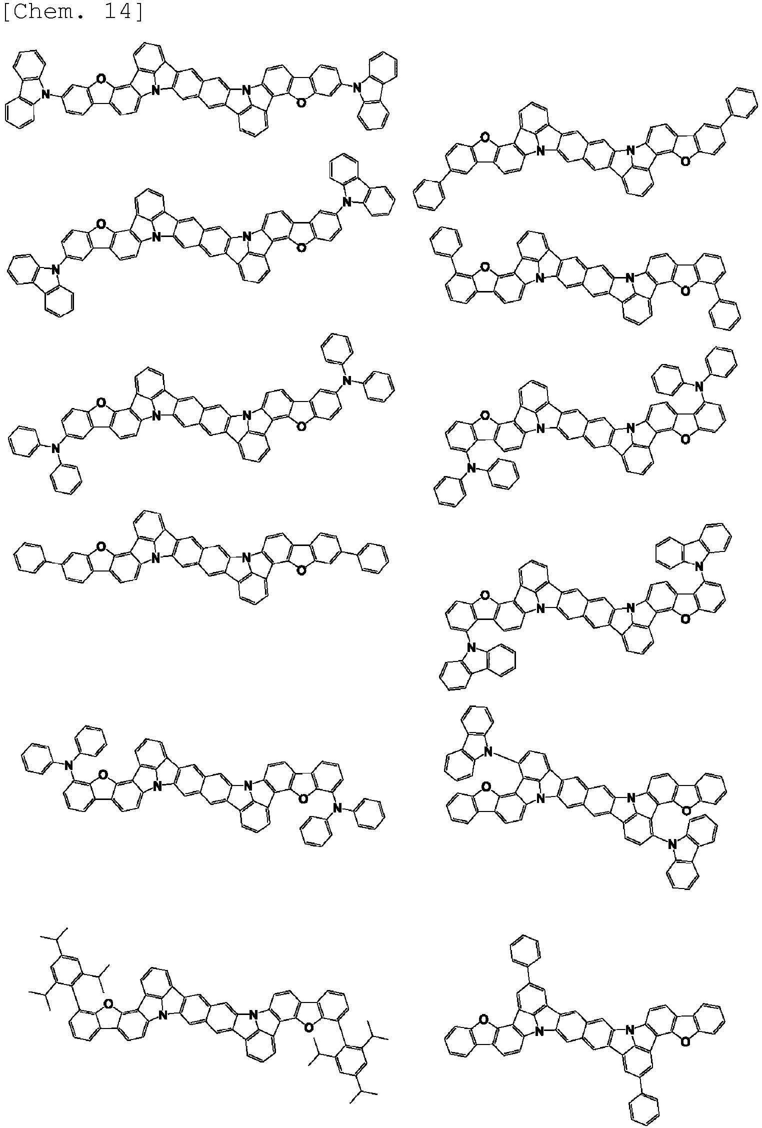

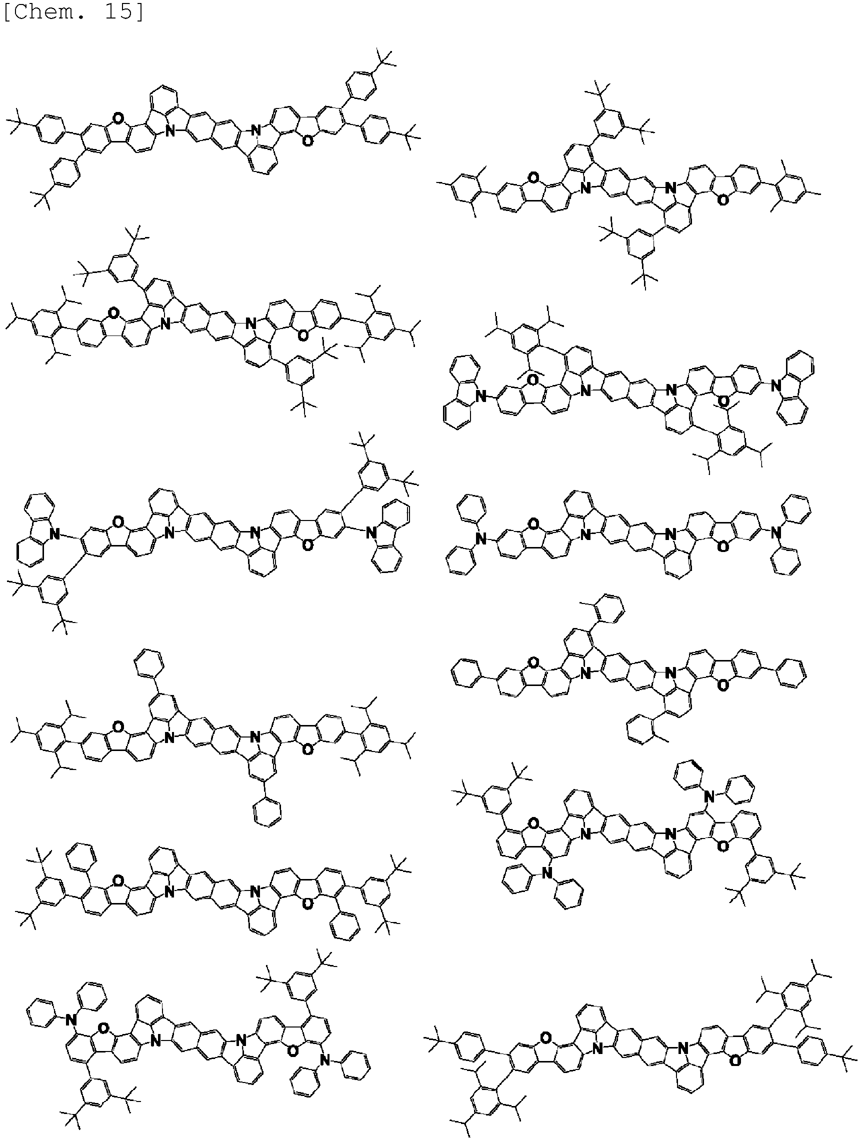

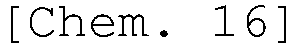

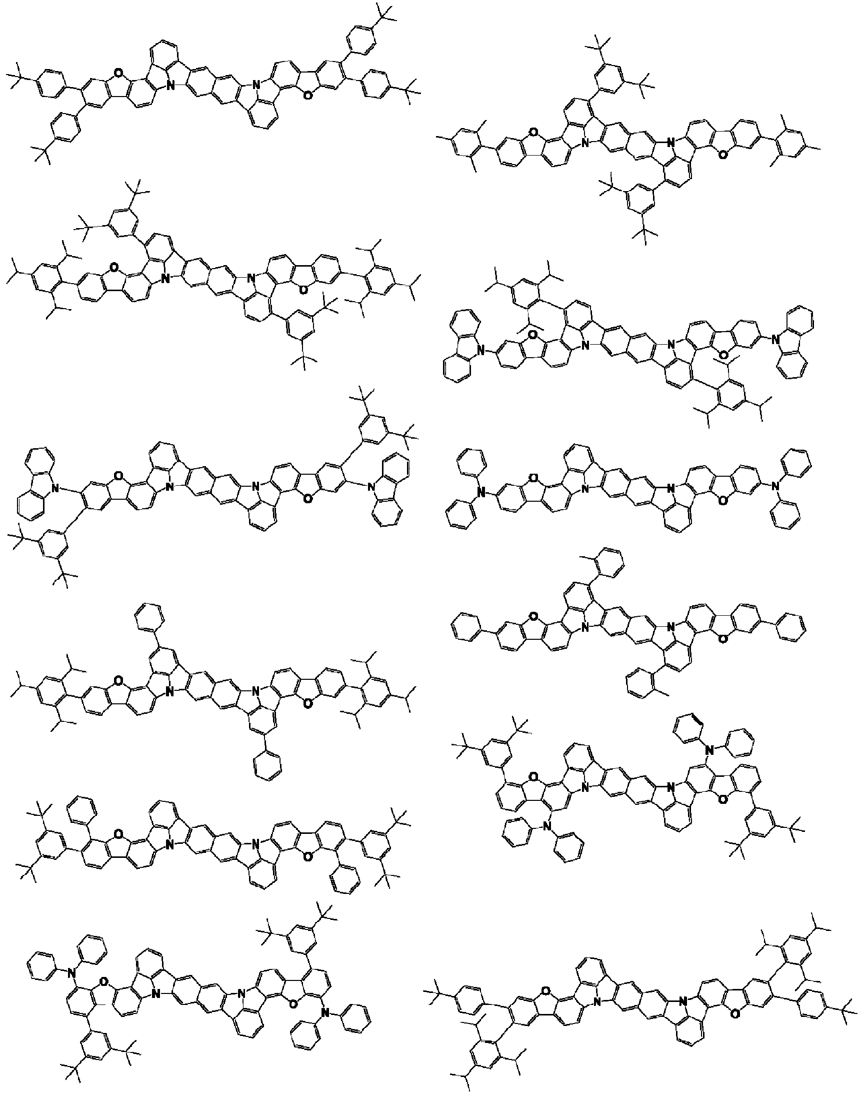

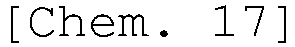

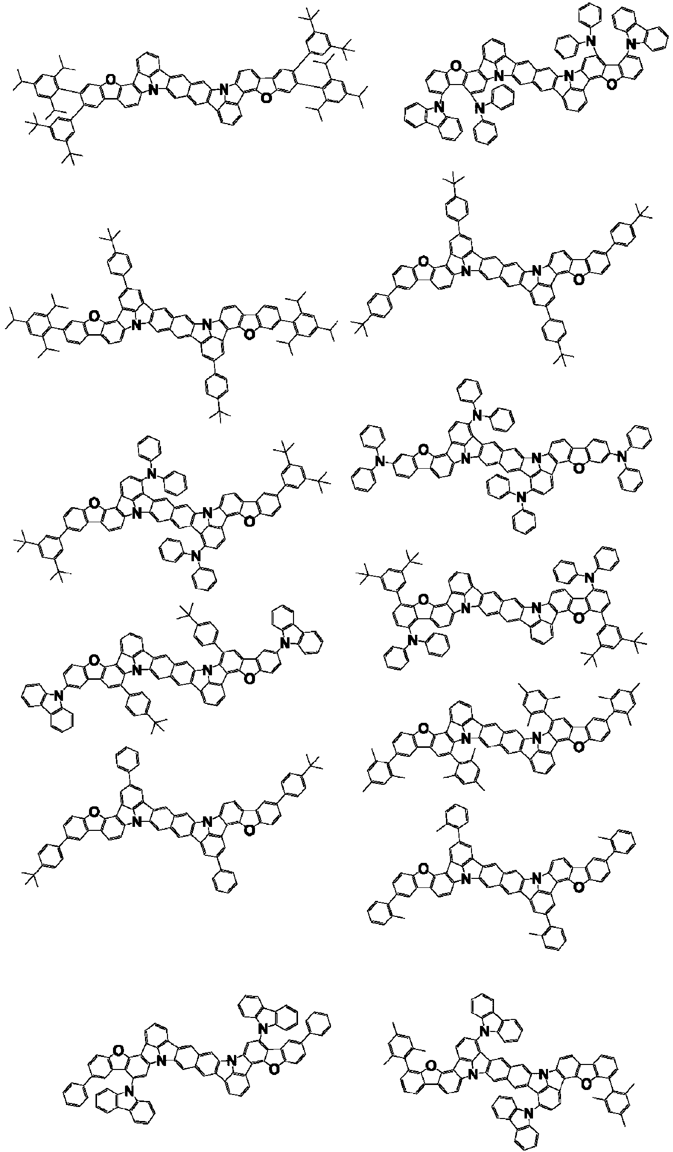

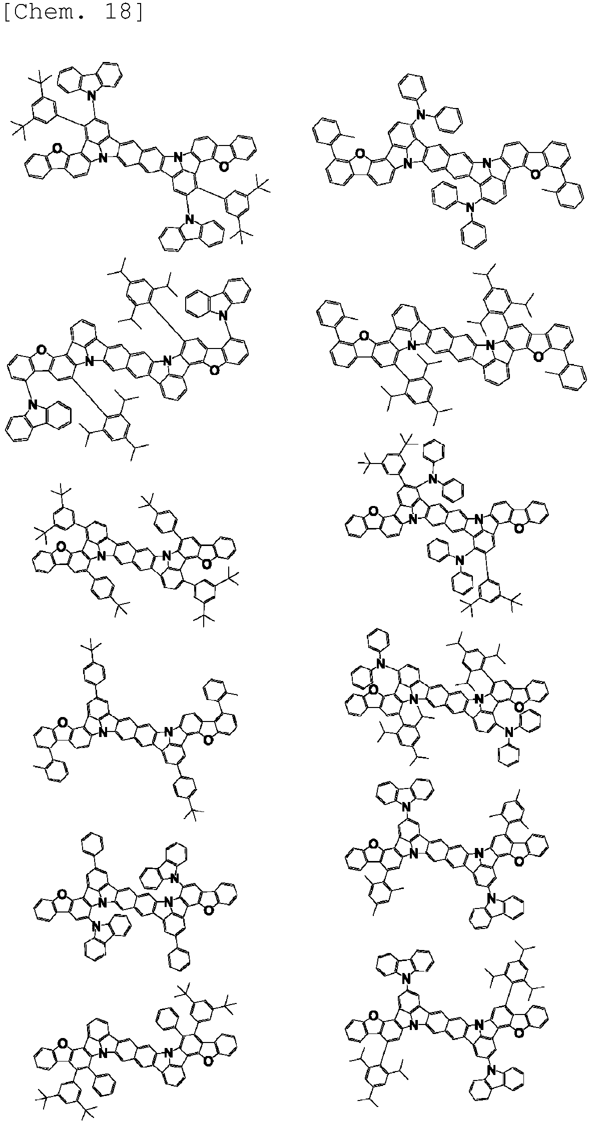

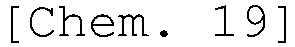

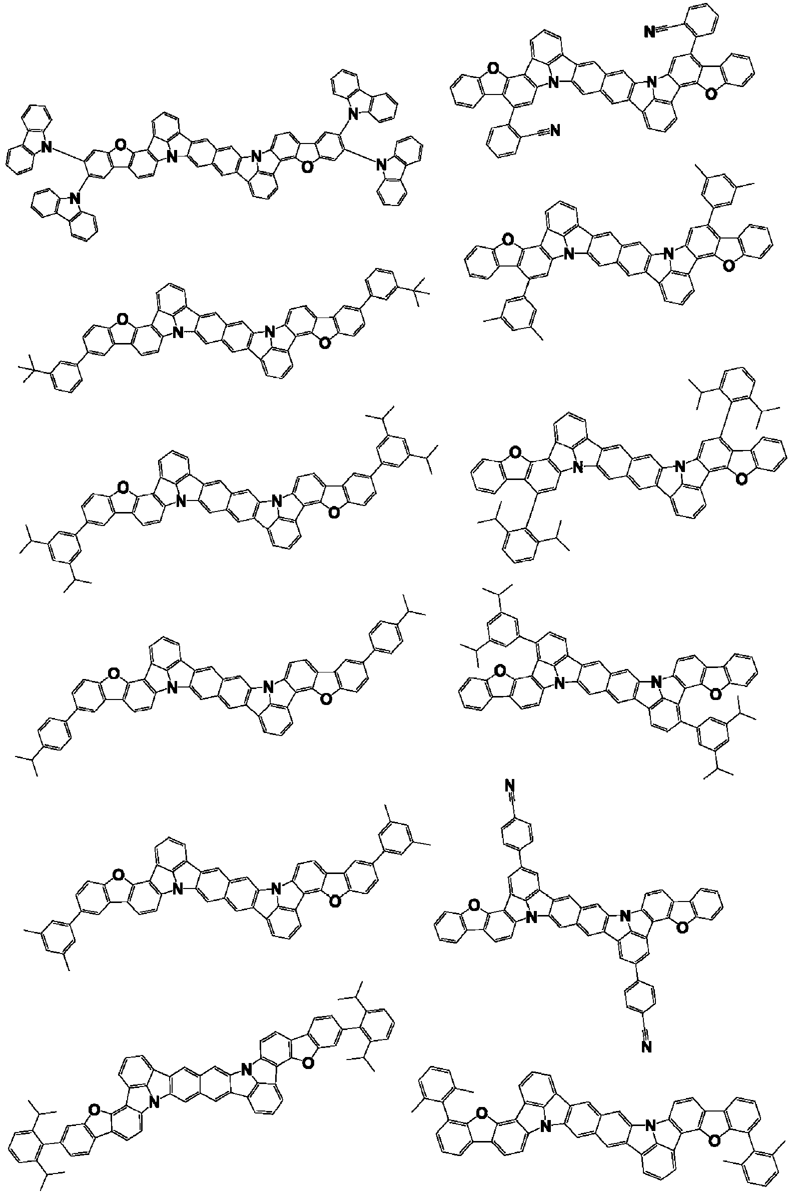

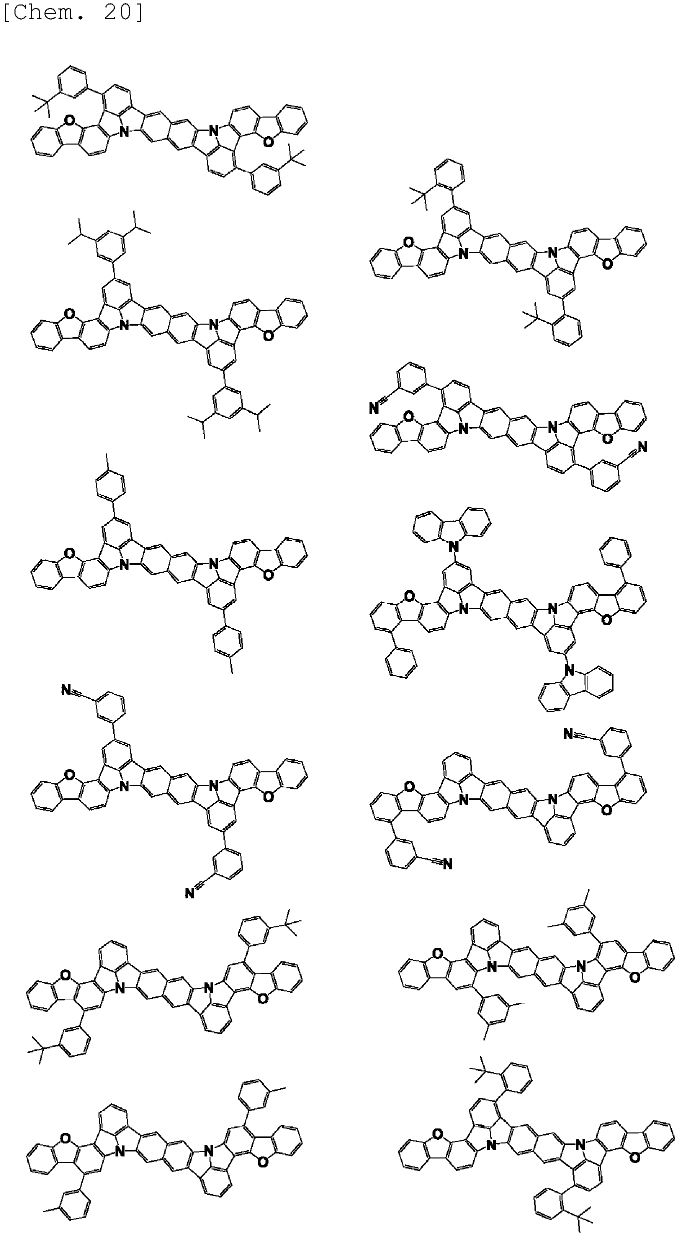

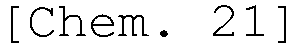

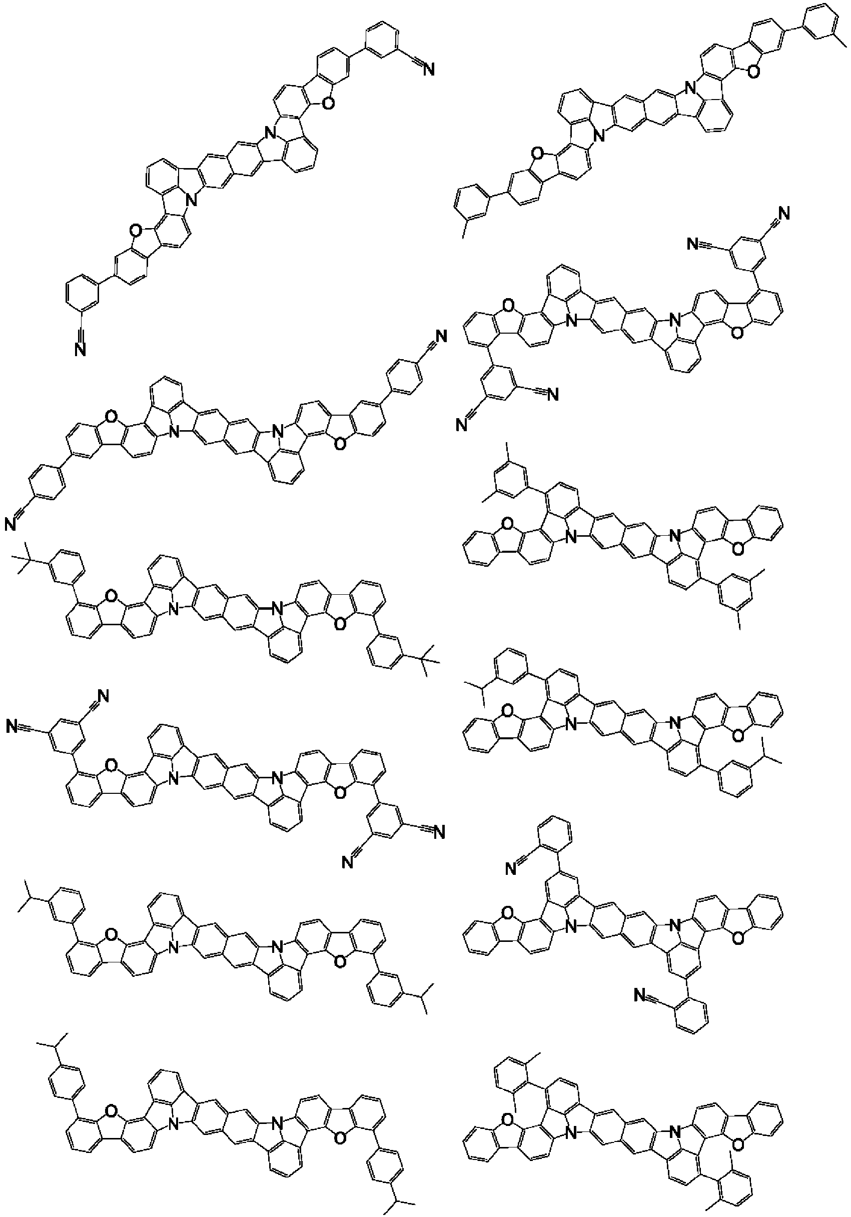

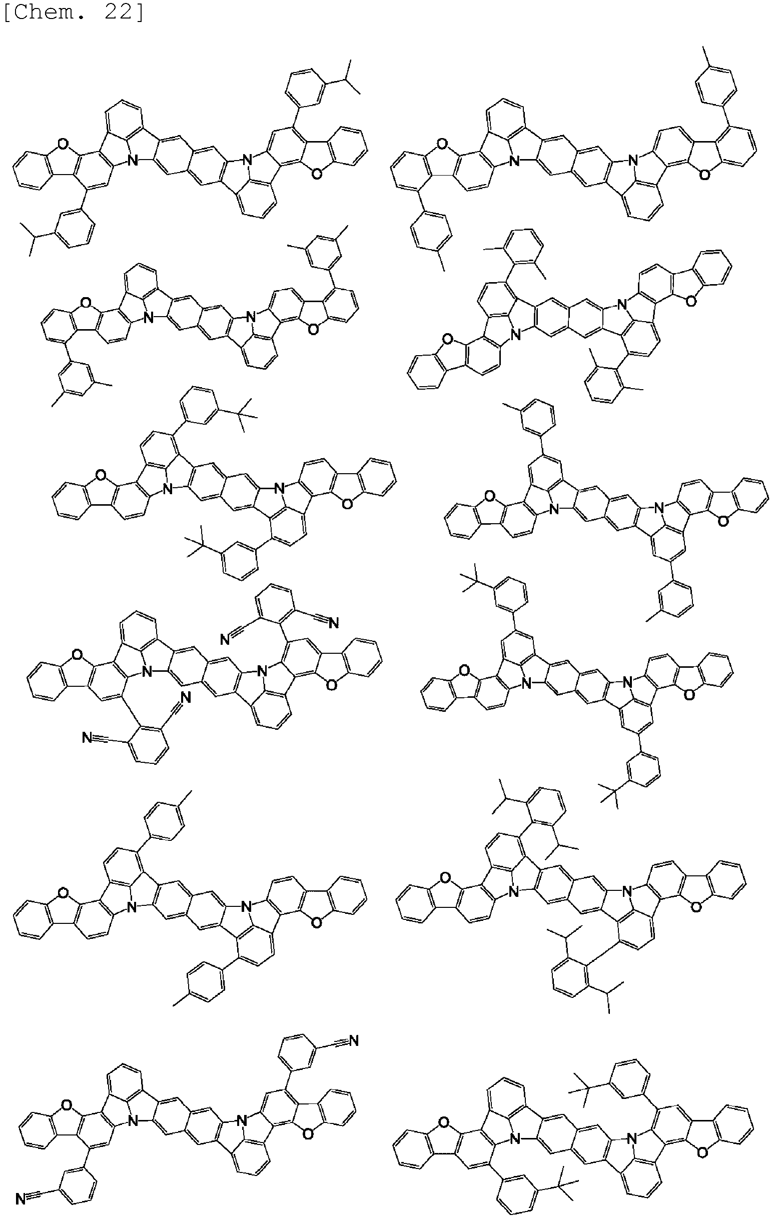

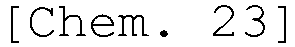

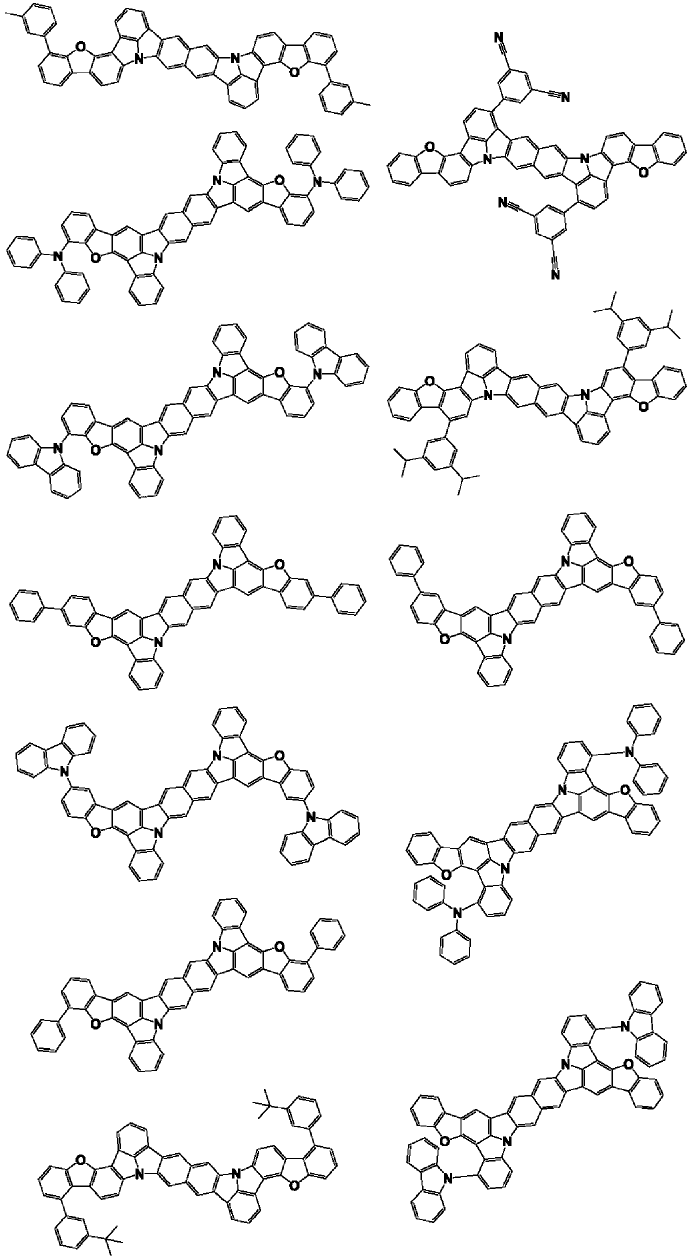

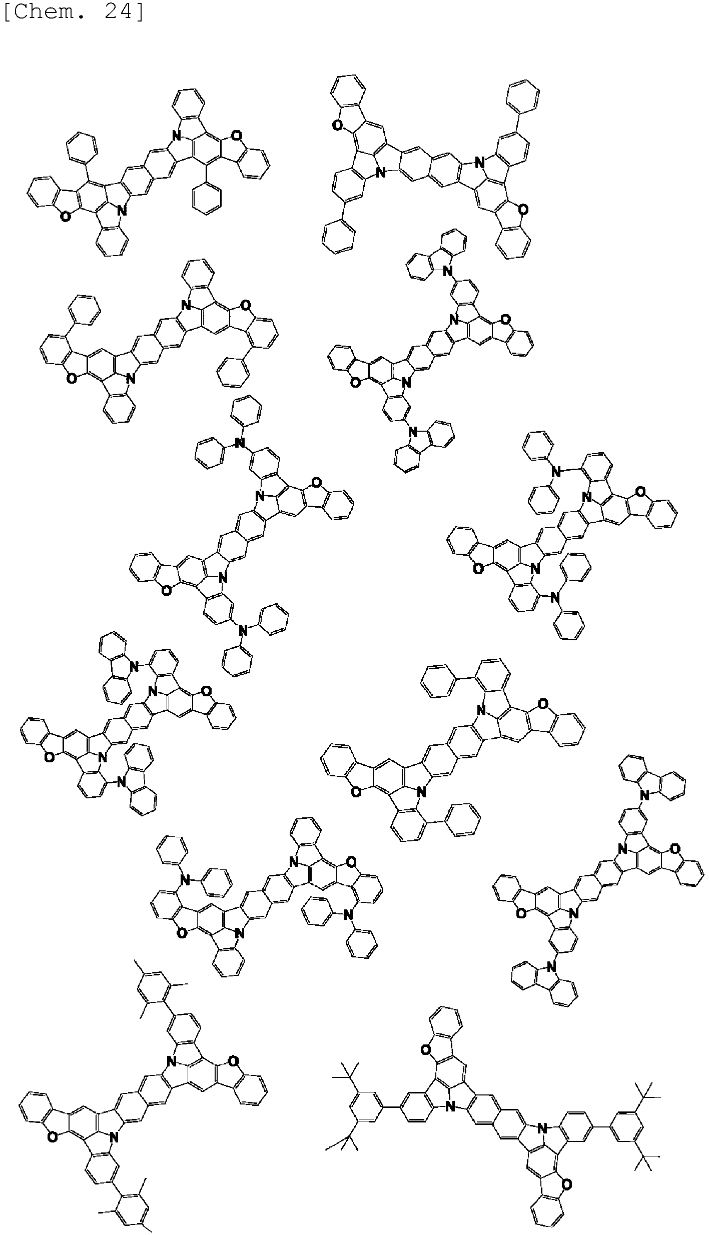

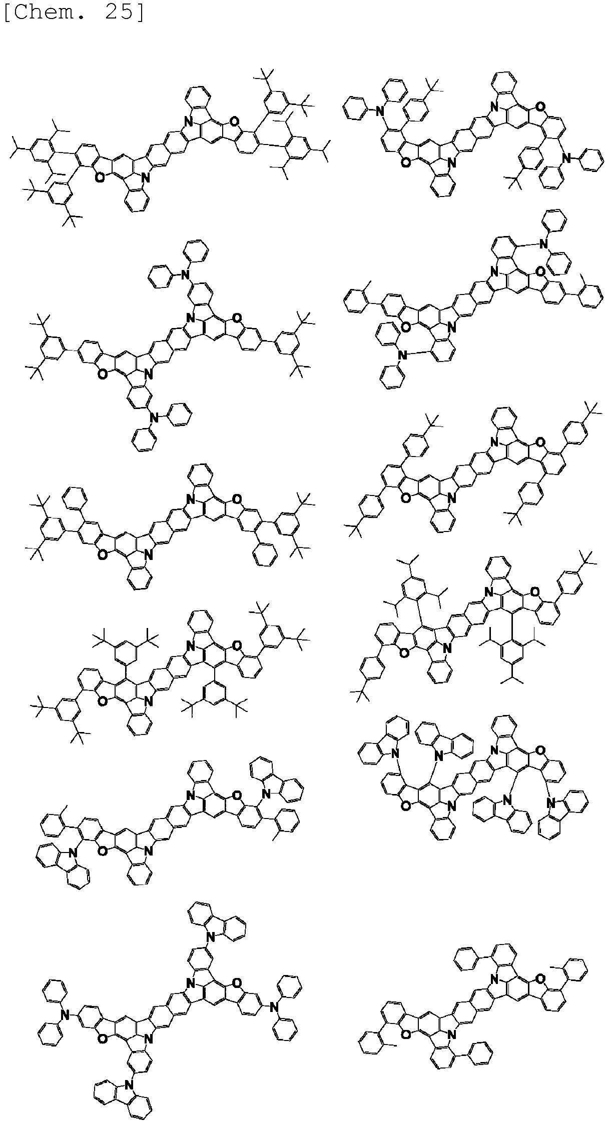

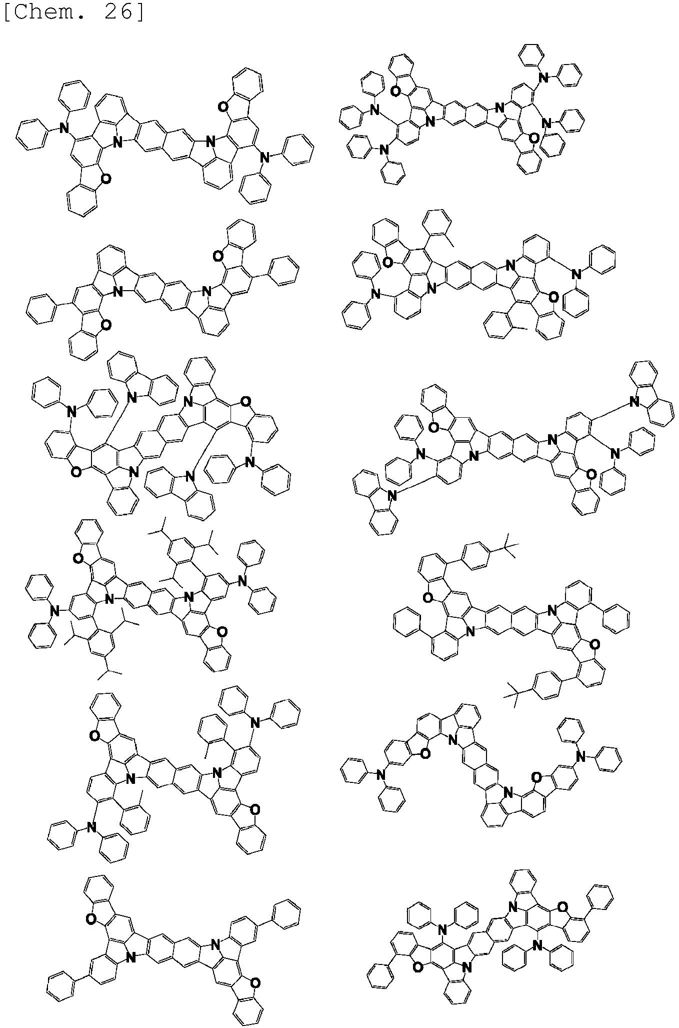

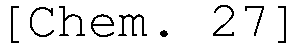

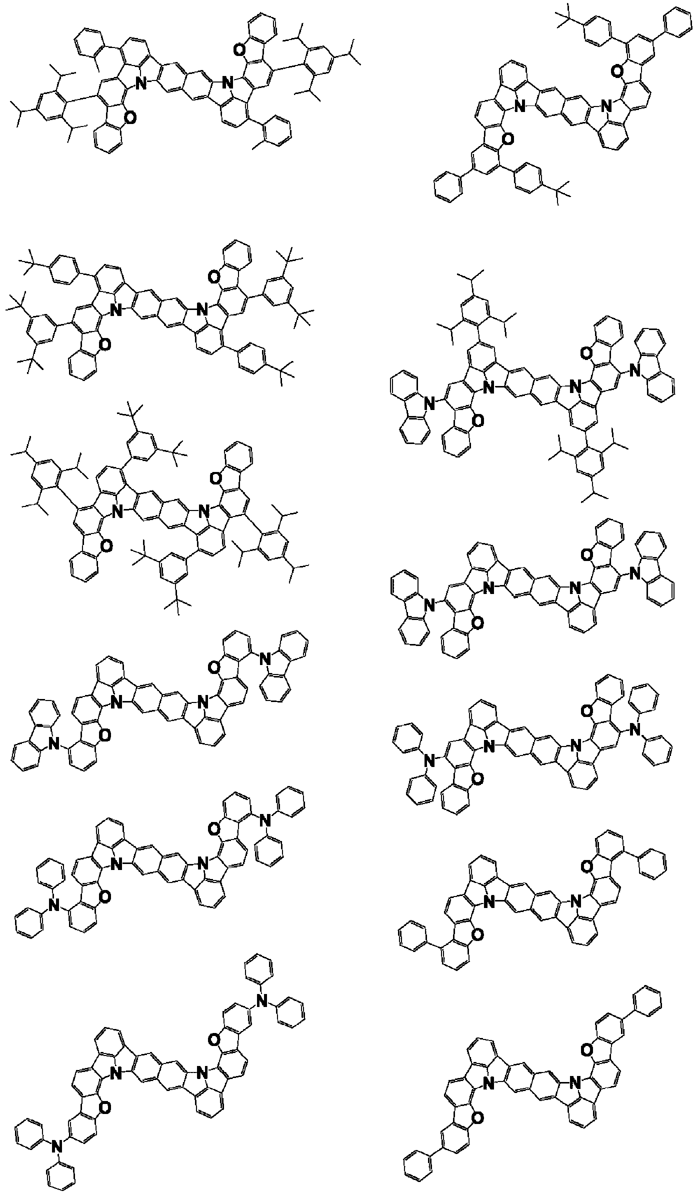

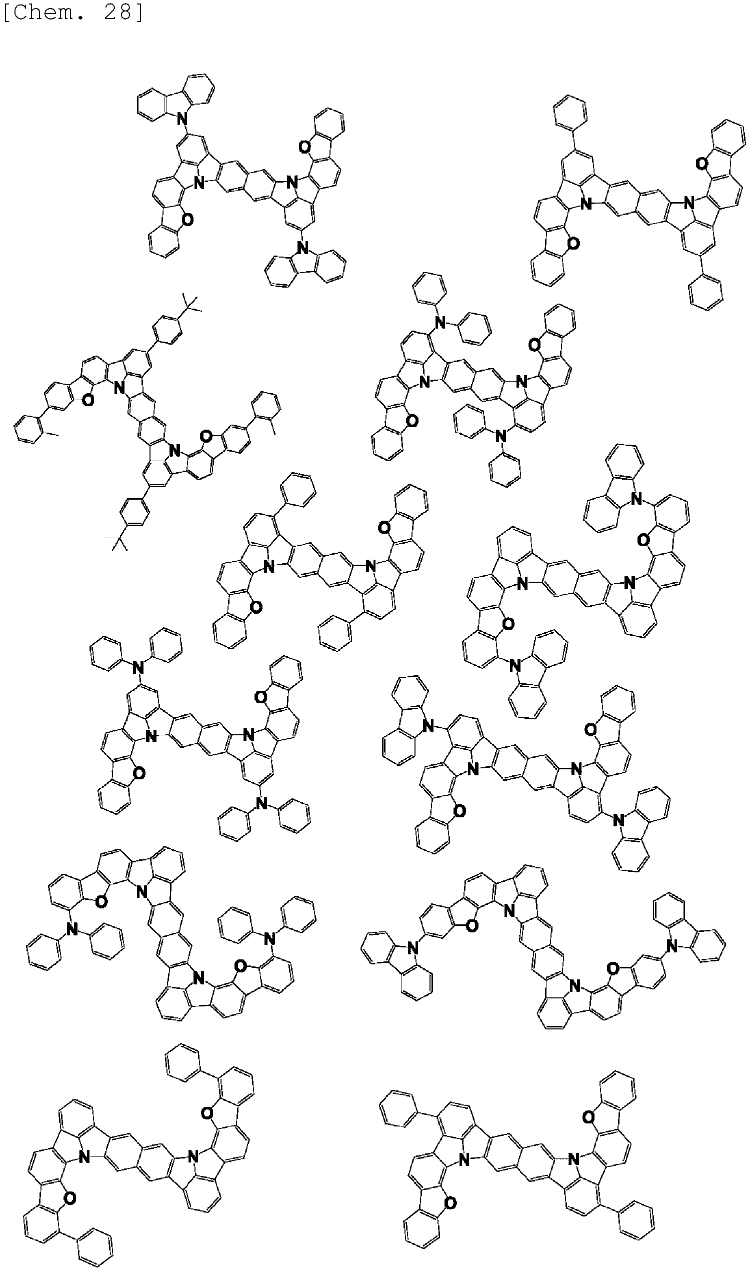

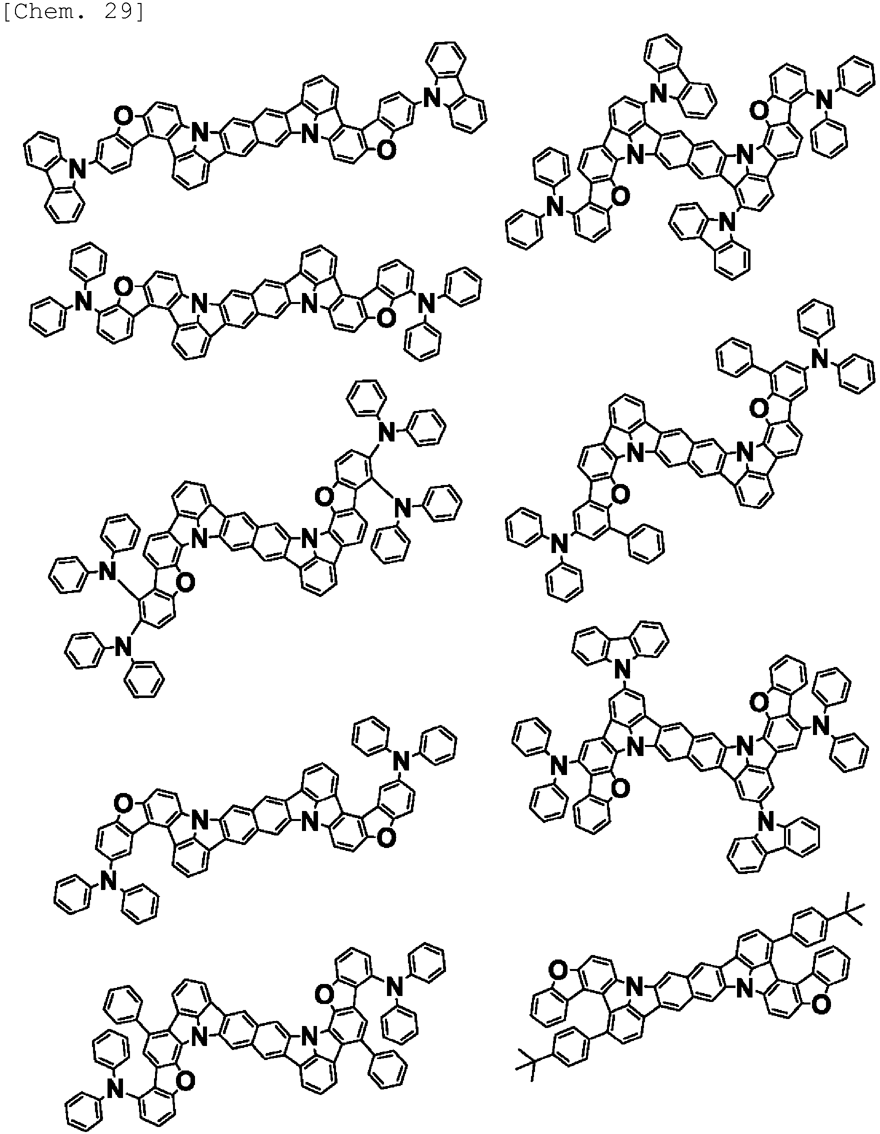

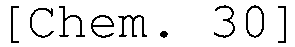

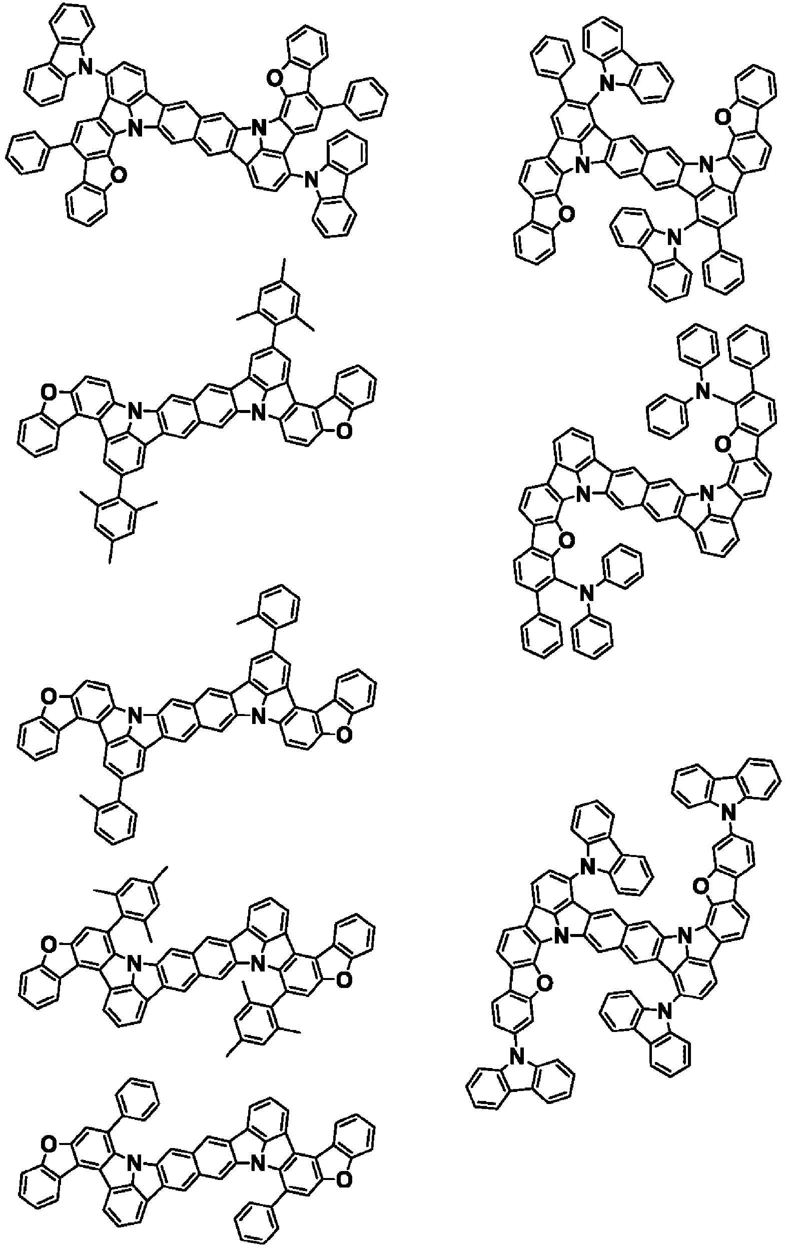

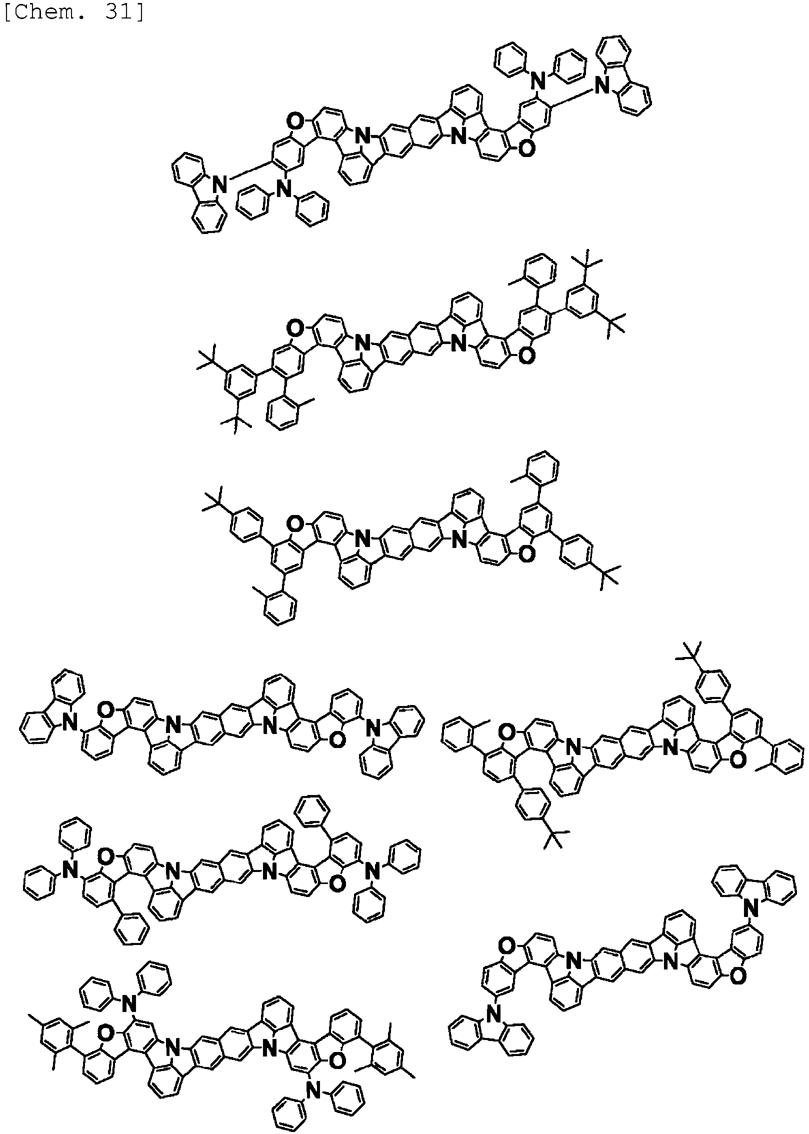

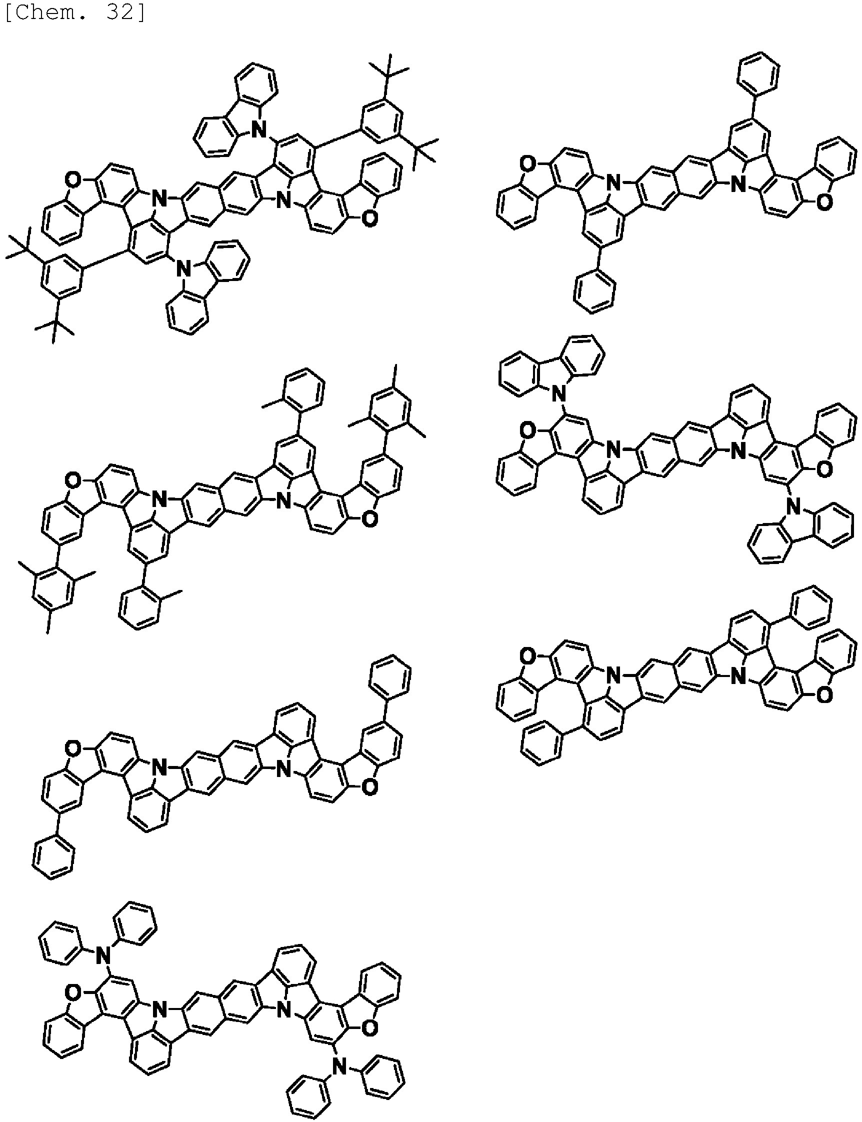

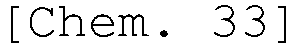

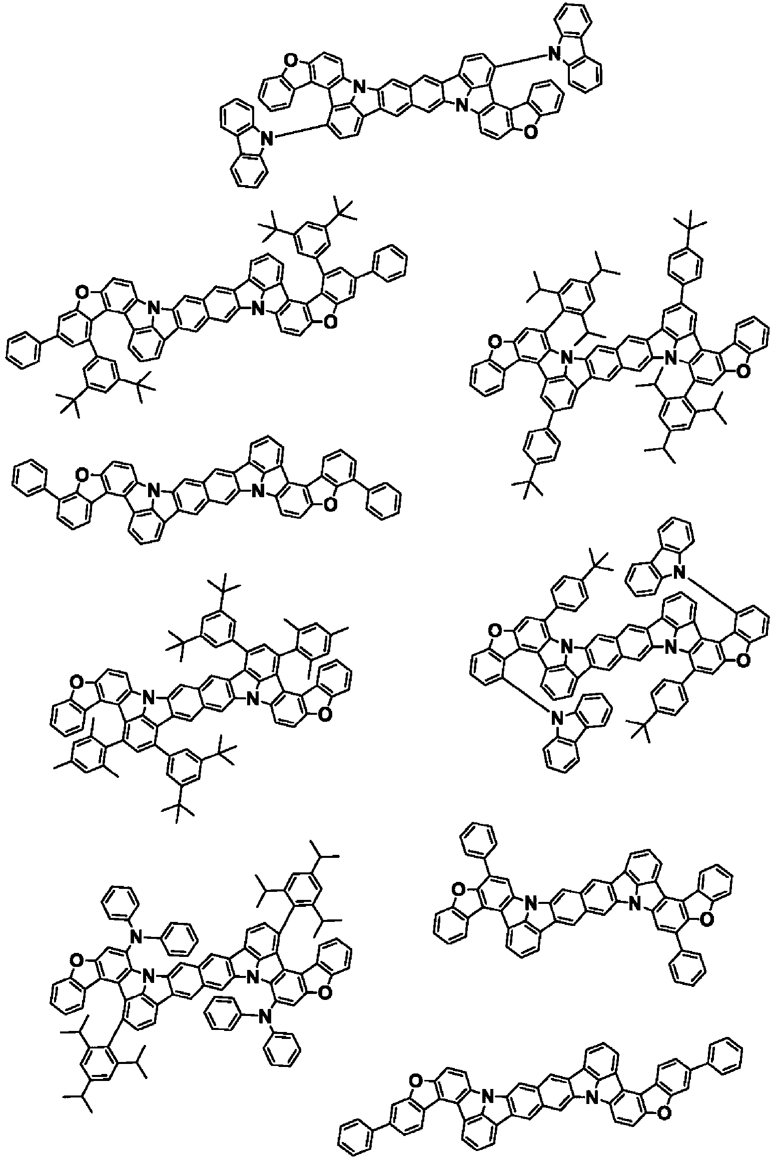

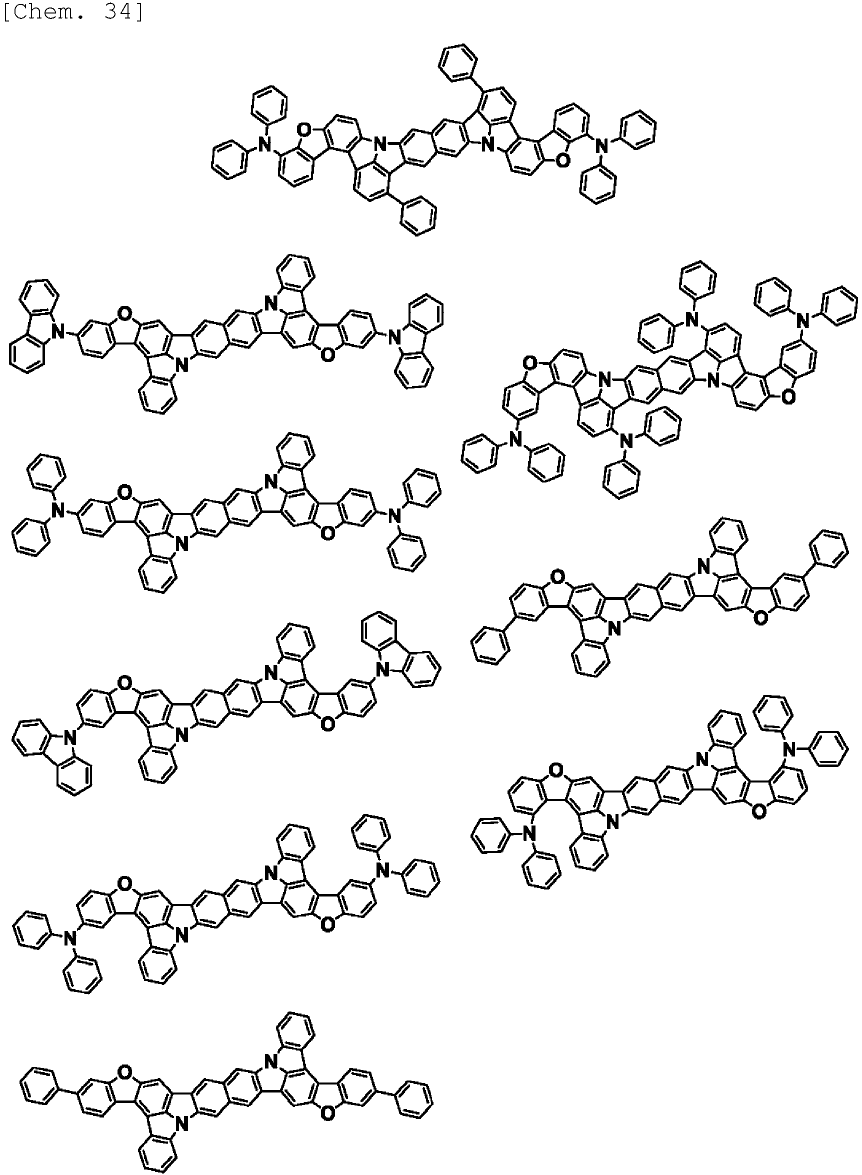

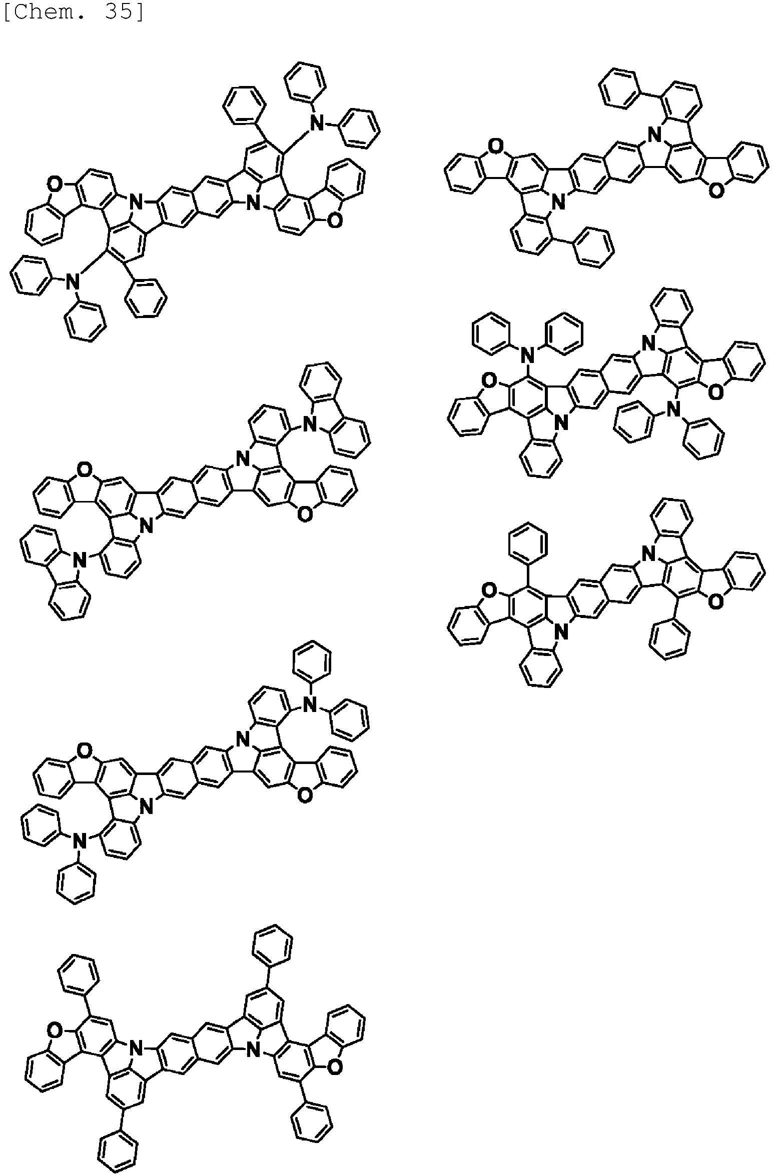

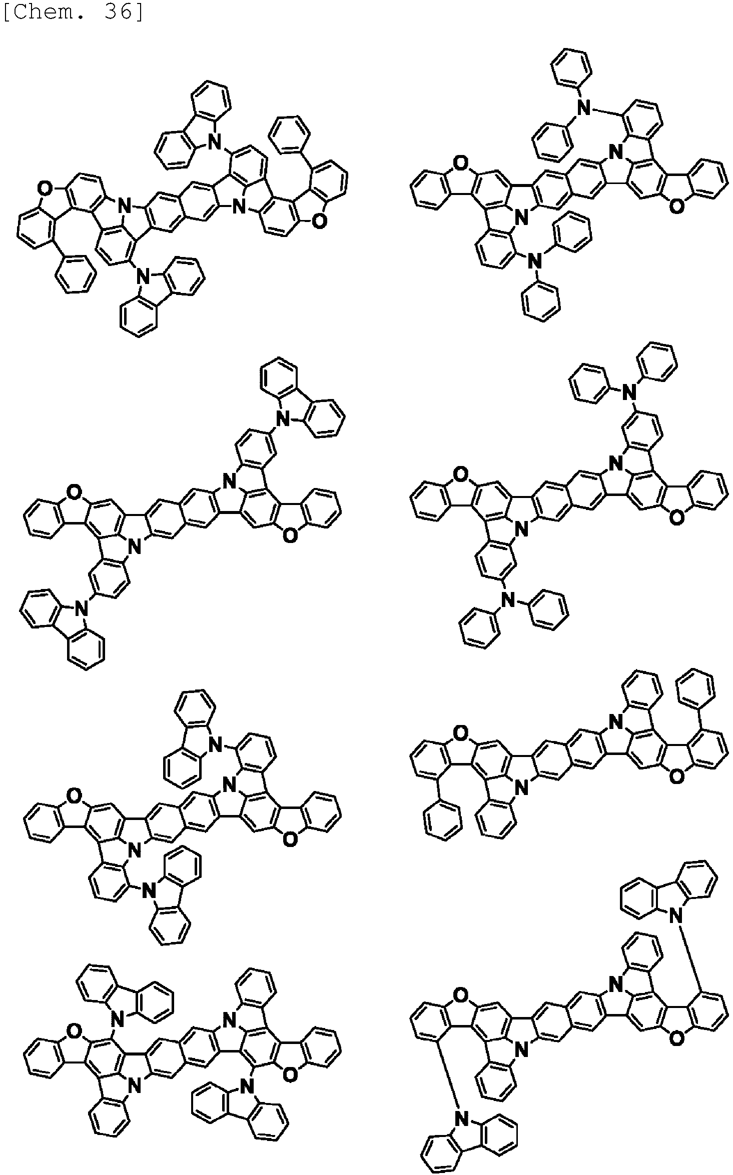

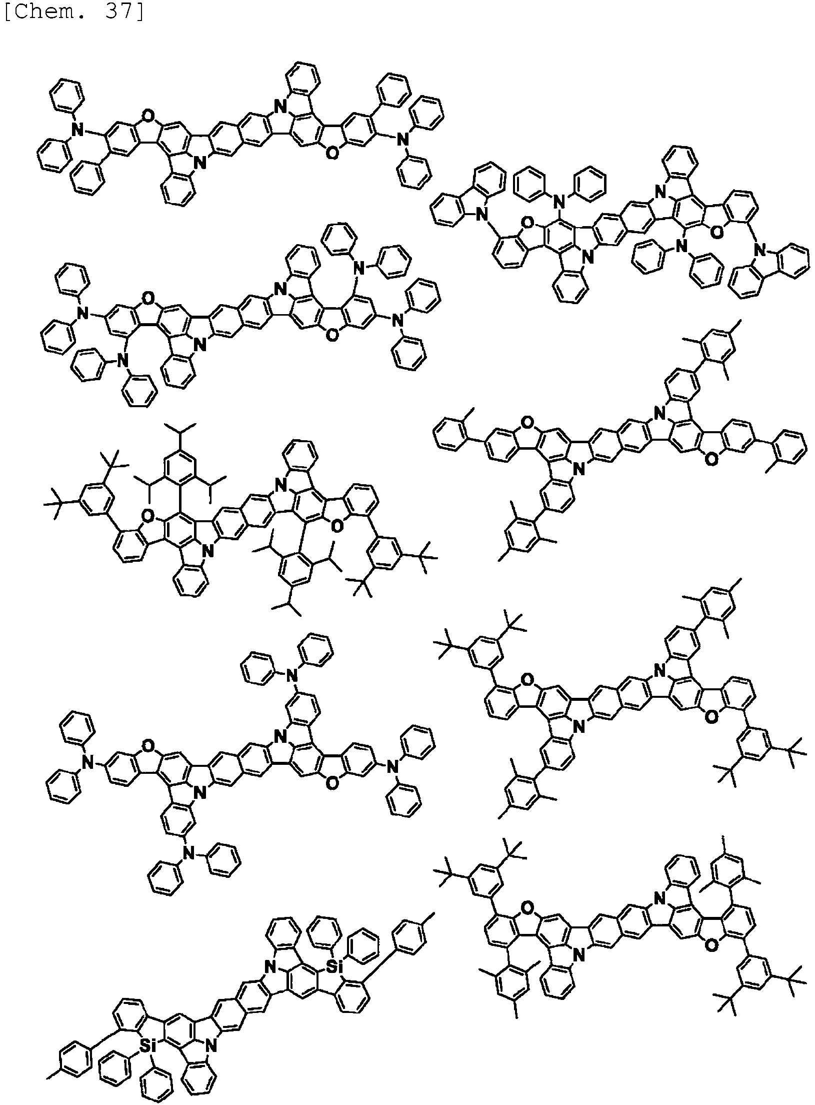

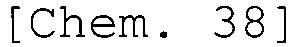

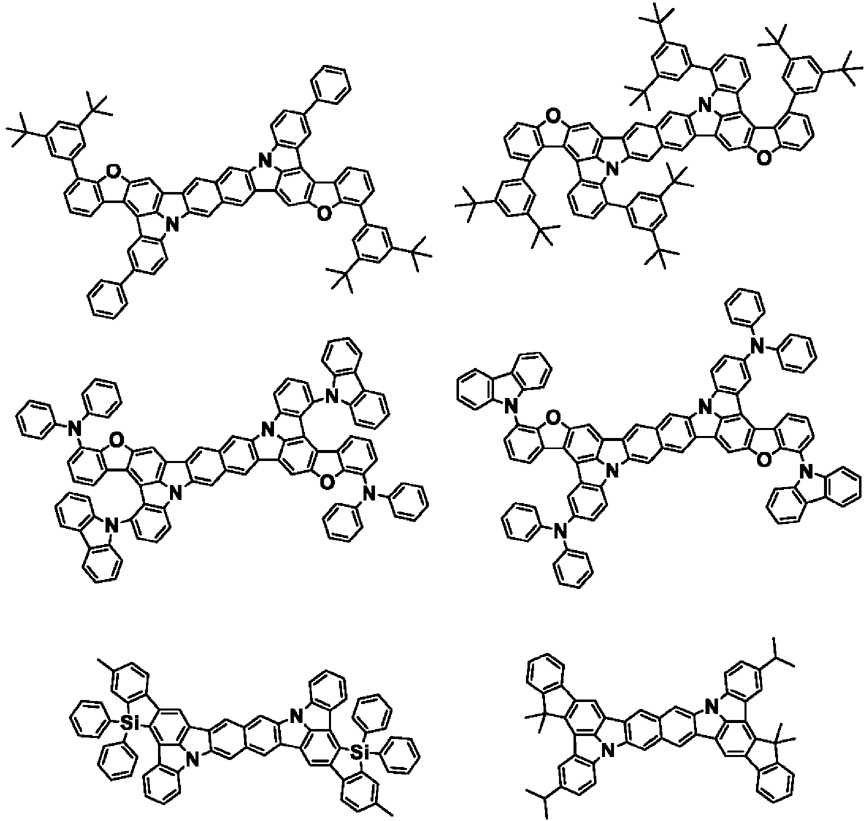

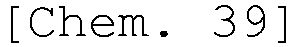

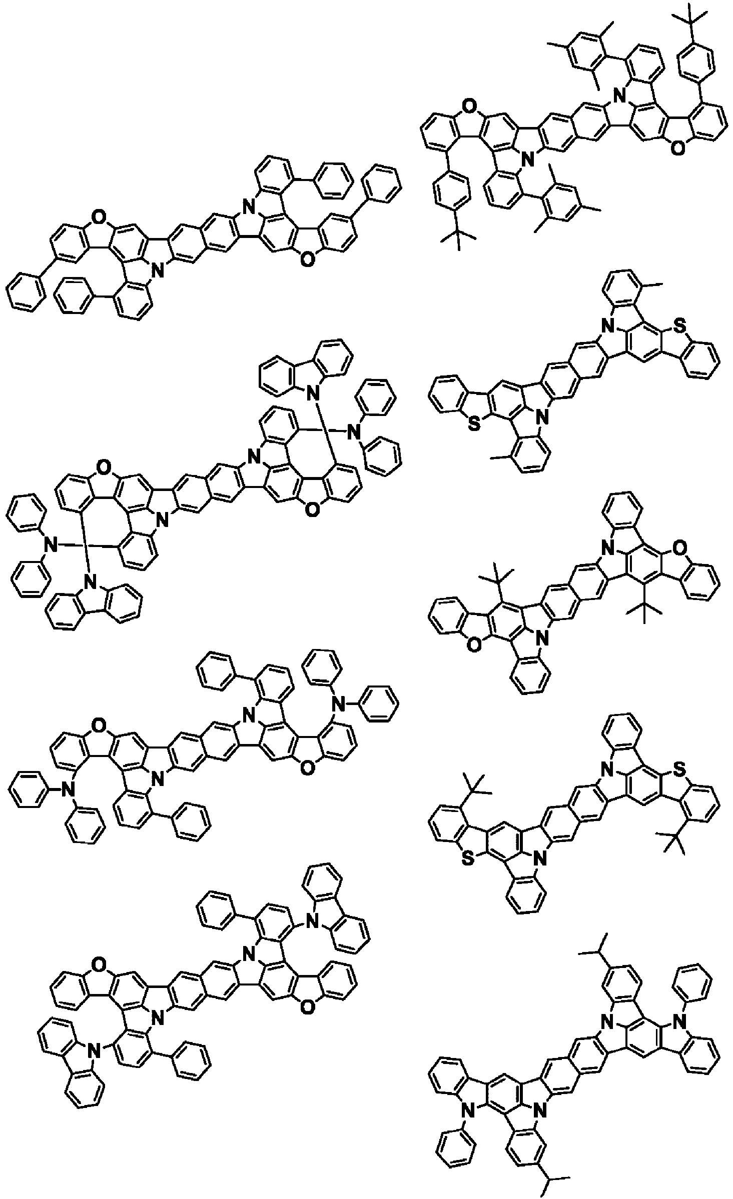

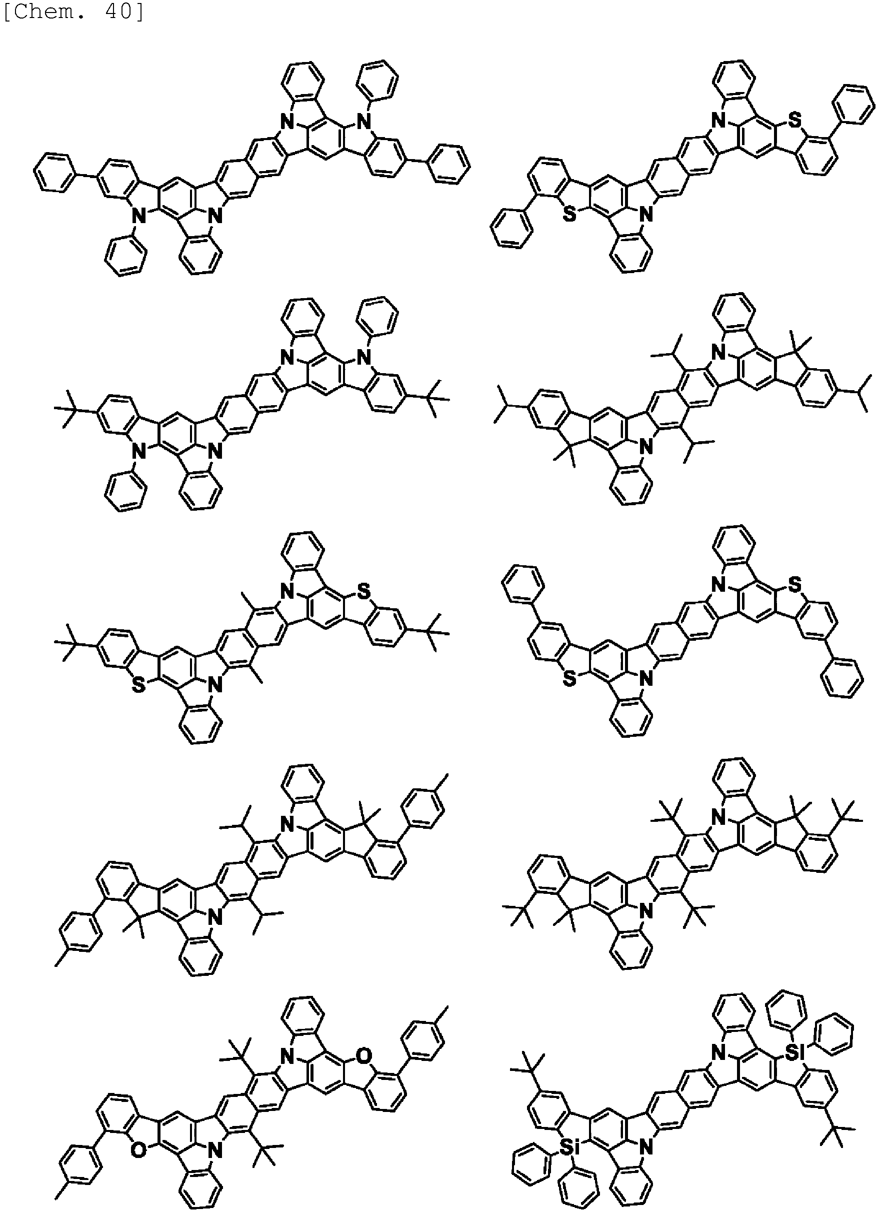

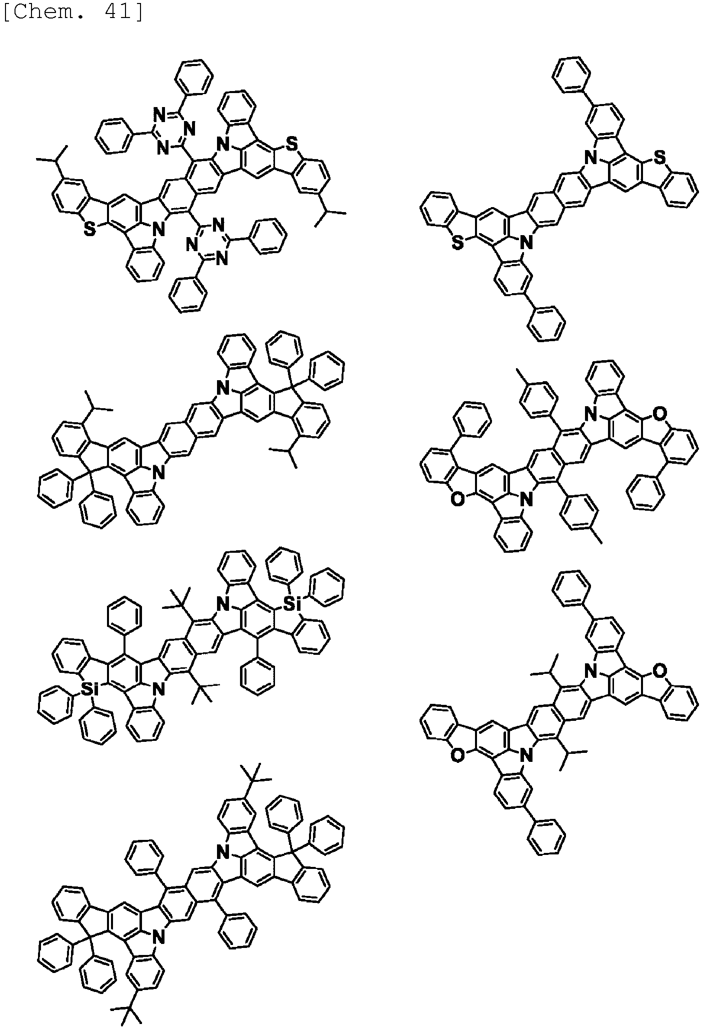

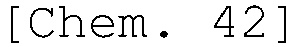

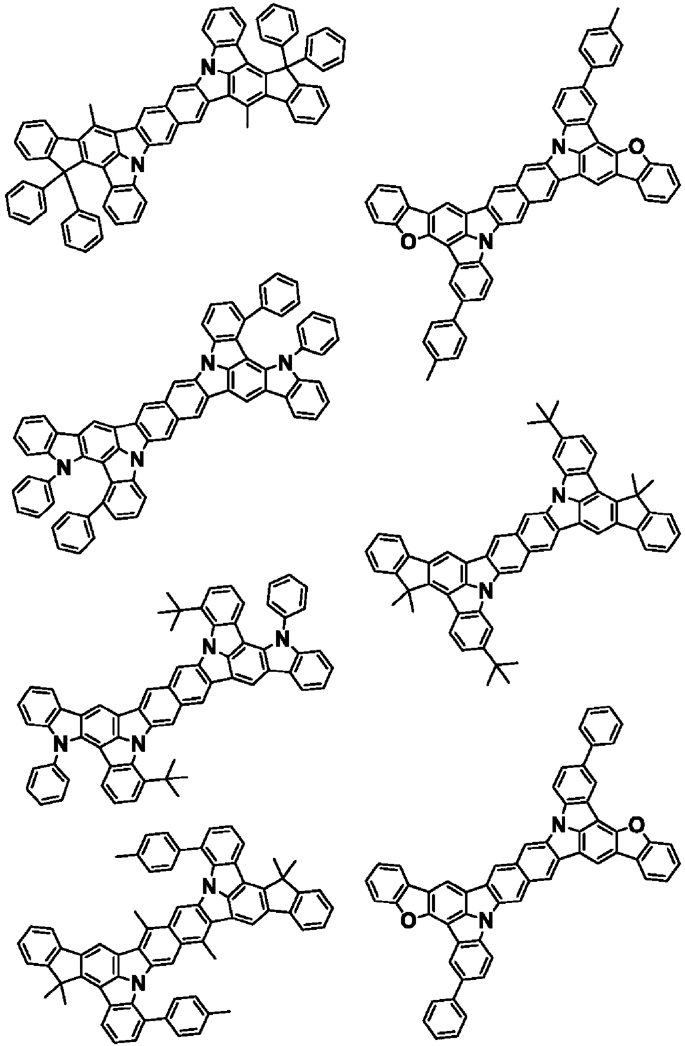

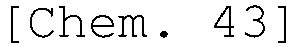

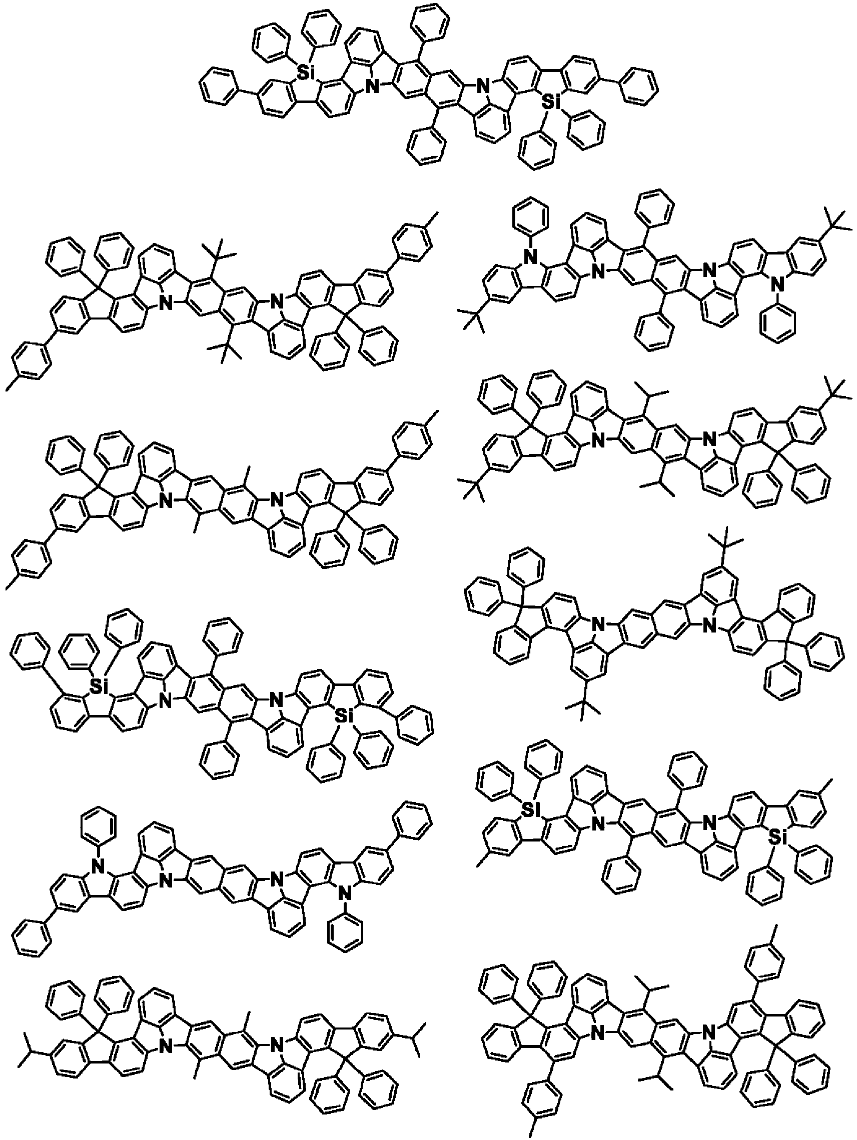

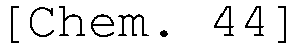

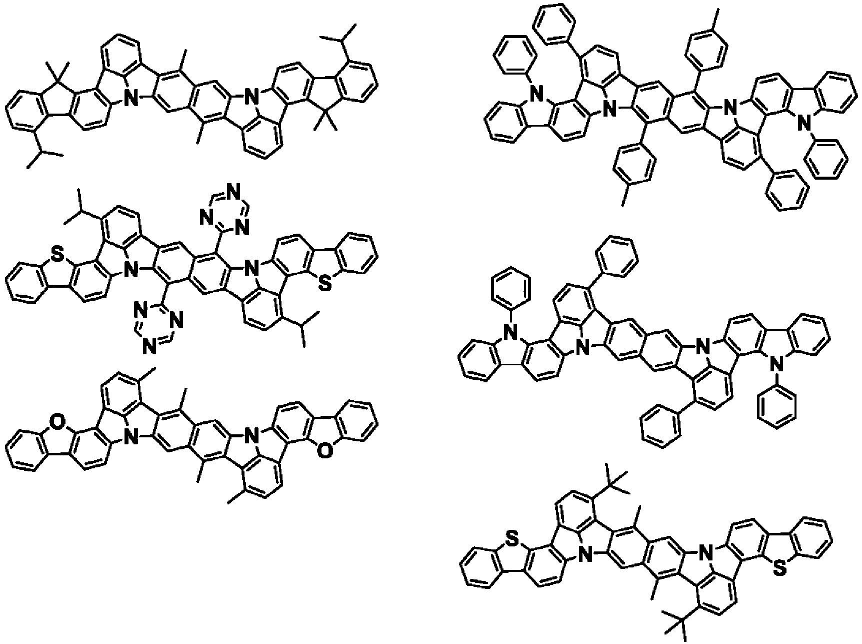

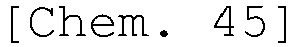

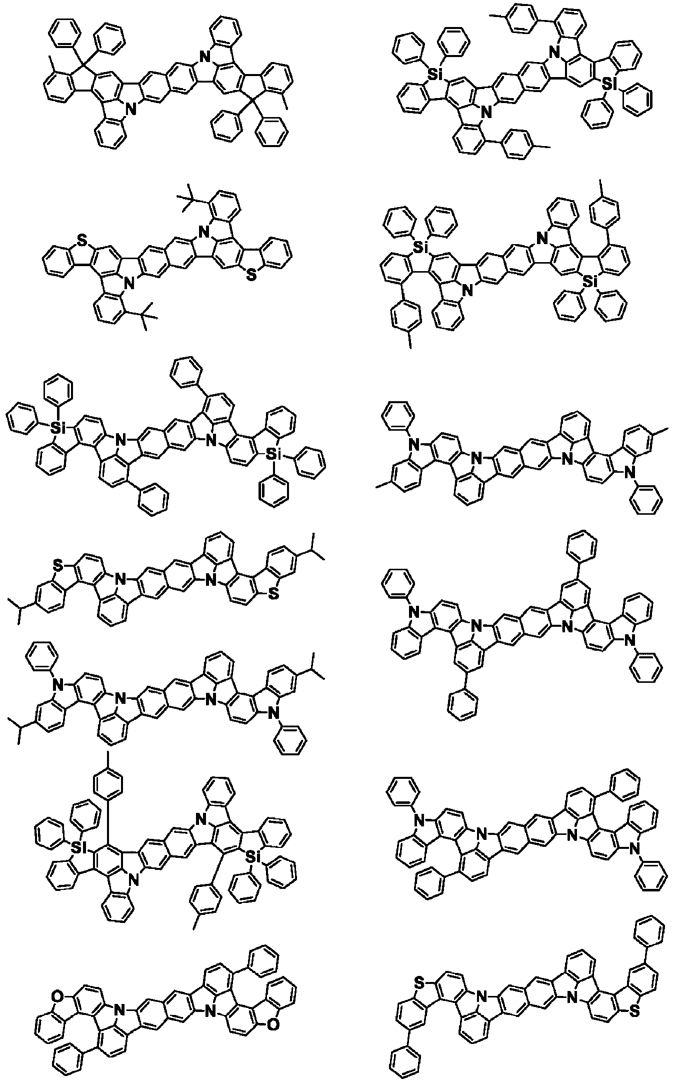

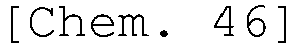

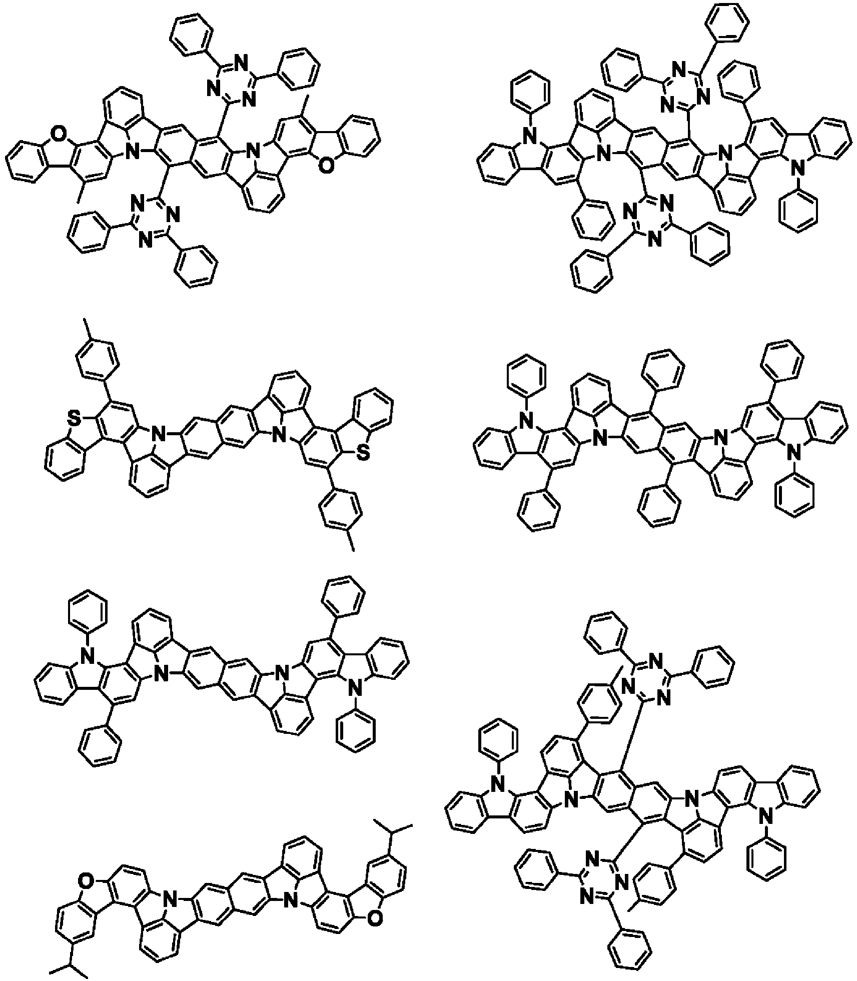

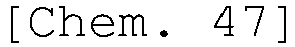

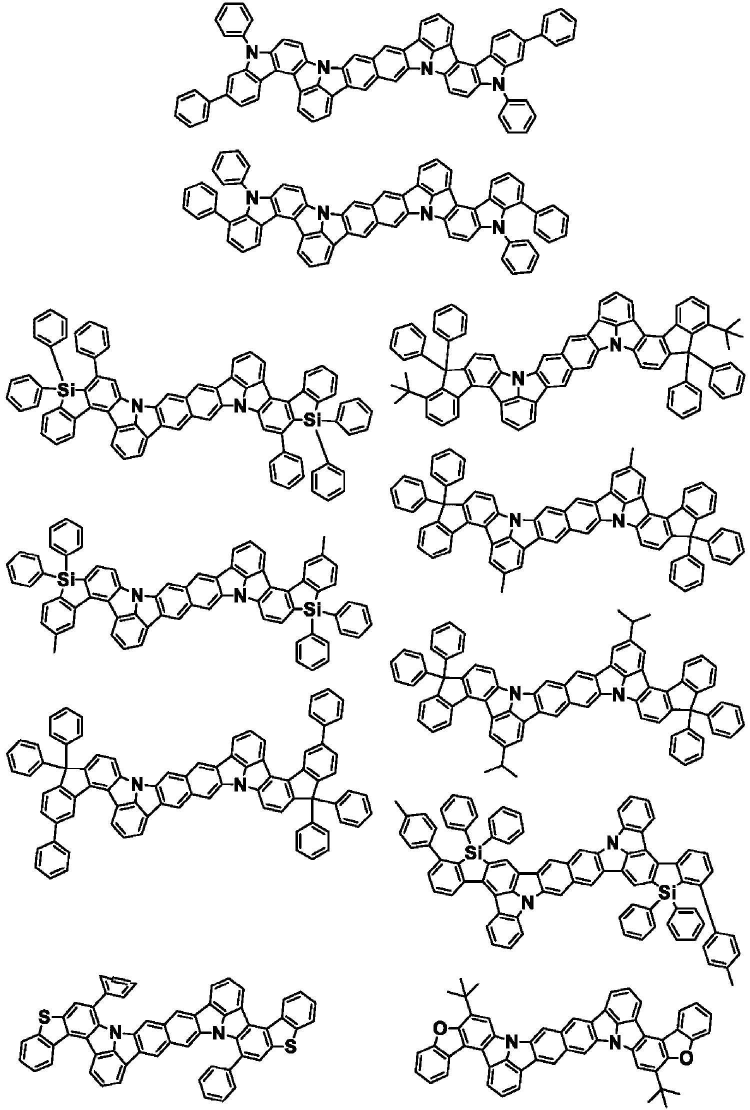

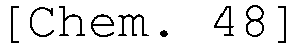

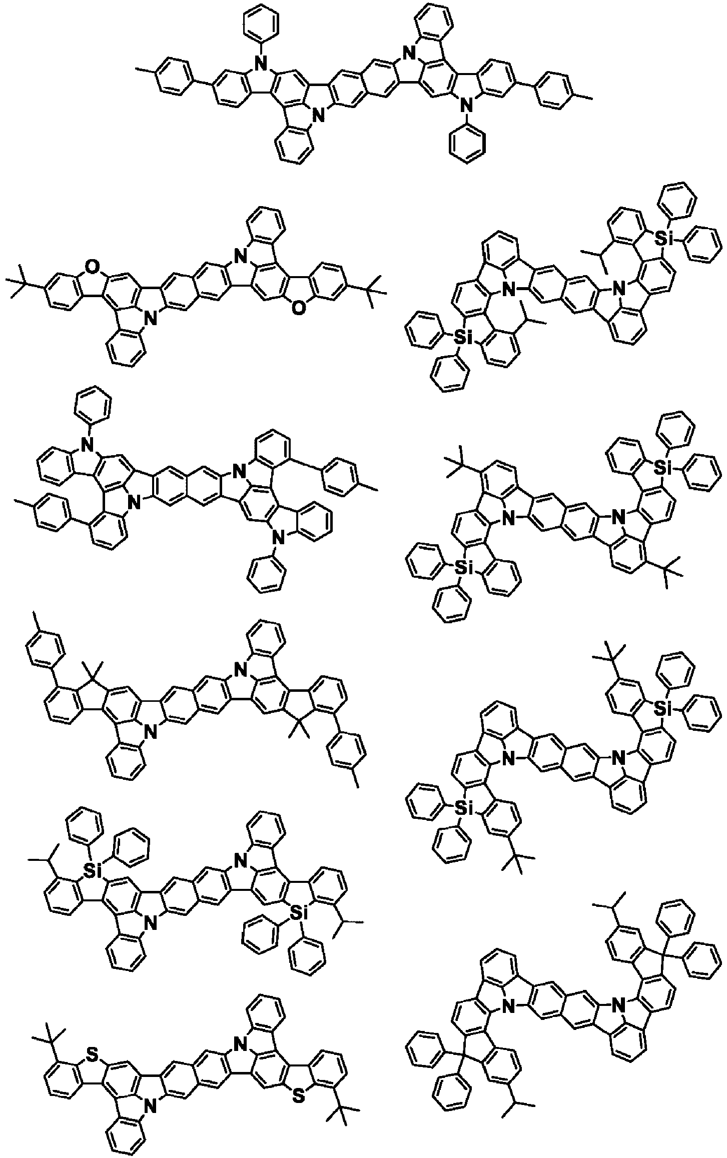

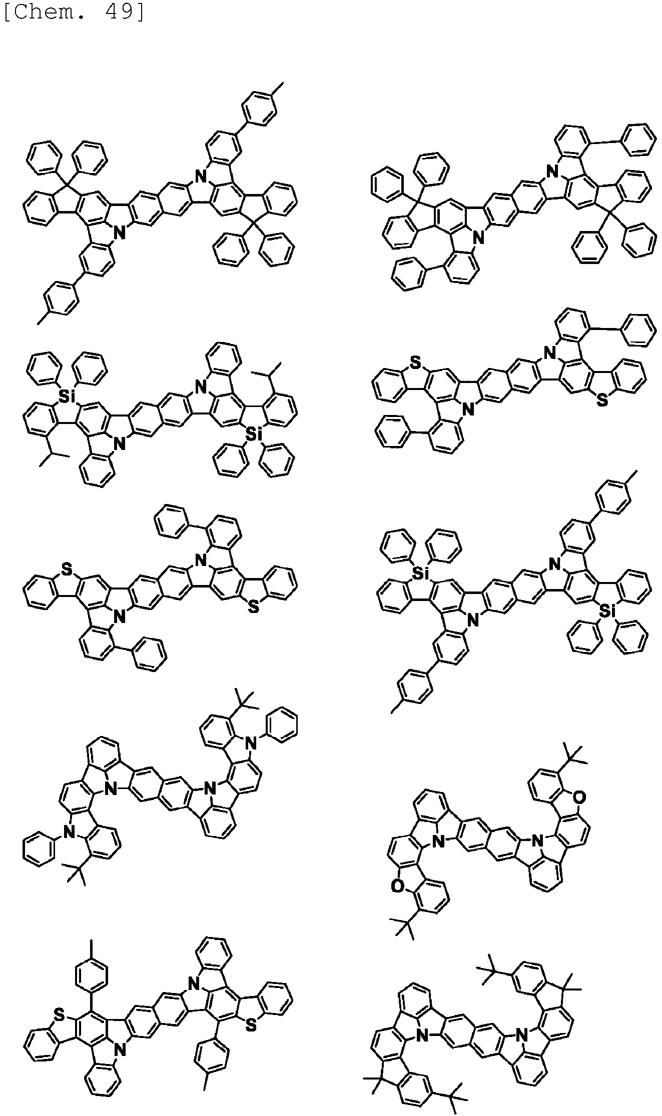

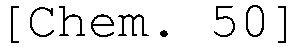

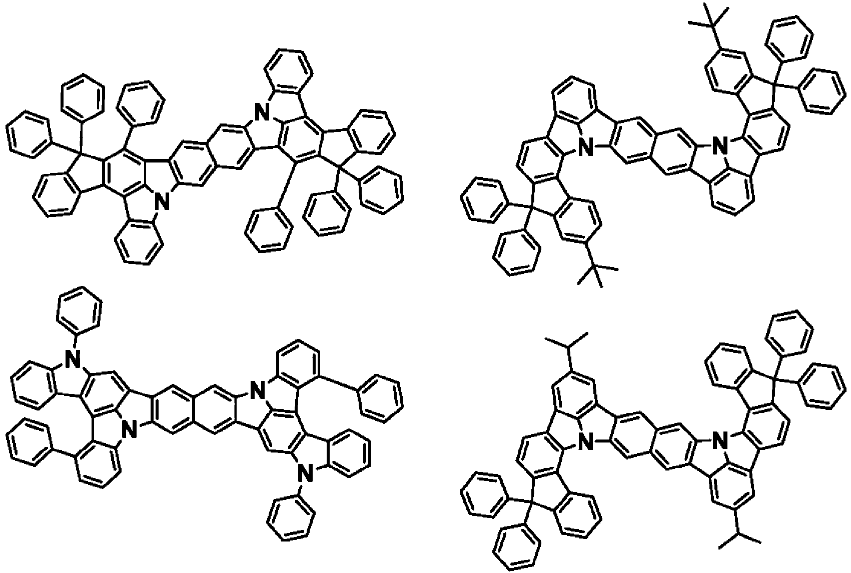

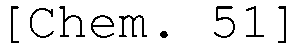

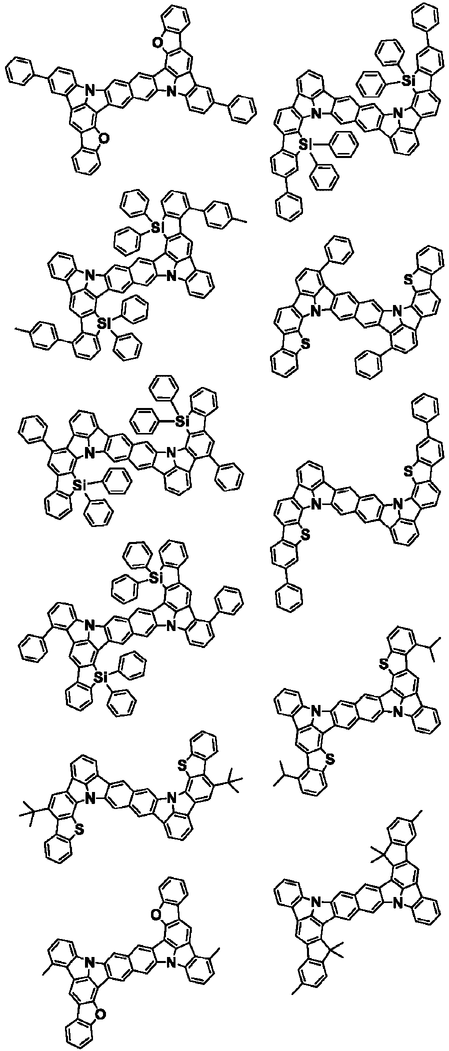

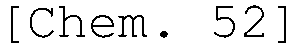

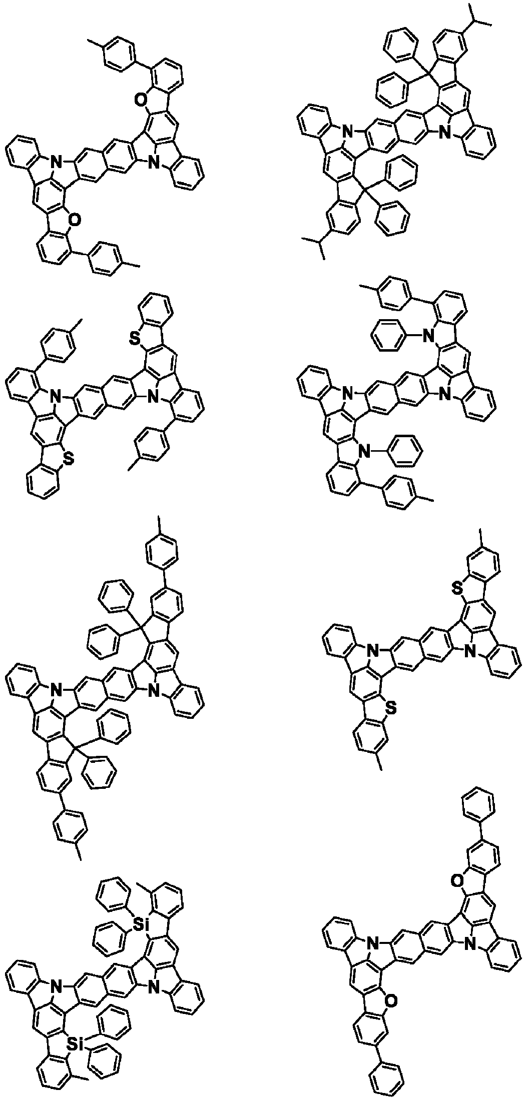

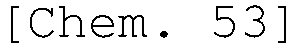

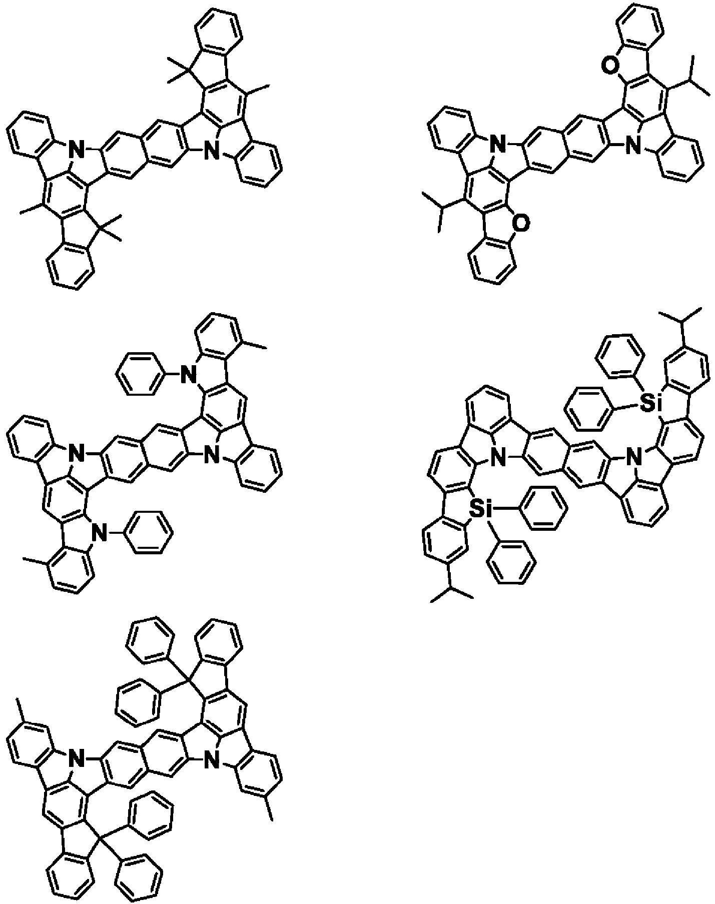

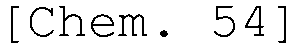

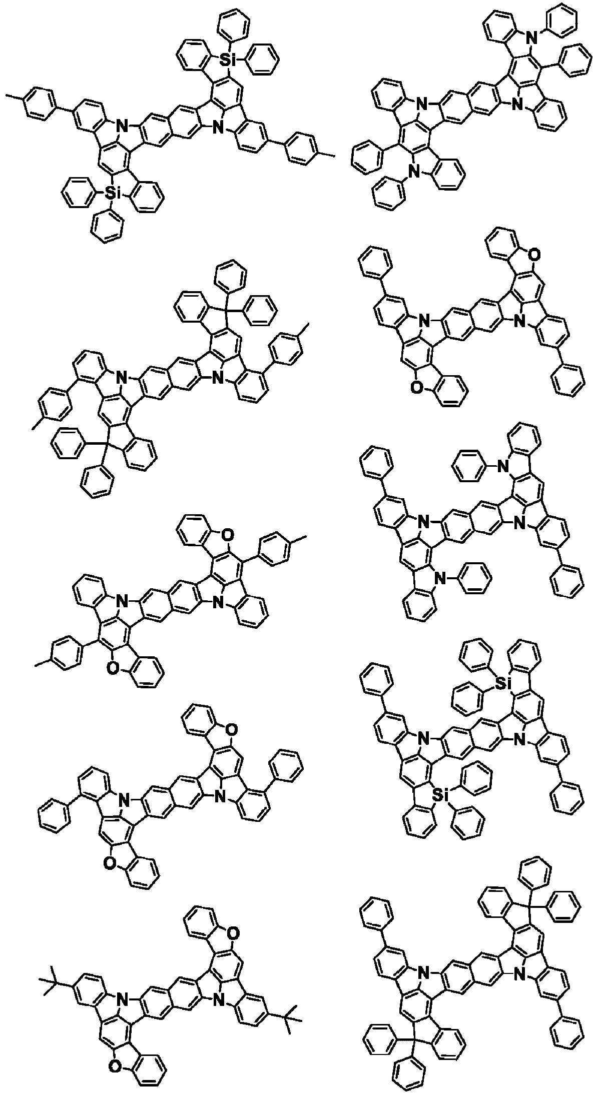

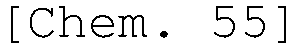

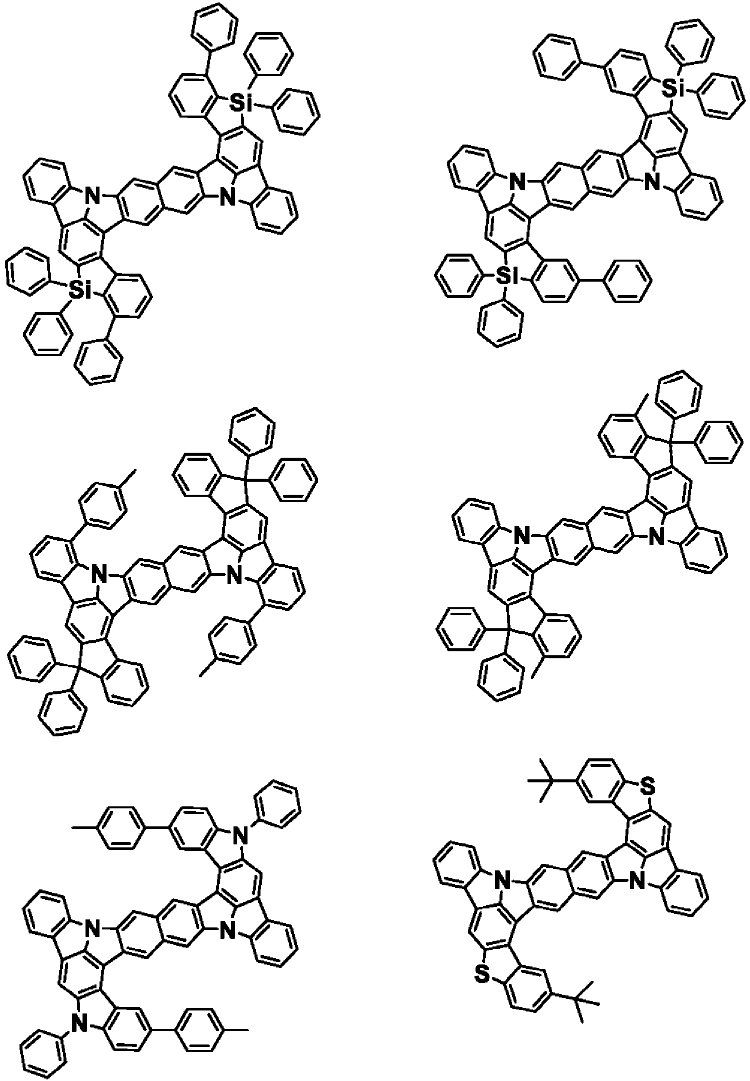

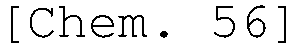

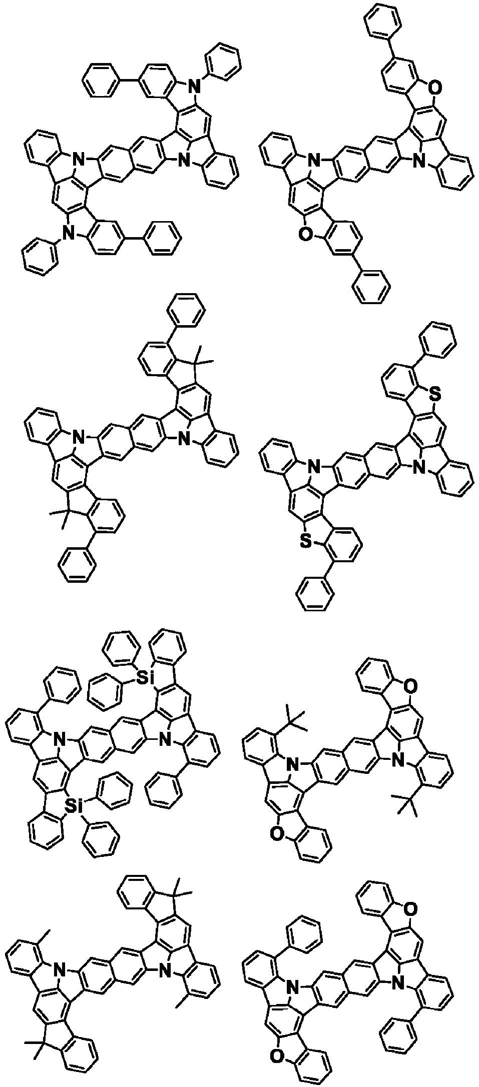

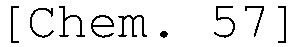

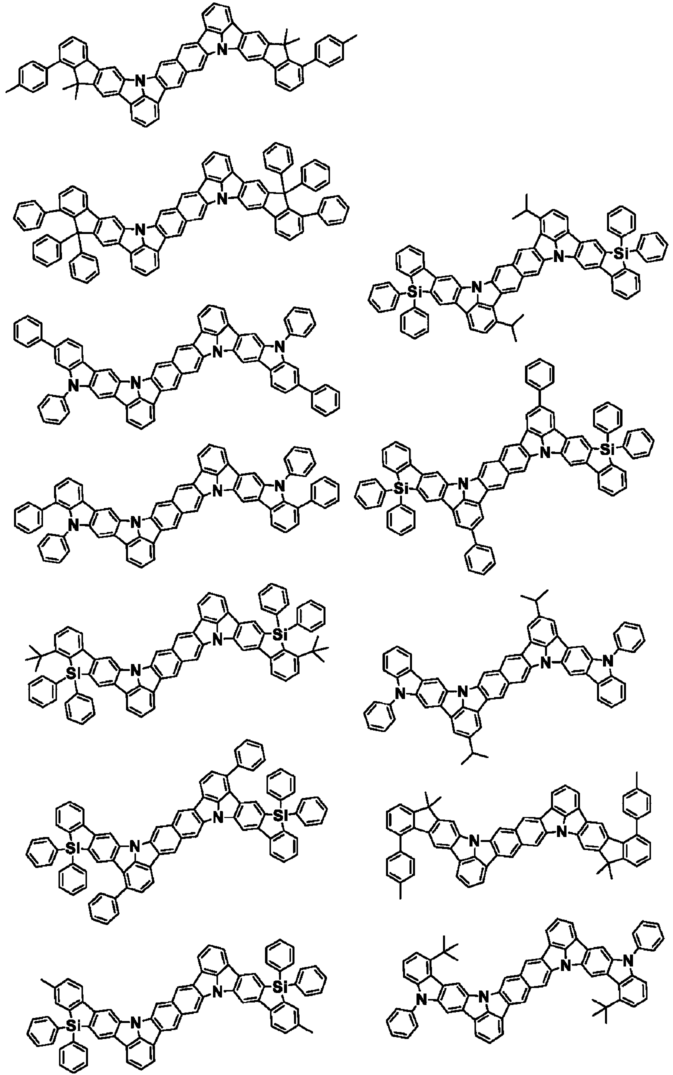

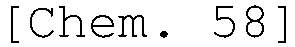

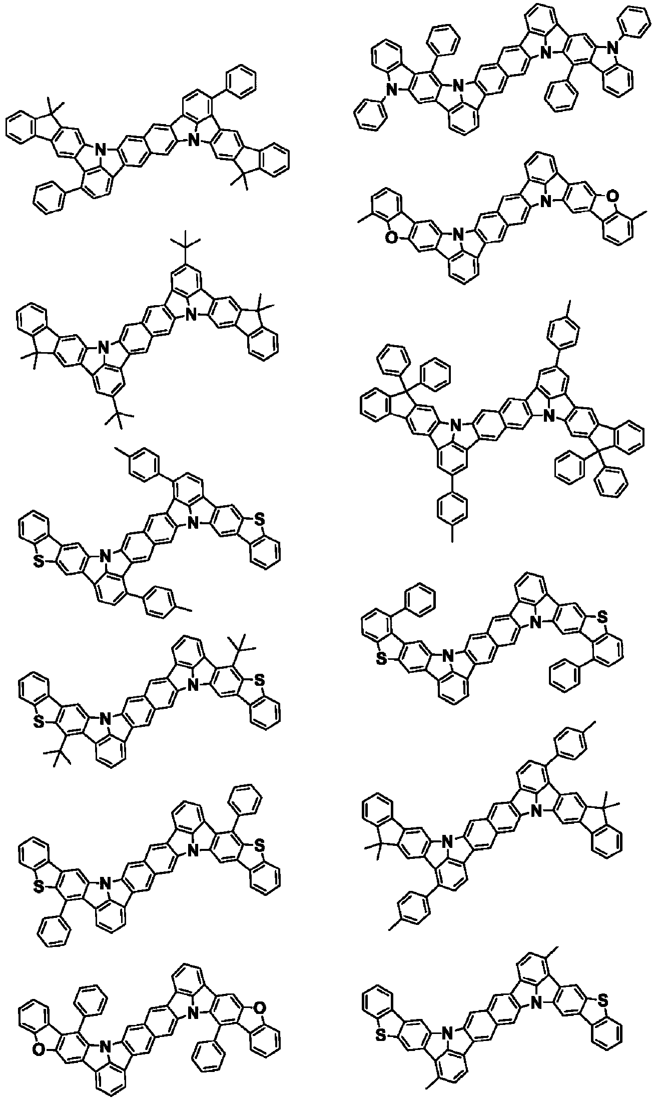

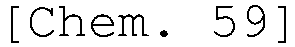

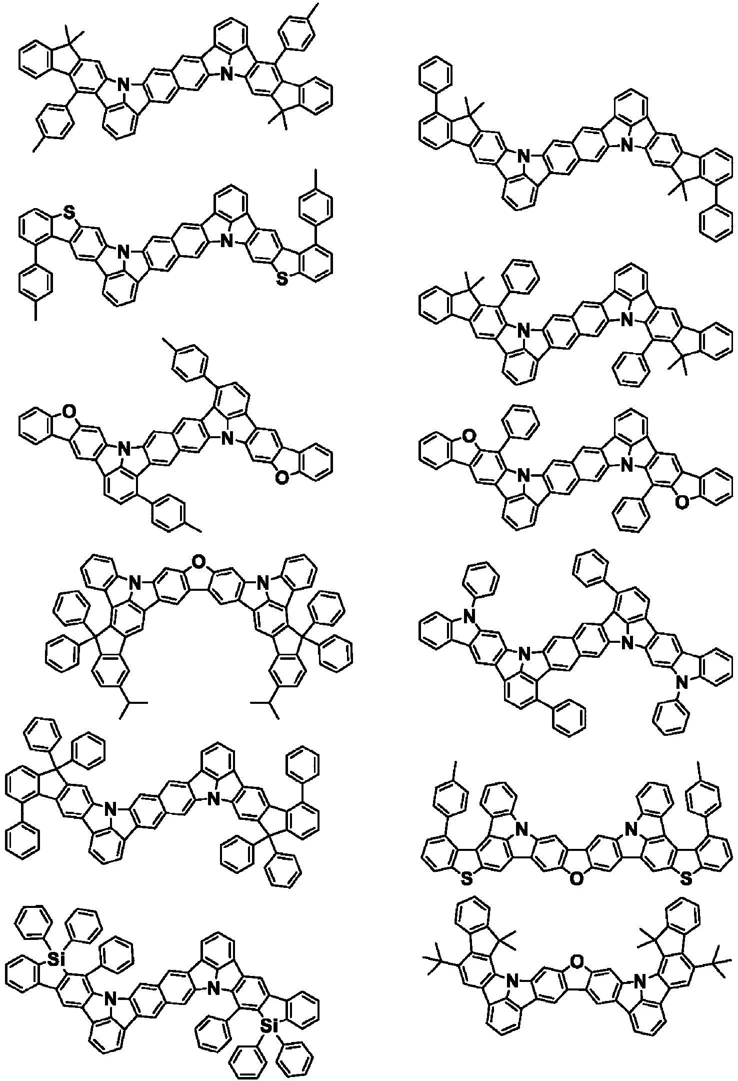

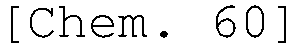

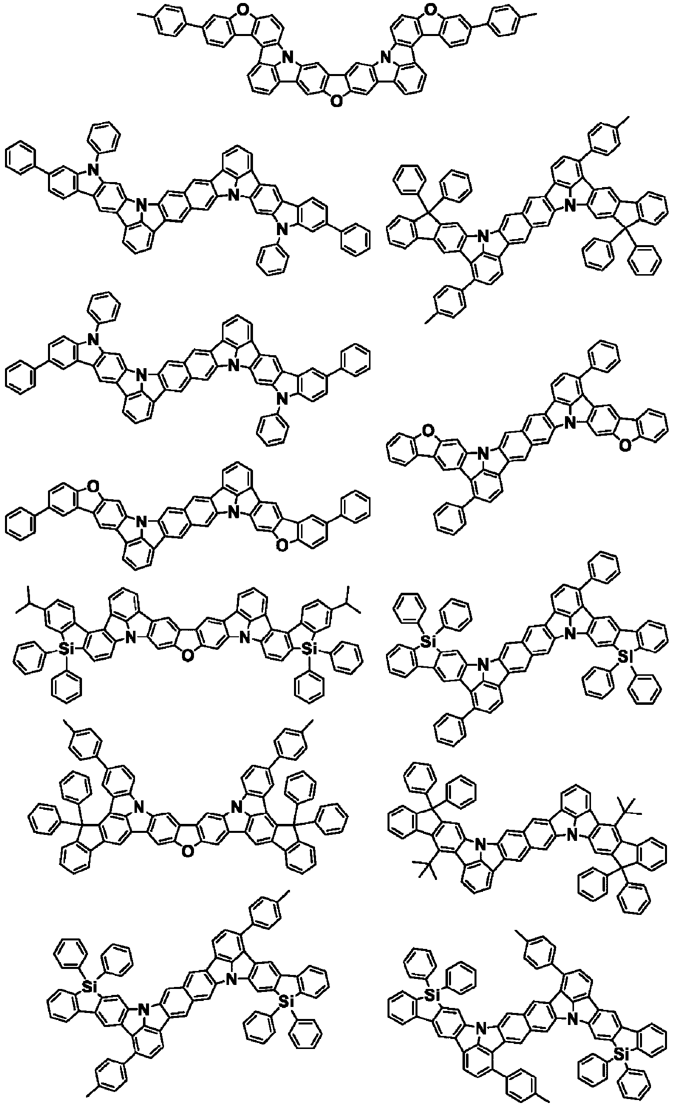

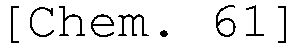

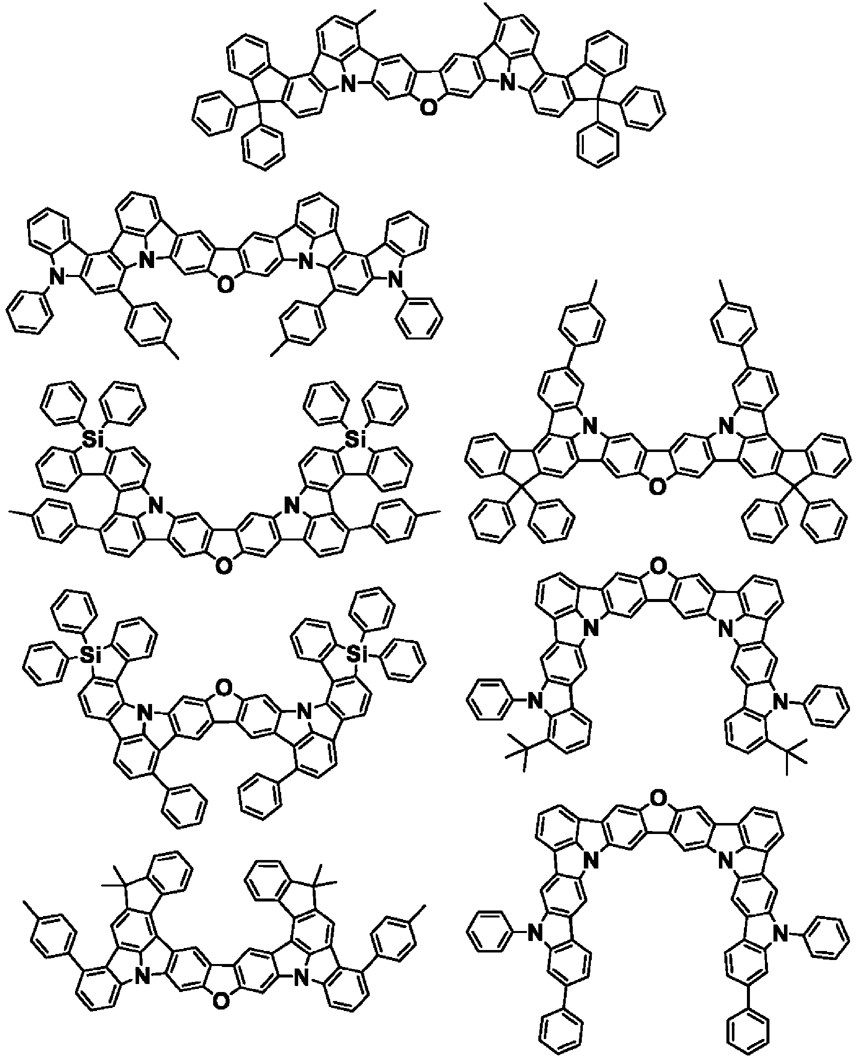

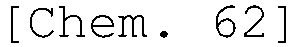

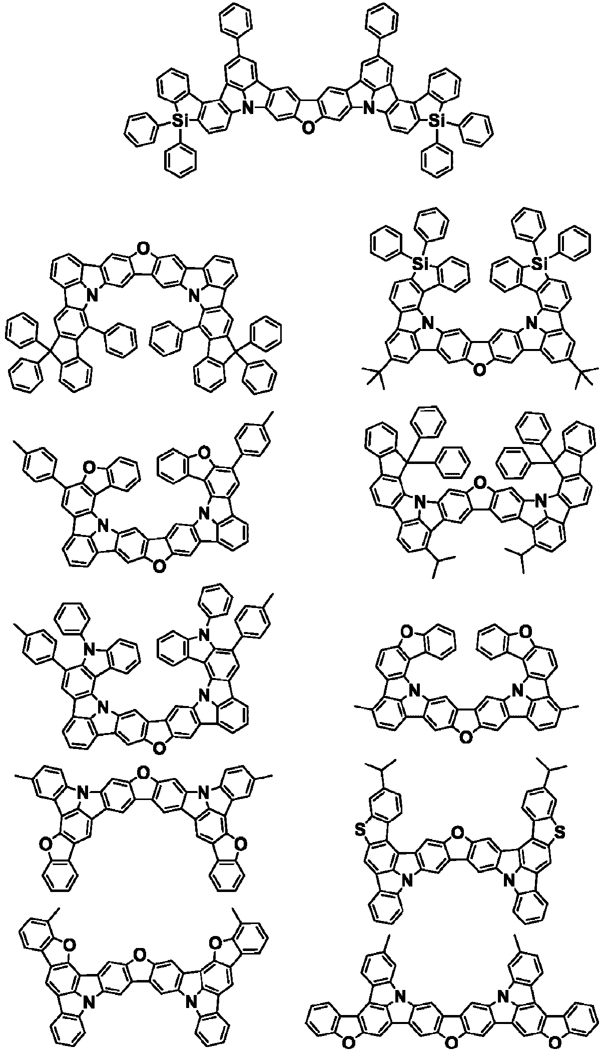

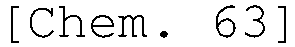

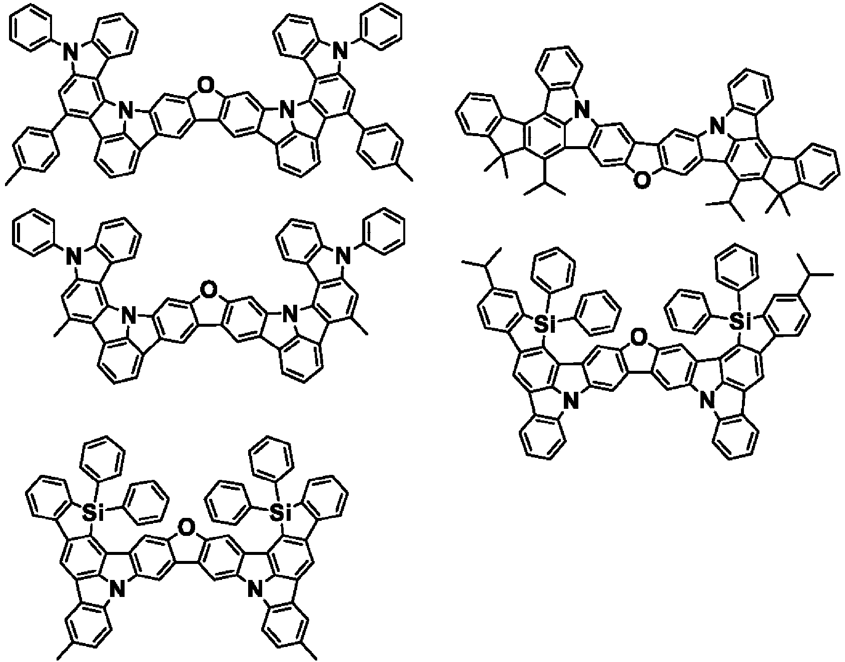

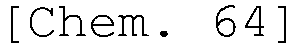

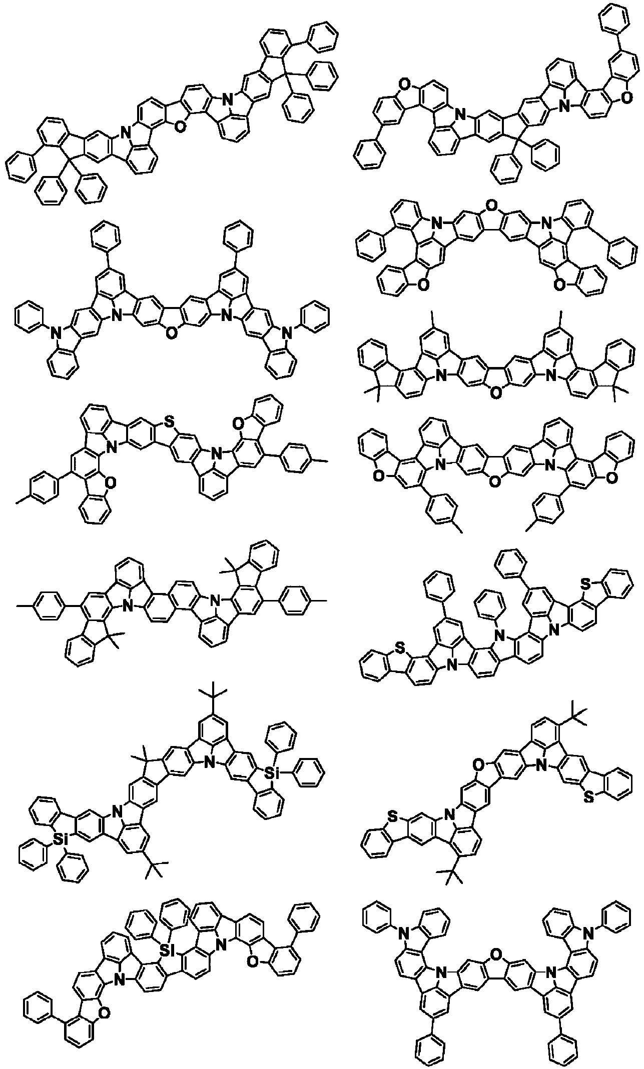

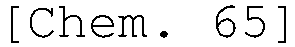

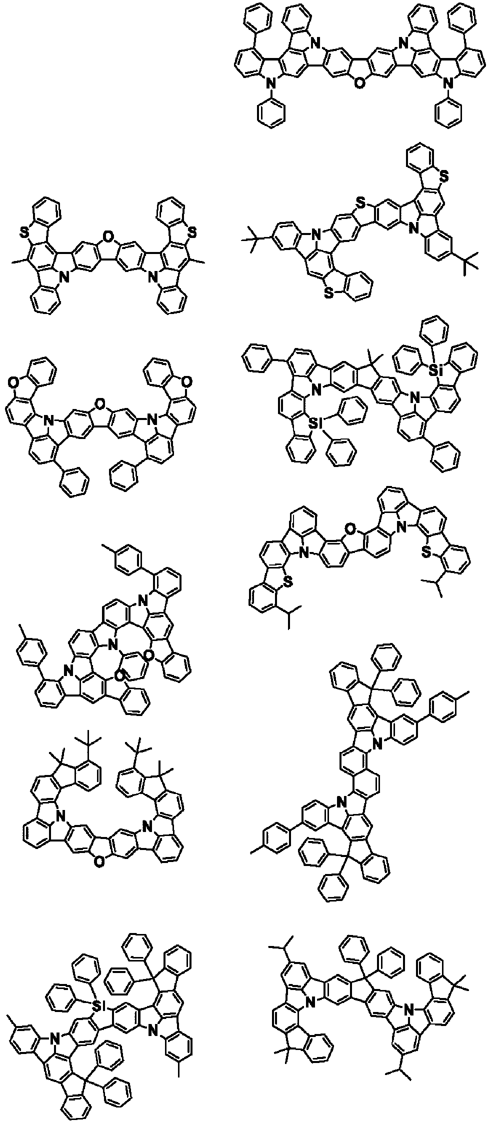

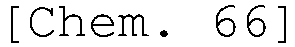

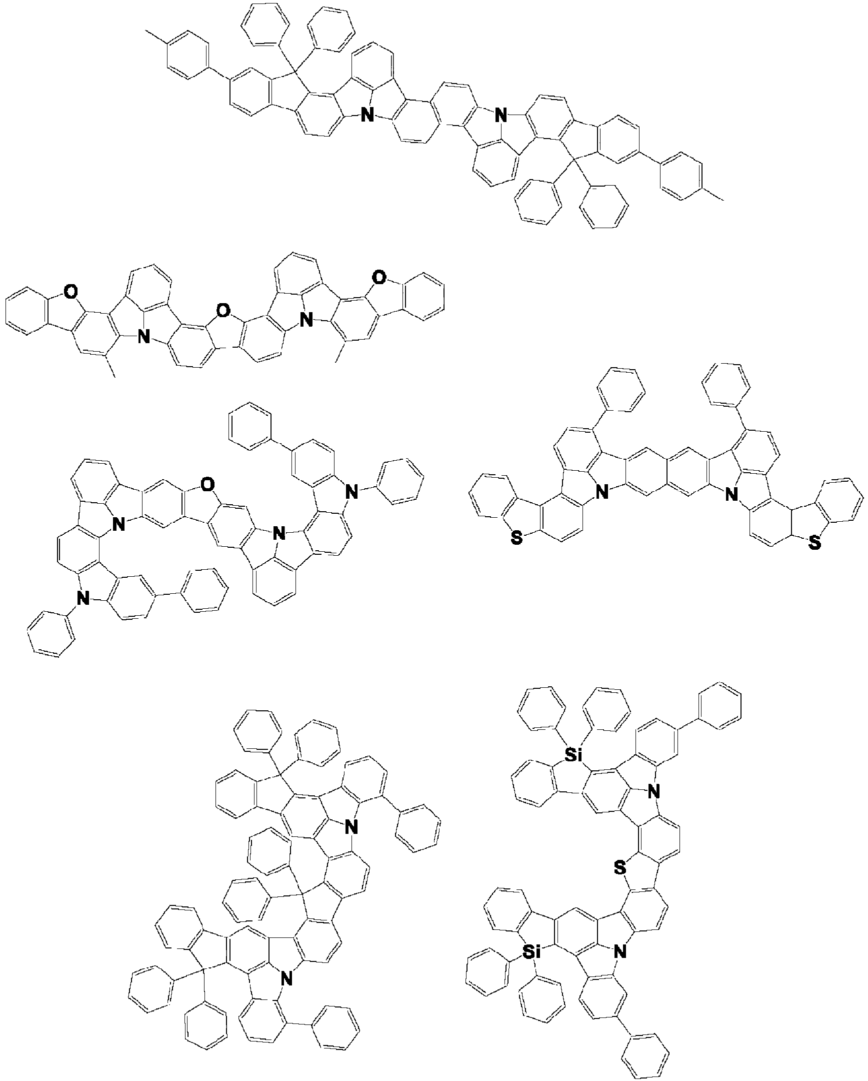

Among the organic compounds according to the present invention, the synthetic route of the organic compound having a central skeleton represented by general formula (1-1) will be described in detail in EXAMPLES given later. Among the organic compounds according to the present invention, the synthetic route of the organic compound having a central skeleton represented by general formula (1-2) is, for example, the following synthetic route, but is not limited thereto.

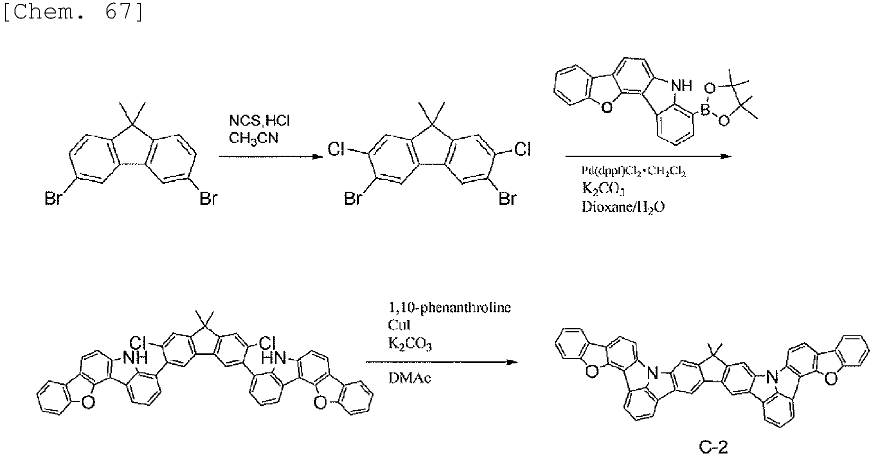

The organic compound according to the present invention preferably further has the following features.

(A) In general formula (1-1), at least four of the bonding sites R^{a} to R^{h} are preferably bonded to general formula (2-1), (2-2), (2-4), (2-5), (2-6), (2-7), (2-8), (2-9), or (2-10).
(B) Among organic compounds having a central skeleton represented by general formula (1-2), general formula (1-2) is preferably a fluorene skeleton.
(C) An organic compound having a central skeleton represented by general formulas (1-1) and (1-2) preferably has a bulky substituent.

Hereinafter, these features will be described.

(A) In general formula (1-1), at least four of the bonding sites R^{a} to R^{h} are preferably bonded to general formula (2-1), (2-2), (2-4), (2-5), (2-6), (2-7), (2-8), (2-9), or (2-10).

Among organic compounds having the feature (A), it is more preferable to bond to (2-1), (2-5), (2-6), (2-7), or (2-8). The organic compound according to the present invention is preferably represented by general formula (3-6) or (3-7).

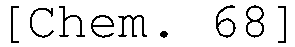

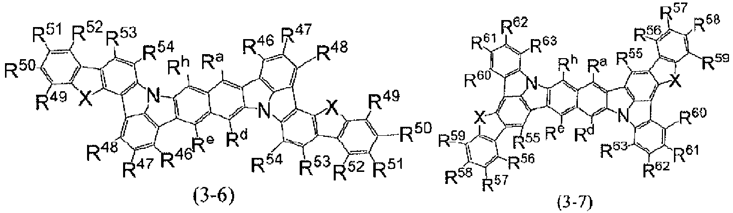

In general formulas (3-6) and (3-7), bonding sites R^{a}, R^{d}, R^{e}, and R^{h} are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. R⁴⁶ to R⁶³ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

In general formulas (3-6) and (3-7), R⁴⁶ to R⁶³ may be hydrogen atoms, and R⁴⁶ to R⁶³ may each independently be selected from the group consisting of a hydrogen atom, a substituted or unsubstituted alkyl group having 1 to 20 carbon atoms, a substituted or unsubstituted aryl group having 6 to 20 carbon atoms, and a substituted or unsubstituted heterocyclic group having 3 to 24 carbon atoms.

The organic compounds having the feature (A), among organic compounds having a central skeleton represented by general formula (1-1) among the organic compounds according to the present invention, are organic compounds having particularly high oscillator strength. The oscillator strength of the organic compounds according to the present invention was determined using Gaussian 16 (Gaussian 16, Revision C.01, M. J. Frisch, et al, Gaussian, Inc., Wallingford CT, 2019.), which is molecular orbital calculation software produced by Gaussian, Inc., USA. The results are shown in Table 2 and Table 3.

### [Table 2]

**Table 2**

| Compound | Oscillator strength | Compound | Oscillator strength |
|---|---|---|---|
| A-1 | 0.24 | A-7 | 0.31 |
| A-2 | 0.12 | A-8 | 0.35 |
| A-3 | 0.08 | A-9 | 0.17 |
| A-4 | 0.13 | A-10 | 0.12 |
| A-5 | 0.23 | Ref-1 | 0.12 |
| A-6 | 0.25 | Ref-2 | 0.10 |

Compounds A-1 to A-10 are the following compounds.

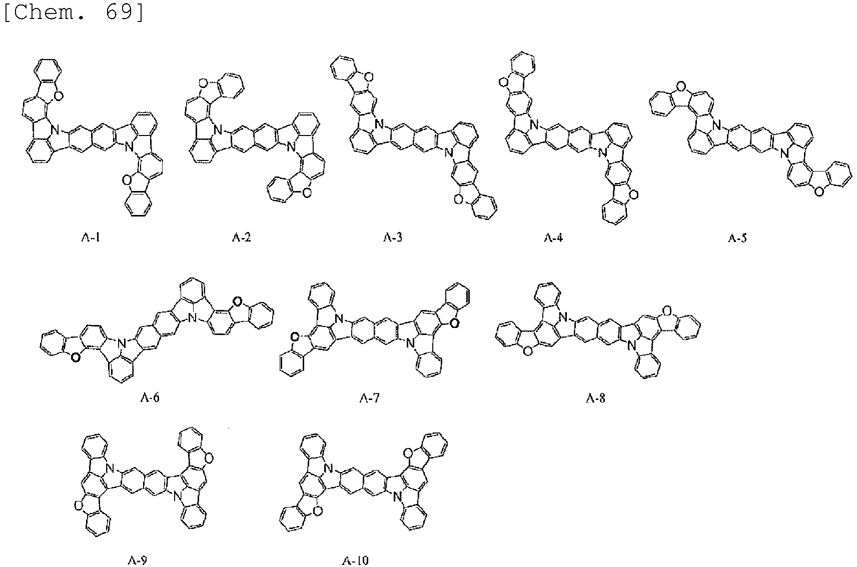

Table 2 shows that the oscillator strength values of compounds A-1, A-2, and A-4 to A-10 are equal to or larger than the oscillator strength values of compound Ref-1 and compound Ref-2. In particular, the oscillator strength of compounds A-1, A-5, A-6, A-7, and A-8 is especially high in the present invention. The organic compounds represented as compounds A-1, A-2, and A-4 to A-10 are each a compound in which general formula (2-1), (2-2), (2-4), (2-5), (2-6), (2-7), (2-8), (2-9), or (2-10) is bonded to the central skeleton represented by general formula (1-1).

### [Table 3]

**Table 3**

| Compound | Oscillator strength | Compound | Oscillator strength |
|---|---|---|---|
| B-1 | 0.22 | B-7 | 0.26 |
| B-2 | 0.13 | B-8 | 0.26 |
| B-3 | 0.11 | B-9 | 0.19 |
| B-4 | 0.14 | B-10 | 0.15 |
| B-5 | 0.19 | Ref-1 | 0.12 |
| B-6 | 0.24 | Ref-2 | 0.10 |

Compounds B-1 to B-10 are the following compounds.

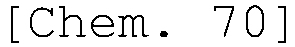

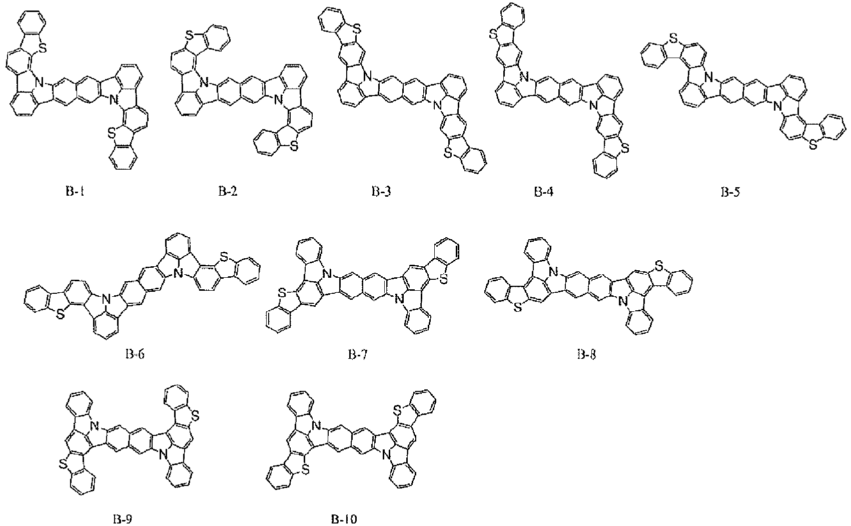

Table 3 shows that the oscillator strength values of compounds B-1, B-2, and B-4 to B-10 are equal to or larger than the oscillator strength values of compound Ref-1 and compound Ref-2. In particular, the oscillator strength of compounds B-1, B-5, B-6, B-7, B-8, and B-9 is especially high in the present invention. The organic compounds represented as compounds B-1, B-2, and B-4 to B-10 are each a compound in which general formula (2-1), (2-2), (2-4), (2-5), (2-6), (2-7), (2-8), (2-9), or (2-10) is bonded to the central skeleton represented by general formula (1-1).

The relationship between oscillator strength and quantum yield (luminous efficiency) will be described. As described in paragraph [0262] of Japanese Patent Laid-Open No. 2020-47930 and paragraph [0035] of Japanese Patent Laid-Open No. 2022-46999, compounds with high oscillator strength are known to exhibit high quantum yield (luminous efficiency).

Therefore, from the viewpoint of oscillator strength, general formula (2-1), (2-2), (2-4), (2-5), (2-6), (2-7), (2-8), (2-9), or (2-10) is preferably bonded to the central skeleton represented by general formula (1-1), a compound in which general formula (2-1), (2-5), (2-6), (2-7), or (2-8) is bonded is more preferred, and a compound in which general formula (2-6), (2-7), or (2-8) is bonded is particularly preferred. X is preferably an oxygen atom or a sulfur atom, more preferably an oxygen atom.

(B) Among organic compounds having a central skeleton represented by general formula (1-2), general formula (1-2) is preferably a fluorene skeleton.

Among organic compounds having the feature (B), an organic compound represented by general formula (4-2) is more preferred.

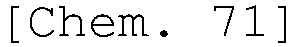

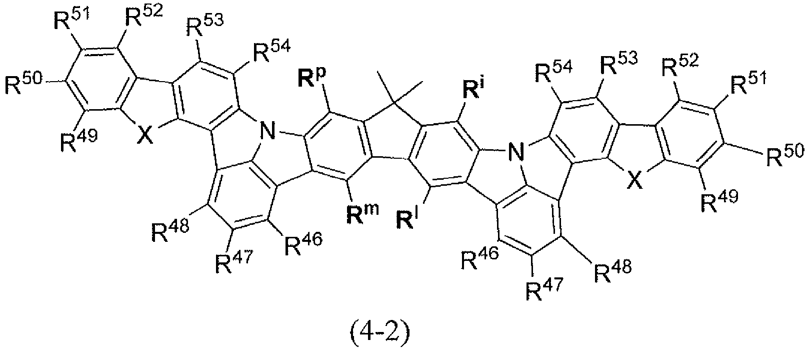

In general formula (4-2), in general formula (4-2), bonding sites Rⁱ, R^{l}, R^{m}, and R^{p} are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. R⁴⁶ to R⁵⁴ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

The organic compounds having the feature (B), among organic compounds having a central skeleton represented by general formula (1-2) among the organic compounds according to the present invention, are organic compounds having particularly high oscillator strength. The oscillator strength of compounds C-1 to C-14 was determined by the method mentioned above. The results are shown in Table 4.

### [Table 4]

**Table 4**

| Compound | Oscillator strength | Compound | Oscillator strength |
|---|---|---|---|
| C-1 | 0.14 | C-9 | 0.02 |
| C-2 | 0.43 | C-10 | 0.13 |
| C-3 | 0.02 | C-11 | 0.18 |
| C-4 | 0.03 | C-12 | 0.03 |
| C-5 | 0.01 | C-13 | 0.02 |
| C-6 | 0.01 | C-14 | 0.01 |
| C-7 | 0.14 | Ref-1 | 0.12 |
| C-8 | 0.05 | Ref-2 | 0.10 |

Compounds C-1 to C-14 are the following compounds.

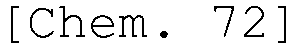

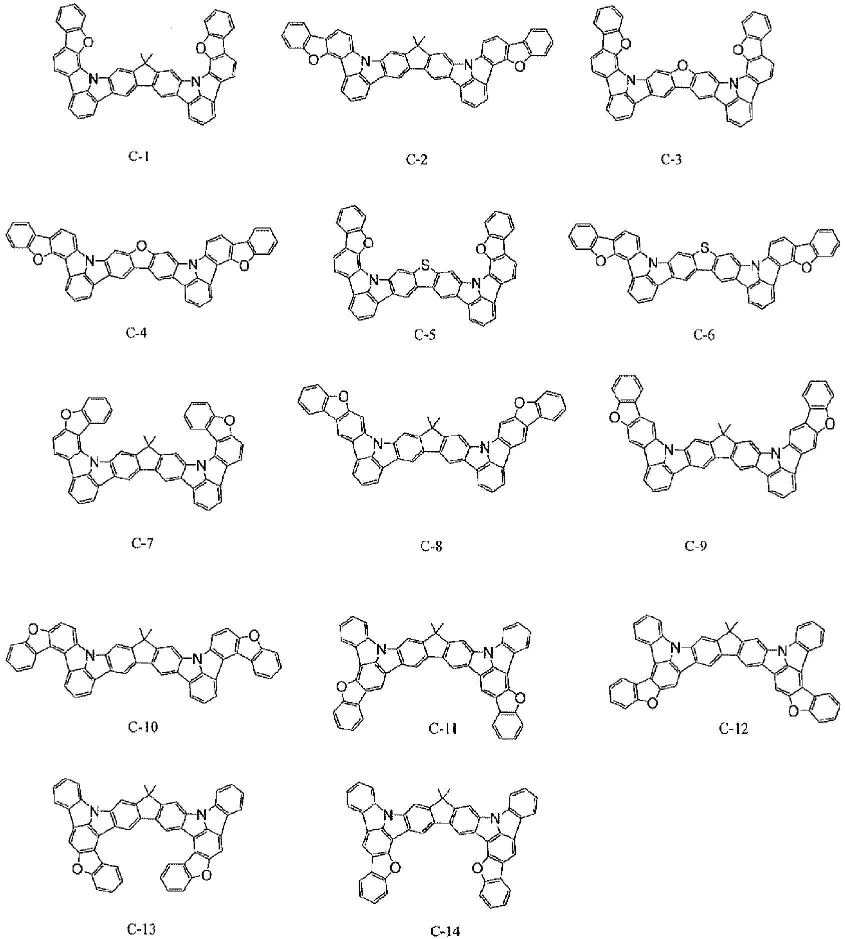

Table 4 shows that among organic compounds having a central skeleton represented by general formula (1-2), particularly when the central skeleton represented by general formula (1-2) is a fluorene skeleton, the organic compound according to the present embodiment tends to exhibit high oscillator strength. This may be explained as follows: the oscillator strength of the fluorene skeleton itself is high, so that the organic compound according to the present invention tends to exhibit high oscillator strength when general formula (1-2) is a fluorene skeleton.

Among the organic compounds according to the present embodiment, those represented by general formula (4-2) exhibit particularly high oscillator strength. This may be explained as follows: the direction of the oscillator moment of fluorene as a central skeleton is in line with the extension direction of a skeleton further fused to a carbazole skeleton, so that particularly high oscillator strength is exhibited.

(C) An organic compound having a central skeleton represented by general formulas (1-1) and (1-2) preferably has a bulky substituent.

Among the organic compounds according to the present invention, an organic compound having a central skeleton represented by general formulas (1-1) and (1-2) preferably has a bulky substituent. Having the feature (C) provides an organic compound that exhibits higher oscillator strength. In the invention according to the present embodiment, having a bulky substituent is preferred because solvent solubility can be improved.

In the organic compound according to the present embodiment, the bulky substituent is a substituted or unsubstituted alkyl group having 1 to 6 carbon atoms, a substituted or unsubstituted aryl group having 6 to 18 carbon atoms, or a substituted or unsubstituted heterocyclic group having 5 to 12 carbon atoms, and is preferably a substituted or unsubstituted alkyl group having 1 to 6 carbon atoms or a substituted or unsubstituted aryl group having 6 to 18 carbon atoms. Specifically, it may be an iso-propyl group, a cyclohexyl group having a phenyl group, a phenyl group, a phenyl group having an alkyl group having 3 to 6 carbon atoms, a para-phenylene group composed of 2 to 3 phenyl groups, a para-phenylene group composed of 2 to 3 phenyl groups and having a tert-butyl group, a meta-phenylene group composed of 2 to 5 phenyl groups, or a meta-phenylene group composed of 2 to 5 phenyl groups and having a methyl group or an iso-propyl group. The phenyl group having an alkyl group having 3 to 6 carbon atoms may be a phenyl group having an iso-propyl group, a tert-butyl group, or a cyclohexyl group. The meta-phenylene group may be a structure in which phenyl groups are bonded linearly, or a structure in which they are bonded in a branched manner.

Table 5 shows the oscillator strength of compounds D-1 to D-20, which are organic compounds according to the present embodiment. The oscillator strength was determined by the method mentioned above.

### [Table 5]

**Table 5**

| Compound | Oscillator strength | Compound | Oscillator strength | Compound | Oscillator strength | Compound | Oscillator strength |
|---|---|---|---|---|---|---|---|
| D-1 | 0.41 | D-6 | 0.43 | 0-11 | 0.40 | D-16 | 0.32 |
| D-2 | 0.48 | D-7 | 0.45 | D-12 | 0.38 | D-17 | 0.42 |
| D-3 | 0.56 | D-8 | 0.42 | D-13 | 0.40 | D-18 | 0.43 |
| D-4 | 0.43 | D-9 | 0.30 | D-14 | 0.38 | D-19 | 0.37 |
| D-5 | 0.47 | D-10 | 0.37 | D-15 | 0.34 | D-20 | 0.38 |

Compounds D-1 to D-20 are the following compounds.

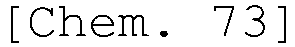

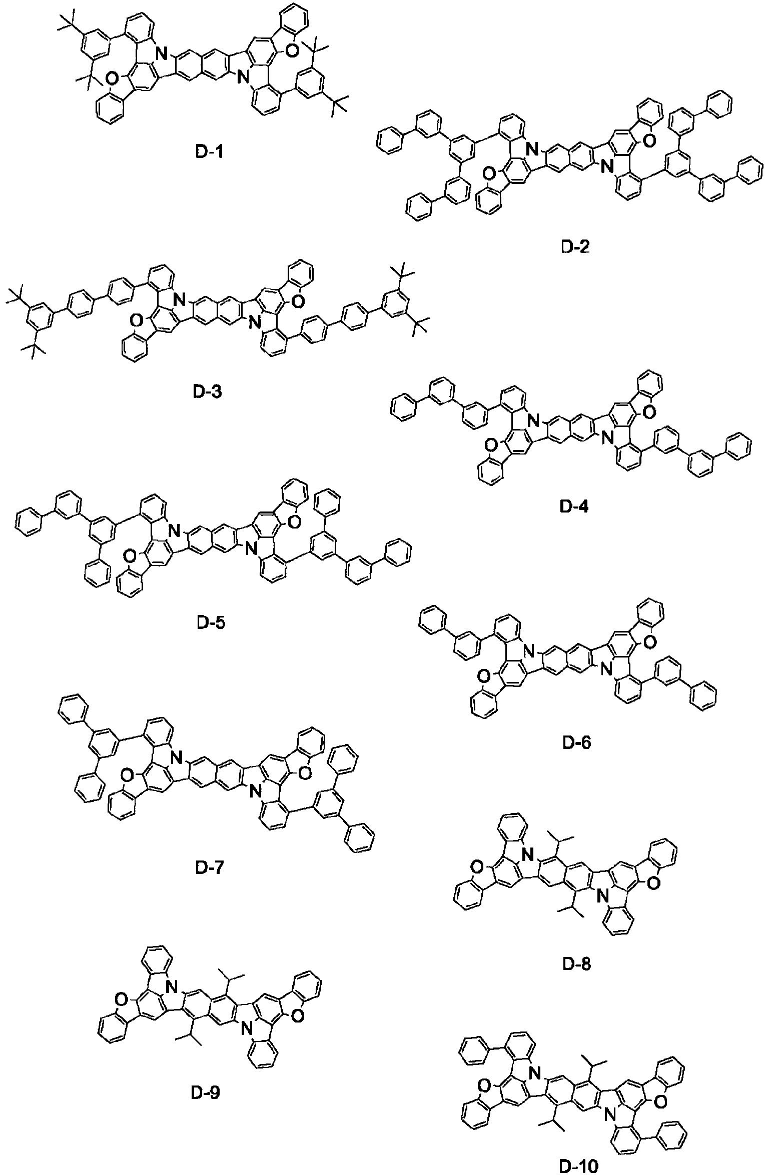

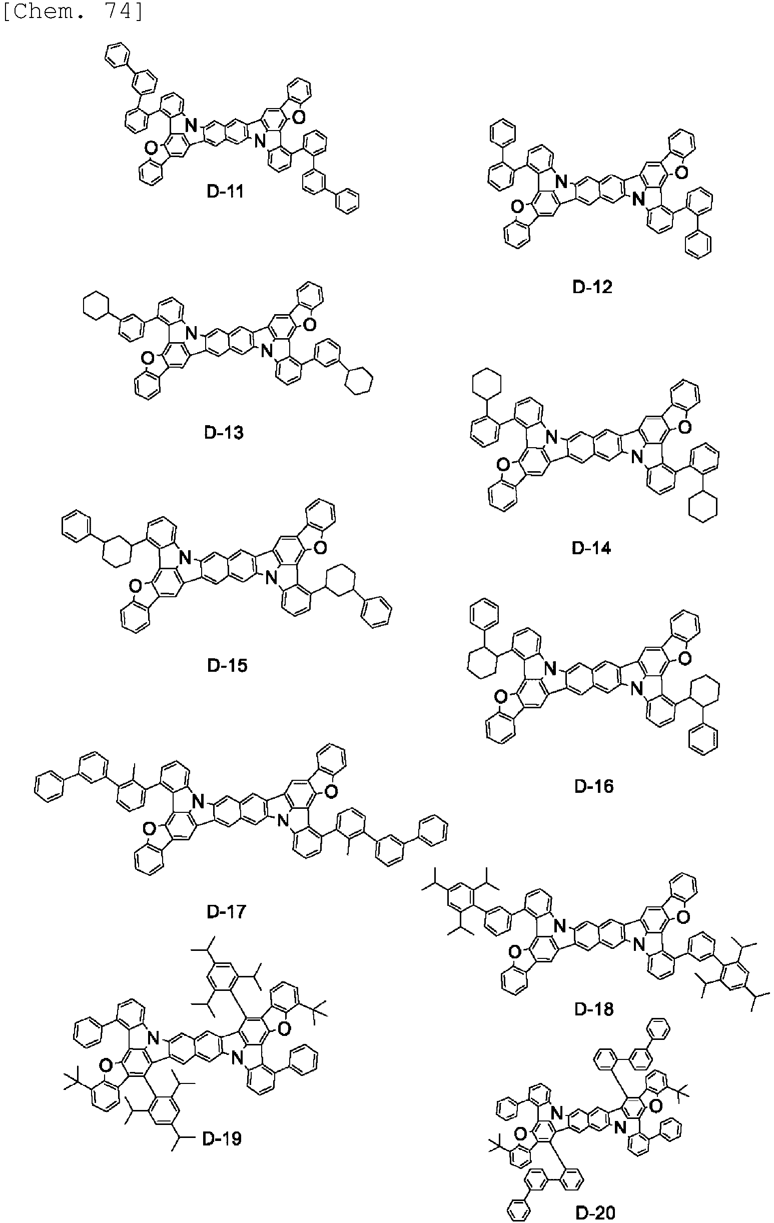

Table 5 shows that organic compounds according to the present embodiment exhibit high oscillator strength. Among the organic compounds according to the present embodiment, it is preferable to have a substituent in the longitudinal direction of an organic compound having a central skeleton represented by general formulas (1-1) and (1-2). This is because such a compound exhibits particularly high oscillator strength.

It follows from the foregoing that the organic compounds according to the present invention are organic compounds having high thermal stability. Furthermore, among the organic compounds according to the present invention, organic compounds having the feature (A) or (B) are organic compounds having particularly high oscillator strength.

### (2) Organic Light-Emitting Element

Next, an organic light-emitting element according to an embodiment of the present invention will be described. The organic light-emitting element according to an embodiment of the present invention includes a first electrode, a second electrode, and an organic compound layer disposed between the electrodes. One of the first electrode and the second electrode is an anode, and the other is a cathode. In the organic light-emitting element according to the present embodiment, the organic compound layer may be a single layer or a stack of a plurality of layers as long as the organic compound layer includes a light-emitting layer. The organic compound according to the present invention may be contained in the organic compound layer, and is preferably contained in the light-emitting layer. When the organic compound layer is a stack of a plurality of layers, the organic compound layer may include, in addition to the light-emitting layer, a hole injection layer, a hole transport layer, an electron blocking layer, a hole/exciton blocking layer, an electron transport layer, an electron injection layer, and the like. The light-emitting layer may be a single layer or a stack of a plurality of layers. When the light-emitting layer includes a plurality of layers, a charge generation layer may be disposed between the light-emitting layers. The charge generation layer may be composed of a compound having a LUMO (Lowest Unoccupied Molecular Orbital) energy level lower than the LUMO energy level of the hole transport layer, and the LUMO energy level of the charge generation layer may be lower than the HOMO energy level of the hole transport layer. The HOMO energy level and the LUMO energy level of the organic compound layer may be the HOMO energy level and the LUMO energy level of an organic compound having the highest weight ratio in the organic compound layer.

HOMO energy levels and LUMO energy levels closer to the vacuum level are described as being "higher". When the LUMO energy level of the charge generation layer is lower than the HOMO energy level of the hole transport layer, it indicates that the LUMO energy level of the charge generation layer is farther from the vacuum level than the HOMO energy level of the hole transport layer is.

In the present specification, the HOMO energy level and the LUMO energy level can be calculated using molecular orbital calculations. The molecular orbital calculations are performed by Density Functional Theory (DFT) or the like, and may be performed using, for example, the B3LYP functional and the 6-31G* basis set. The molecular orbital calculations can be performed using, for example, Gaussian 09 (Gaussian 09, Revision C.01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. Iyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010.).

The HOMO energy level and the LUMO energy level in the present specification can be calculated using an ionization potential and a band gap. The HOMO energy level can be estimated by measuring an ionization potential. The ionization potential can be measured with a measurement apparatus such as AC-3 after a compound to be measured has been dissolved in a solvent such as toluene or a vapor-deposited film of the compound to be measured has been formed on a substrate such as glass. The band gap can be measured by a measurement in which a compound to be measured is dissolved in a solvent such as toluene and irradiated with excitation light. The band gap can be determined by measuring the absorption edge of an absorption spectrum of the excitation light. Alternatively, the band gap can be measured by depositing the compound to be measured on a substrate such as glass and applying excitation light to the vapor-deposited film. In the measurement, the absorption edge of an absorption spectrum in which the vapor-deposited film absorbs the excitation light is measured, whereby the band gap can be determined.

The LUMO energy level can be calculated using values of the band gap and the ionization potential. By subtracting the value of the ionization potential from the value of the band gap, the LUMO energy level can be estimated.

The LUMO energy level can also be estimated from a reduction potential. For example, a one-electron reduction potential is estimated using CV (cyclic voltammetry) measurement. The CV measurement can be performed, for example, in a 0.1 M solution of tetrabutylammonium perchlorate in DMF using Ag/Ag+ as a reference electrode, Pt as a counter electrode, and glassy carbon as a working electrode. The LUMO energy level can be estimated by adding the difference between the obtained reduction potential of the compound and the reduction potential of ferrocene to - 4.8 eV.

In the organic light-emitting element according to an embodiment of the present invention, when the organic compound according to the present invention is contained in the light-emitting layer, the light-emitting layer may be a layer formed only of the organic compound according to the present invention or a layer formed of the organic compound according to the present invention and other compounds. When the light-emitting layer is a layer formed of the organic compound according to the present invention and other compounds, the organic compound according to the present invention may be used as a host material or a guest material of the light-emitting layer. The organic compound may also be used as an assist material that can be contained in the light-emitting layer. The host material, which is also referred to as a "host" or a "first compound", is a compound having the largest mass ratio among the compounds constituting the light-emitting layer. The guest material, which is also referred to as a "guest", a "dopant material", a "dopant", or a "third compound", is a compound having a mass ratio smaller than that of the host among the compounds constituting the light-emitting layer and is a compound responsible for main light emission. Thus, the guest material may be referred to as a light-emitting material. The assist material, which is also referred to as an "assist" or a "second compound", is a compound that has a mass ratio smaller than that of the host material among the compounds constituting the light-emitting layer and that assists the light emission of the guest material. The assist material is also referred to as a second host.

The lowest excited singlet energy of the host material is defined as S1 (H), the lowest excited singlet energy of the guest material as S1 (D), and the lowest excited singlet energy of the assist material as S1 (A). The guest material may be regarded as the organic compound according to the present invention. At this time, the organic light-emitting element according to the present embodiment preferably satisfies S1 (H) > S1 (D) or S1 (H) > S1 (A) > S1 (D). When the lowest excited singlet energies of the compounds contained in the organic light-emitting element according to the present embodiment satisfy the above relationship, excitons can be efficiently transferred to the guest material, so that the organic light-emitting element has higher luminous efficiency.

When the organic compound according to the present invention is used as the guest material of the light-emitting layer, the concentration of the guest material may be 0.01 mass% or more and less than 50 mass% relative to the whole light-emitting layer, and is preferably 0.01 mass% or more and 10 mass% or less, more preferably 0.01 mass% or more and 5 mass% or less. When the light-emitting layer contains the assist material, the mass of the assist material relative to the whole light-emitting layer may be 1 mass% or more and less than 50 mass%, and is preferably 10 mass% or more and less than 50 mass%.

The present inventors have conducted various studies and found that when the organic compound according to the present invention is used as a host material or a guest material of a light-emitting layer, particularly, as a guest material of a light-emitting layer, an element that outputs light with high efficiency and high luminance and has very high durability can be provided. This light-emitting layer may have a single-layer structure or a multilayer structure. The light-emitting layer may contain a light-emitting material having any other emission color so as to emit light having a color mixed with blue, which is the emission color of the present embodiment. The multilayer structure refers to a state in which the light-emitting layer and another light-emitting layer are stacked on top of each other. In this case, the emission color of the organic light-emitting element is not limited to blue. More specifically, the emission color may be white or an intermediate color. In the case of white, the other light-emitting layer emits light of a color other than blue, that is, red or green. The light-emitting layer is formed by vapor deposition or coating. Details thereof will be described in EXAMPLES given later.

The organic compound according to the present invention can be used as a constituent material of an organic compound layer other than the light-emitting layer constituting the organic light-emitting element according to the present embodiment. Specifically, the organic compound may be used as a constituent material of, for example, the electron transport layer, the electron injection layer, the hole transport layer, the hole injection layer, or the hole blocking layer. In this case, the emission color of the organic light-emitting element is not limited to blue. More specifically, the emission color may be white or an intermediate color.

### (3) Other Compounds

In addition to the organic compound according to the present invention, conventionally known low-molecular-weight and high-molecular-weight hole injection compounds or hole transport compounds, host materials, luminescent compounds, electron injection compounds or electron transport compounds, and the like can optionally be used in combination. Examples of these compounds will be described below.

As hole injection and transport materials, materials that facilitate injection of holes from the anode and that have so high hole mobility that enables injected holes to be transported to the light-emitting layer are preferred. To suppress, for example, crystallization of the organic compound in the organic light-emitting element, materials having high glass-transition temperatures are preferred. Examples of low-molecular-weight and high-molecular-weight materials having hole injection and transport properties include triarylamine derivatives, arylcarbazole derivatives, phenylenediamine derivatives, stilbene derivatives, phthalocyanine derivatives, porphyrin derivatives, poly(vinylcarbazole), poly(thiophene), and other conductive polymers. These hole injection and transport materials are also suitable for use in the electron blocking layer. When the hole injection layer is formed by a coating method, a mixture of polyethylenedioxythiophene and polystyrene sulfonate (PEDOT:PSS), which is commonly used as a hole injection material, may be used. Non-limiting specific examples of compounds usable as hole injection and transport materials are shown below.

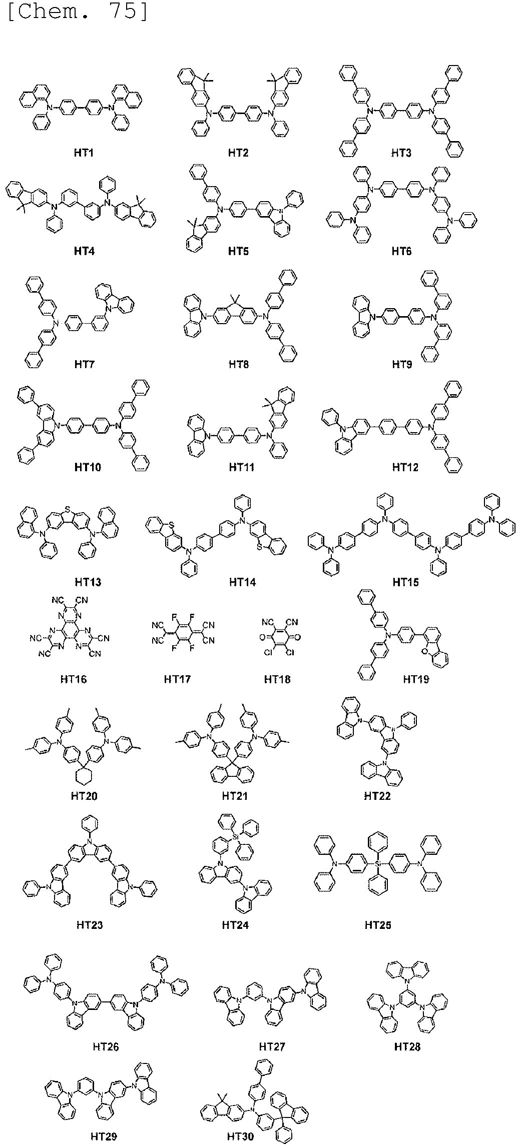

Among the hole injection and transport materials given above, HT16 to HT18 can be used for a layer in contact with the anode to achieve a lower driving voltage. HT16 is widely used in organic light-emitting elements. HT2 to HT7, HT10, HT12, and HT22 to 28 may be used for an organic compound layer adjacent to HT16. Hole transport polymer compounds such as polyphenylene vinylene (PPV), polyfluorene (PF), polyvinyl carpazole (PVK), and derivatives thereof may be used. Alternatively, for example, insulator layers of inorganics such as SiO2 and SiN and organic silicon polymers such as siloxane can also be used. A plurality of materials may be used for one organic compound layer.

Examples of guest materials mainly involved in the light-emitting function include donor-acceptor type organic compounds, boron-containing complexes, indocarbazole fused-ring compounds, fused-ring compounds (e.g., fluorene derivatives, naphthalene derivatives, pyrene derivatives, perylene derivatives, tetracene derivatives, anthracene derivatives, and rubrene), quinacridone derivatives, coumarin derivatives, stilbene derivatives, organic aluminum complexes such as tris(8-quinolinolato)aluminum, iridium complexes, platinum complexes, rhenium complexes, copper complexes, europium complexes, ruthenium complexes, and polymer derivatives such as poly(phenylenevinylene) derivatives, poly(fluorene) derivatives, and poly(phenylene) derivatives. When the light-emitting layer is produced by a coating method, a polymer compound having light-emitting properties is mainly used. This is because polymer compounds tend to exhibit high glass transition temperatures and thus are less likely to undergo crystallization than low-molecular-weight compounds. Examples of materials that are specifically used include polymer compounds such as polyphenylene vinylene (PPV), polyfluorene (PF), polyvinyl carpazole (PVK), and derivatives thereof.

Non-limiting specific examples of compounds usable as light-emitting materials are shown below.

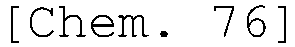

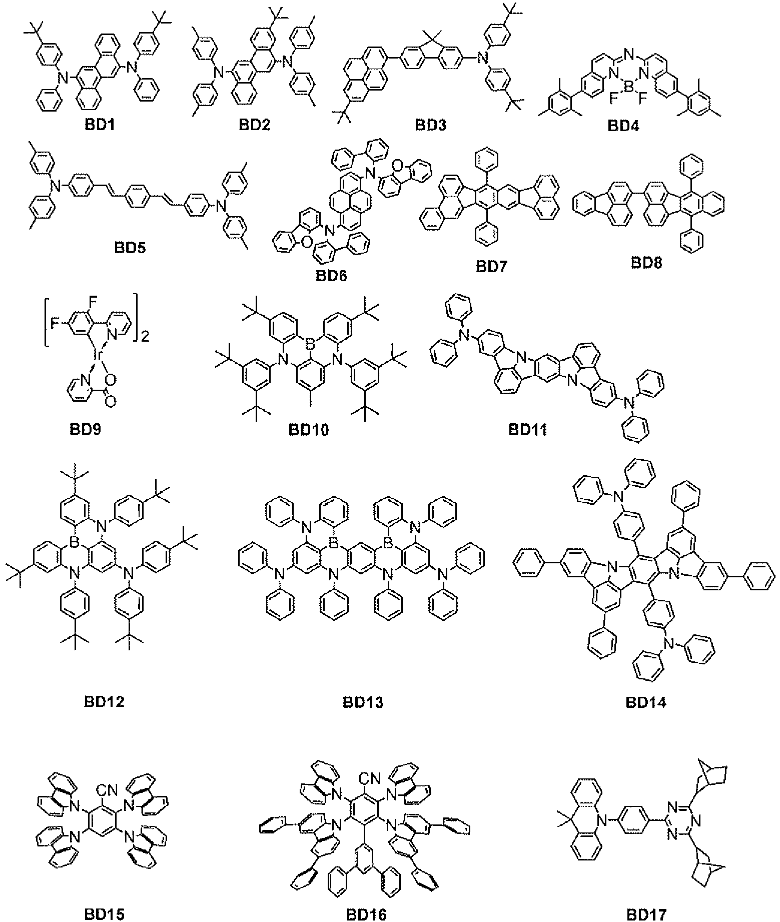

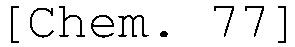

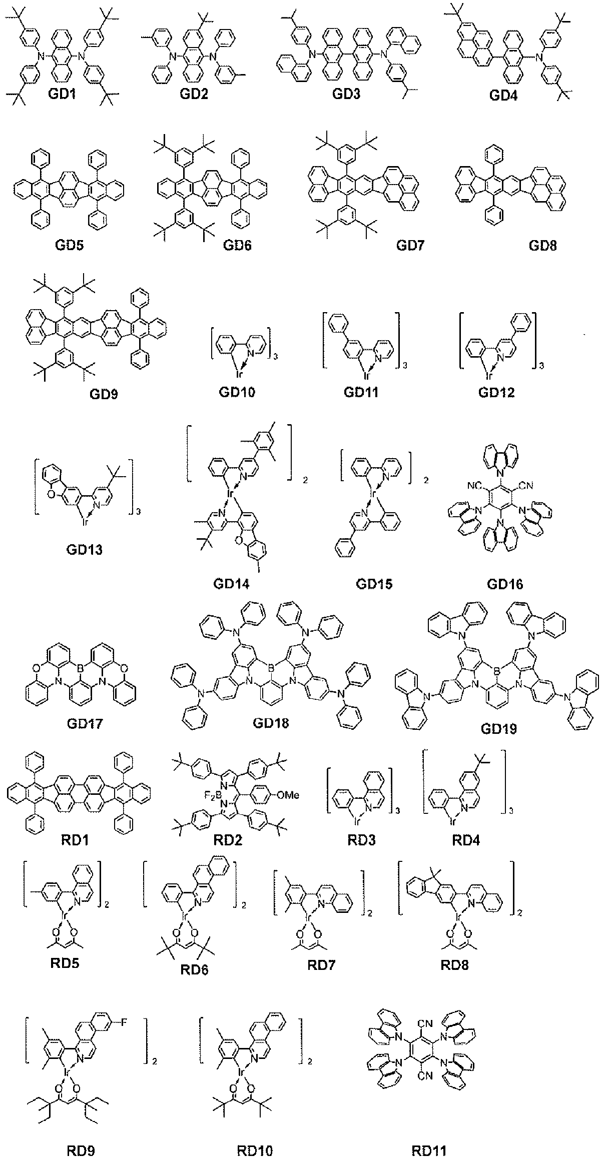

Non-limiting specific examples of compounds usable as host materials or assist materials contained in the light-emitting layer are shown below.

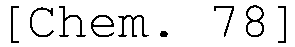

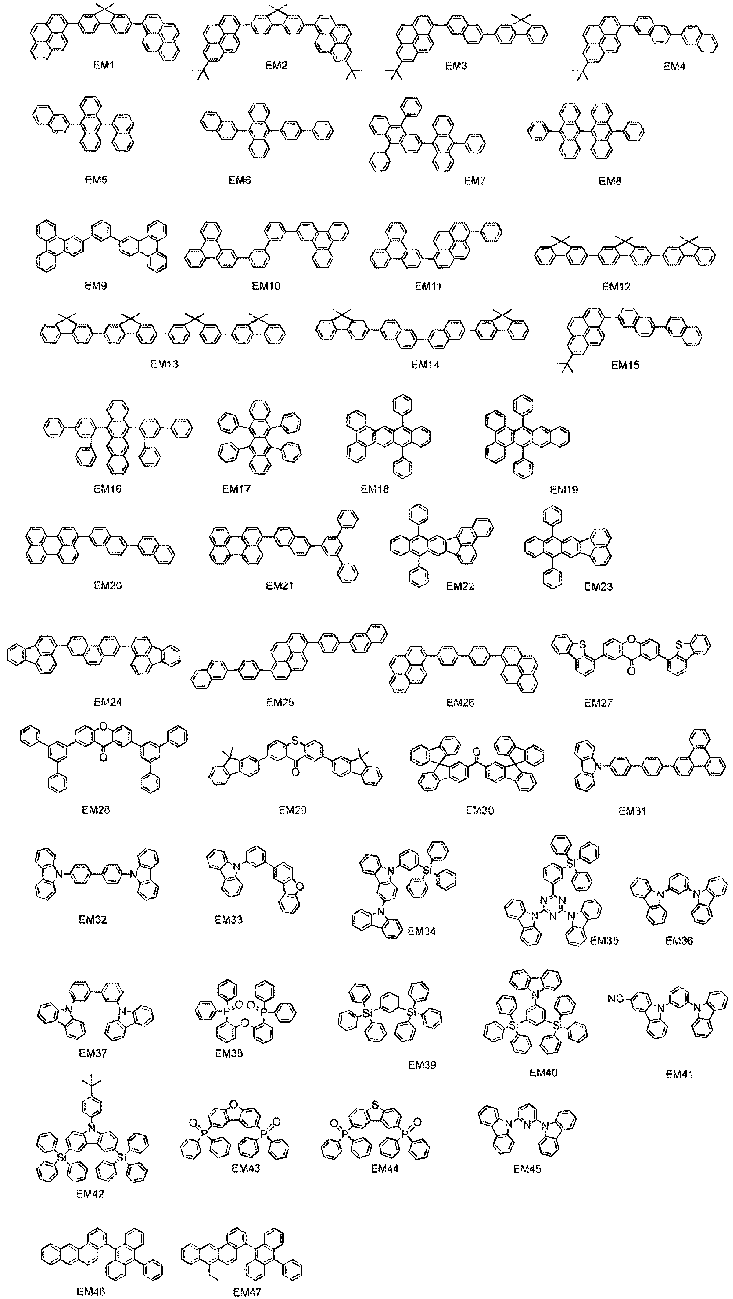

Any electron transport material capable of transporting electrons injected from the cathode to the light-emitting layer can be freely selected in consideration of, for example, the balance with the hole mobility of a hole transport material. Examples of materials capable of transporting electrons include oxadiazole derivatives, oxazole derivatives, pyrazine derivatives, triazole derivatives, triazine derivatives, quinoline derivatives, quinoxaline derivatives, phenanthroline derivatives, organic aluminum complexes, and fused-ring compounds (e.g., fluorene derivatives, naphthalene derivatives, chrysene derivatives, and anthracene derivatives). These electron transport materials are also suitable for use for the hole blocking layer.

Non-limiting specific examples of compounds usable as electron transport materials are shown below.

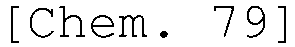

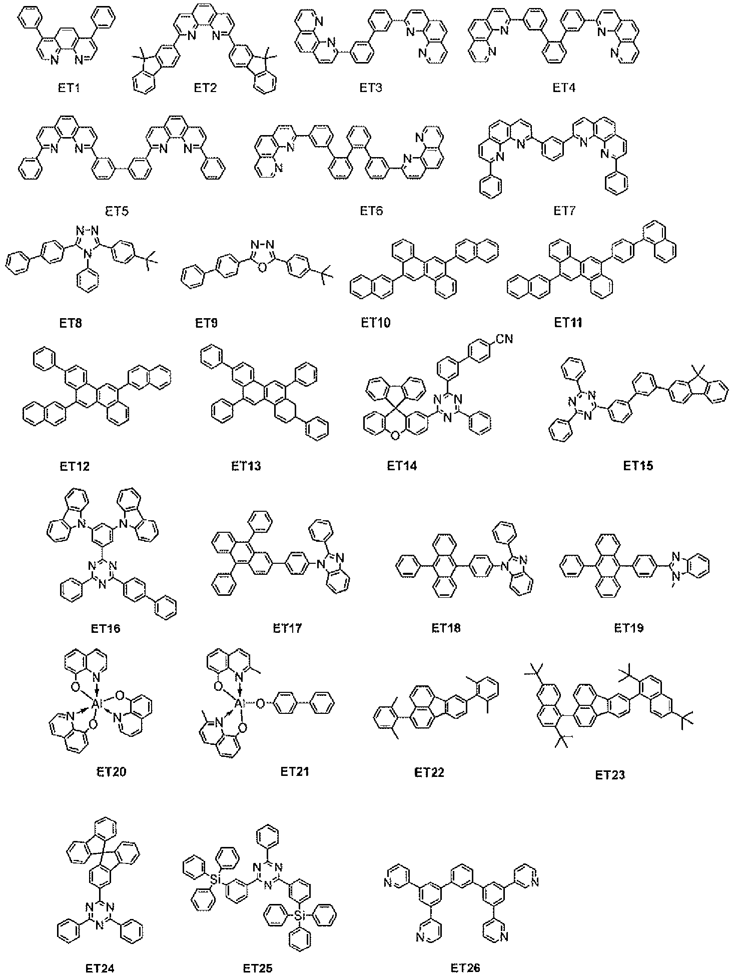

Any electron injection material capable of readily injecting electrons from the cathode can be freely selected in consideration of, for example, the balance with hole injectability. An n-type dopant and a reducing dopant are also contained as organic compounds. Examples include alkali metal-containing compounds such as lithium fluoride, lithium complexes such as lithium quinolinol, benzimidazolidene derivatives, imidazolidene derivatives, fulvalene derivatives, and acridine derivatives.

These can also be used in combination with the electron transport materials above.

The organic compound according to the present invention can also be used in the form of an ink composition.

An ink composition according to the present embodiment contains at least one compound represented by general formula (1-1) or (1-2). Using the ink composition according to the present embodiment enables the layers formed of organic compounds constituting the organic light-emitting element, particularly, the light-emitting layer, to be formed by a coating method, so that a large-area element can be easily produced at a relatively low cost. Examples of solvents that dissolve the compound represented by general formula (1-1) or (1-2) include toluene, xylene, mesitylene, dioxane, methylnaphthalene, tetrahydrofuran, diglyme, 1,2-dichlorobenzene, and 1,2-dichloropropane. These solvents can be used alone or in combination of two or more. Of these, those having an appropriate evaporation rate, specifically, solvents having a boiling point of about 70°C to 200°C are preferably used because a thin film having a uniform thickness is easily provided. The ink composition according to the present embodiment may further contain a compound that serves as an additive. Examples of the compound that serves as an additive include the above-described known light-emitting layer hosts or light-emission assist materials, hole transport materials, light-emitting materials, and electron transport materials.

In the ink composition according to the present embodiment, the concentration of the compound represented by general formula (1-1) or (1-2) is preferably 0.05 wt% or more and 20 wt% or less, more preferably 0.1 wt% or more and 5 wt% or less, relative to the whole composition.

The ink composition according to the present embodiment can be formed into a film by a spin coating method, a bar coating method, a slit coating method, an ink-jet method, a nozzle coating method, a casting method, a gravure printing method, or the like, whereby an organic layer of the organic light-emitting element described below can be formed.

### (4) Configuration of Organic Light-Emitting Element

Hereinafter, constituent members constituting the organic light-emitting element according to the present embodiment will be described.

The organic light-emitting element is provided by forming an insulating layer, a first electrode, an organic compound layer, and a second electrode on a substrate. On the second electrode, a protective layer, a color filter, a microlens, etc. may be disposed. When the color filter is disposed, a planarization layer may be disposed between the color filter and the protective layer. The planarization layer may be formed of, for example, an acrylic resin. This also applies to the case where the planarization layer is disposed between the color filter and the microlens.

### [Substrate]

Examples of the substrate include quartz, glass, silicon wafers, resins, and metals. The substrate may have switching elements, such as transistors, and wiring lines disposed thereon, and may have an insulating layer disposed thereon. The insulating layer may be made of any material as long as a contact hole can be formed therein so as to allow formation of a wiring line connecting to the first electrode and insulation from unconnected wiring lines can be provided. For example, resins such as polyimide, silicon oxide, silicon nitride, and the like can be used.

### [Electrode]

The electrodes may be a pair of electrodes. The pair of electrodes are the first electrode and the second electrode. Specifically, the pair of electrodes may be an anode and a cathode. When an electric field is applied in a direction in which the organic light-emitting element emits light, one of the electrodes at a higher potential is the anode, and the other is the cathode. Stated another way, one of the electrodes that supplies holes to the light-emitting layer is the anode, and the other electrode that supplies electrons to the light-emitting layer is the cathode.

The constituent material of the anode preferably has as high a work function as possible. For example, elemental metals such as gold, platinum, silver, copper, nickel, palladium, cobalt, selenium, vanadium, and tungsten, mixtures containing these metals, alloys obtained by combining these metals, and metal oxides such as tin oxide, zinc oxide, indium oxide, indium tin oxide (ITO), and indium zinc oxide can be used. Conductive polymers such as polyaniline, polypyrrole, and polythiophene can also be used.

These electrode materials may be used alone or in combination of two or more. The anode may be composed of a single layer or a plurality of layers.

When the anode is used as a reflection electrode, for example, chromium, aluminum, silver, titanium, tungsten, molybdenum, an alloy thereof, or a stack thereof can be used. These materials can also be used to function as a reflective film that does not have a role of an electrode. When the anode is used as a transparent electrode, it may be, for example, but not necessarily, a transparent conductive layer made of an oxide such as indium tin oxide (ITO) or indium zinc oxide. Photolithography can be used to form the electrode.

The constituent material of the cathode preferably has a low work function. Examples of such materials include alkali metals such as lithium, alkaline earth metals such as calcium, elemental metals such as aluminum, titanium, manganese, silver, lead, and chromium, and mixtures thereof. Alloys obtained by combining these elemental metals can also be used. For example, magnesium-silver, aluminum-lithium, aluminum-magnesium, silver-copper, and zinc-silver alloys can be used. Metal oxides such as indium tin oxide (ITO) can also be used. These electrode materials may be used alone or in combination of two or more. The cathode may be composed of a single layer or a plurality of layers. In particular, silver is preferably used, and a silver alloy is more preferred to reduce aggregation of silver. As long as aggregation of silver can be reduced, the content ratio in the alloy is not limited. For example, the ratio of silver to other metals may be, for example, 1:1 or 3:1.

The cathode is not particularly limited; a conductive layer formed of an oxide such as ITO may be used to provide a top-emission element, or a reflection electrode formed of aluminum (Al) or the like may be used to provide a bottom-emission element. The method of forming the cathode is not particularly limited, but the use of DC sputtering, AC sputtering, or the like is more preferred because good film coverage can be achieved and the resistance tends to decrease.

### [Organic Compound Layer]

The organic compound layer may be formed of a single layer or a plurality of layers. When the organic compound layer includes a plurality of layers, the layers may be referred to as a hole injection layer, a hole transport layer, an electron blocking layer, a light-emitting layer, a hole blocking layer, an electron transport layer, and an electron injection layer depending on their functions. The organic compound layer is composed mainly of an organic compound and may contain an inorganic atom and an inorganic compound. For example, the organic compound layer may contain copper, lithium, magnesium, aluminum, iridium, platinum, molybdenum, zinc, or the like. The organic compound layer may be disposed between the first electrode and the second electrode and may be disposed in contact with the first electrode and the second electrode.

The organic compound layers (e.g., the hole injection layer, the hole transport layer, the electron blocking layer, the light-emitting layer, the hole blocking layer, the electron transport layer, and the electron injection layer) constituting the organic light-emitting element according to the present embodiment are formed by the following methods.

The organic compound layers constituting the organic light-emitting element according to the present embodiment can be formed using a dry process such as vacuum deposition, ionized deposition, sputtering, or plasma. Instead of the dry process, a wet process in which a solution in an appropriate solvent is used to form a layer by a known coating method (e.g., spin coating, dipping, a casting method, an LB method, or an ink jet method) can also be used.

When the layers are formed by, for example, vacuum deposition or solution coating, the layers are unlikely to undergo crystallization or the like and are highly stable over time. When a coating method is used for film formation, an appropriate binder resin can be used in combination to form a film.

Examples of the binder resin include polyvinylcarbazole resins, polycarbonate resins, polyester resins, ABS resins, acrylic resins, polyimide resins, phenol resins, epoxy resins, silicone resins, and urea resins, but are not limited thereto.

These binder resins may be used alone as a homopolymer or copolymer or may be used as a mixture of two or more. In addition, known additives such as plasticizers, antioxidants, and UV absorbers may be used in combination as required.

### [Protective Layer]

A protective layer may be disposed on the cathode. For example, a glass member provided with a moisture absorbent can be bonded onto the cathode to reduce the entry of, for example, water into the organic compound layer, thereby reducing the occurrence of display failure. In another embodiment, a passivation film made of silicon nitride or the like may be disposed on the cathode to reduce the entry of, for example, water into the organic compound layer. For example, the protective layer may be formed in a manner that after the formation of the cathode, the resultant is conveyed to another chamber without breaking the vacuum, and a silicon nitride film having a thickness of 2 µm is formed by CVD. After the film formation by CVD, atomic layer deposition (ALD) may be performed to form a protective layer. The material of the film formed by ALD is not limited and may be, for example, silicon nitride, silicon oxide, or aluminum oxide. On the film formed by ALD, silicon nitride may further be formed by CVD. The film formed by ALD may have a smaller thickness than the film formed by CVD. Specifically, the thickness of the film formed by ALD may be 50% or less, or even 10% or less of the thickness of the film formed by CVD.

### [Color Filter]

A color filter may be disposed on the protective layer. For example, a color filter may be formed on another substrate so as to correspond to the size of the organic light-emitting element and bonded to the substrate having the organic light-emitting element disposed thereon. Alternatively, a color filter may be patterned on the above-described protective layer by photolithography. The color filter may be formed of a polymer.

### [Planarization Layer]

A planarization layer may be disposed between the color filter and the protective layer. The planarization layer is disposed for the purpose of reducing unevenness of the underlying layer. The planarization layer may be referred to as a material resin layer without limiting the purpose. The planarization layer may be formed of an organic compound. The organic compound may have a low molecular weight or a high molecular weight, and preferably has a high molecular weight.

The planarization layer may be disposed on opposite surfaces of the color filter, and the constituent materials thereof may be the same or different. Specific examples include polyvinylcarbazole resins, polycarbonate resins, polyester resins, ABS resins, acrylic resins, polyimide resins, phenol resins, epoxy resins, silicone resins, and urea resins.

### [Microlens]

The organic light-emitting element according to the present embodiment may include, on its light-emitting side, an optical member such as a microlens. The microlens can be formed of an acrylic resin, an epoxy resin, or the like. The microlens may be used to increase the amount of light extracted from the organic light-emitting element and to control the direction of the extracted light. The microlens may have a hemispherical shape. In the case of a hemispherical shape, among tangents to the hemisphere, there is a tangent parallel to the insulating layer, and the point of contact between this tangent and the hemisphere is the vertex of the microlens. The vertex of the microlens can be determined in the same manner in any sectional view. That is, among tangents to the semicircle of the microlens in a sectional view, there is a tangent parallel to the insulating layer, and the point of contact between this tangent and the semicircle is the vertex of the microlens.

The midpoint of the microlens can also be defined. In a section of the microlens, a line segment from the start point of an arc shape to the start point of another arc shape is imagined, and the midpoint of the line segment can be referred to as the midpoint of the microlens. The section used to determine the vertex and the midpoint may be a section perpendicular to the insulating layer.

### [Opposite Substrate]

An opposite substrate may be disposed on the planarization layer. The opposite substrate is disposed at a position opposite to the above-described substrate and thus is referred to as the opposite substrate. The constituent material of the opposite substrate may be the same as that of the above-described substrate. When the above-described substrate is a first substrate, the opposite substrate may be a second substrate.

### [Pixel Circuit]

A light-emitting apparatus may include a pixel circuit connected to the light-emitting element. The pixel circuit may be an active matrix-type circuit which independently controls the light emission of a first light-emitting element and a second light-emitting element. The active matrix-type circuit may be voltage programmed or current programmed. A drive circuit includes the pixel circuit for each pixel. The pixel circuit may include a light-emitting element, a transistor that controls the emission luminance of the light-emitting element, a transistor that controls the timing of light emission, a capacitor that holds the gate voltage of the transistor that controls the emission luminance, and a transistor for providing connection to GND not through the light-emitting element.

The light-emitting apparatus has a display region and a peripheral region disposed around the display region. The display region includes the pixel circuit, and the peripheral region includes a display control circuit. The mobility of the transistor constituting the pixel circuit may be lower than the mobility of a transistor constituting the display control circuit.

The gradient of the current-voltage characteristics of the transistor constituting the pixel circuit may be smaller than the gradient of the current-voltage characteristics of the transistor constituting the display control circuit. The gradient of the current-voltage characteristics can be determined on the basis of what is called Vg-Ig characteristics.

The transistor constituting the pixel circuit is a transistor connected to a light-emitting element such as the first light-emitting element.

### [Pixel]

The organic light-emitting apparatus includes a plurality of pixels. Each pixel includes subpixels that emit light of colors different from each other. The subpixels may respectively have, for example, R, G, and B emission colors.

In each pixel, a region also referred to as a pixel aperture emits light. This region is the same as the first region. The size of the pixel aperture may be 15 µm or less and 5 µm or more. More specifically, the size may be, for example, 11 µm, 9.5 µm, 7.4 µm, or 6.4 µm.

The distance between the subpixels may be 10 µm or less, specifically 8 µm, 7.4 µm, or 6.4 µm.

The pixels may be in a known arrangement when viewed in plan. For example, the arrangement may be the stripe arrangement, the delta arrangement, the PenTile arrangement, or the Bayer arrangement. The shape of the subpixels in plan view may be any known shape. Examples include quadrangles, such as rectangles and rhombuses, and hexagons. It is appreciated that shapes that are not exactly rectangles but are similar to rectangles are also regarded as rectangles. The shape of the subpixels and the pixel arrangement can be used in combination.

### (5) Applications of Organic Light-Emitting Element According to Present Embodiment

The organic light-emitting element according to the present embodiment can be used as a constituent member of a display apparatus or a lighting apparatus. Other applications include an exposure light source in an electrophotographic image-forming apparatus, a backlight in a liquid crystal display, and a light-emitting apparatus including a white light source with a color filter.

The display apparatus may be an image information processor that includes an image input unit to which image information from an area CCD, a linear CCD, a memory card, or the like is input, includes an information-processing unit configured to process the input information, and displays the input image on a display unit.

The display unit of an image pickup apparatus or an ink-jet printer may have a touch panel function. The touch panel function may be activated by any system, such as an infrared system, an electrostatic capacitive system, a resistive film system, or an electromagnetic induction system. The display apparatus may also be used as a display unit of a multifunctional printer.

Next, a display apparatus according to the present embodiment will be described with reference to the drawings.

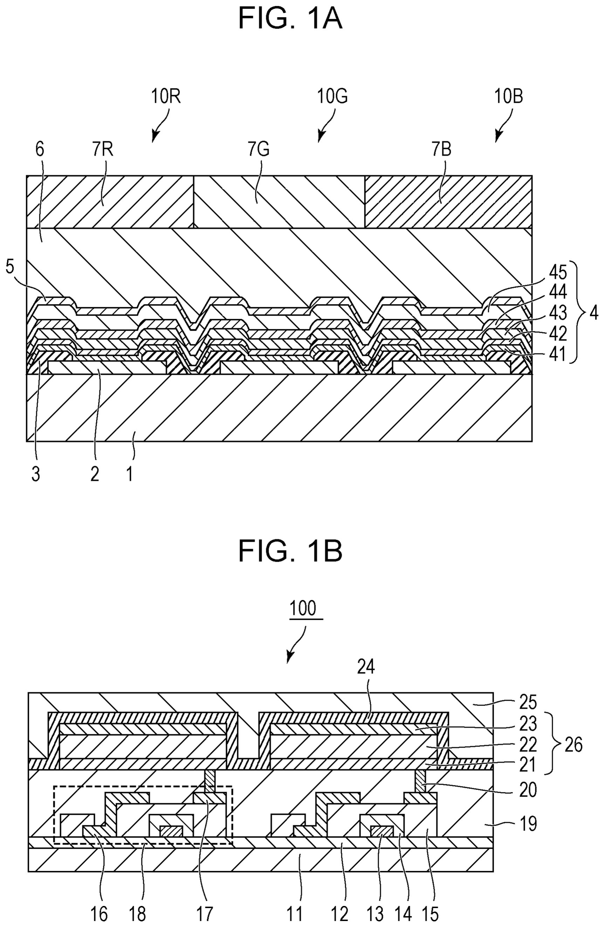

Fig. 1A and Fig. 1B are schematic sectional views each showing an example of a display apparatus including an organic light-emitting element and a transistor connected to the organic light-emitting element. The transistor is an example of an active element. The transistor may be a thin film transistor (TFT).

Fig. 1A is an example of a pixel that is a component of the display apparatus according to the present embodiment. The pixel includes subpixels 10. The subpixels are divided into 10R, 10G, and 10B according to their light emission. The emission color may be distinguished on the basis of the wavelength of light emitted from a light-emitting layer, or light emitted from the subpixels may undergo selective transmission or color conversion through a color filter or the like. Each of the subpixels includes, on an interlayer insulating layer 1, a reflective electrode 2 that is a first electrode, an insulating layer 3 that covers the edge of the reflective electrode 2, an organic compound layer 4 that covers the first electrode and the insulating layer, a transparent electrode 5, a protective layer 6, and a color filter 7.

The interlayer insulating layer 1 may include a transistor and a capacitor element below or inside the interlayer insulating layer 1. The transistor and the first electrode may be electrically connected to each other through a contact hole (not illustrated) or the like.

The insulating layer 3 is also referred to as a bank or a pixel-separating film. The insulating layer 3 is disposed so as to cover the edge of the first electrode and surround the first electrode. A portion in which the insulating layer is not disposed is in contact with the organic compound layer 4 and serves as a light-emitting region.

The organic compound layer 4 includes a hole injection layer 41, a hole transport layer 42, a first light-emitting layer 43, a second light-emitting layer 44, and an electron transport layer 45.

The second electrode 5 may be a transparent electrode, a reflective electrode, or a semitransparent electrode.

The protective layer 6 reduces permeation of water into the organic compound layer. Although the protective layer is illustrated as a single layer, it may be constituted by a plurality of layers. The layers may be constituted by an inorganic compound layer and an organic compound layer.

The color filter 7 is divided into 7R, 7G, and 7B according to their color. The color filter may be formed on a planarization film (not illustrated). A resin protective layer (not illustrated) may be disposed on the color filter. The color filter may be formed on the protective layer 6. The color filter may be bonded after being formed on an opposite substrate such as a glass substrate.

In a display apparatus 100 in Fig. 1B, an organic light-emitting element 26 and a TFT 18 as an example of a transistor are illustrated. The display apparatus 100 includes a substrate 11 made of glass, silicon, or the like and an insulating layer 12 disposed thereon. An active element 18 such as a TFT is disposed on the insulating layer, and a gate electrode 13, a gate insulating film 14, and a semiconductor layer 15 of the active element are disposed. The active element 18 also includes the semiconductor layer 15, a drain electrode 16, and a source electrode 17. An insulating film 19 is disposed over the active element 18. An anode 21 constituting the organic light-emitting element 26 and the source electrode 17 are connected to each other through a contact hole 20 extending through the insulating film.

The electrodes (the anode and the cathode) included in the organic light-emitting element 26 and the electrodes (the source electrode and the drain electrode) included in the TFT need not necessarily be electrically connected to each other in the manner illustrated in Fig. 1B. It is only required that either the anode or the cathode be electrically connected to either the source electrode or the drain electrode of the TFT. TFT stands for a thin film transistor.

Although the organic compound layer is illustrated as a single layer in the display apparatus 100 in Fig. 1B, the organic compound layer 22 may be constituted by a plurality of layers. A first protective layer 24 and a second protective layer 25 for reducing deterioration of the organic light-emitting element are disposed over the cathode 23.

Although a transistor is used as a switching element in the display apparatus 100 in Fig. 1B, another switching element may be used instead.

The transistor used in the display apparatus 100 in Fig. 1B may be not only a transistor obtained using a single-crystal silicon wafer but also a thin film transistor including a substrate and an active layer on an insulating surface of the substrate. The active layer may be made of, for example, single-crystal silicon, non-single-crystal silicon such as amorphous silicon or microcrystalline silicon, or a non-single-crystal oxide semiconductor such as indium zinc oxide or indium gallium zinc oxide. The thin film transistor is also referred to as a TFT element.

The transistor included in the display apparatus 100 in Fig. 1B may be formed in a substrate such as a Si substrate. The phrase "formed in a substrate" means producing a transistor by processing a substrate itself, such as a Si substrate. That is, having a transistor in a substrate can also mean that the substrate and the transistor are integrally formed.

The emission luminance of the organic light-emitting element according to the present embodiment is controlled by a TFT, which is an example of a switching element, and disposing a plurality of the organic light-emitting elements in a screen enables a display of an image with different emission luminances. The switching element according to the present embodiment is not limited to a TFT, and may be a transistor formed of low-temperature polysilicon or an active-matrix driver formed on a substrate such as a Si substrate. The phrase "on a substrate" may also be referred to as "in a substrate". Whether a transistor is provided in the substrate or a TFT is used is chosen depending on the size of the display unit. For example, when the display unit has a size of about 0.5 inches, the organic light-emitting element is preferably disposed on a Si substrate.

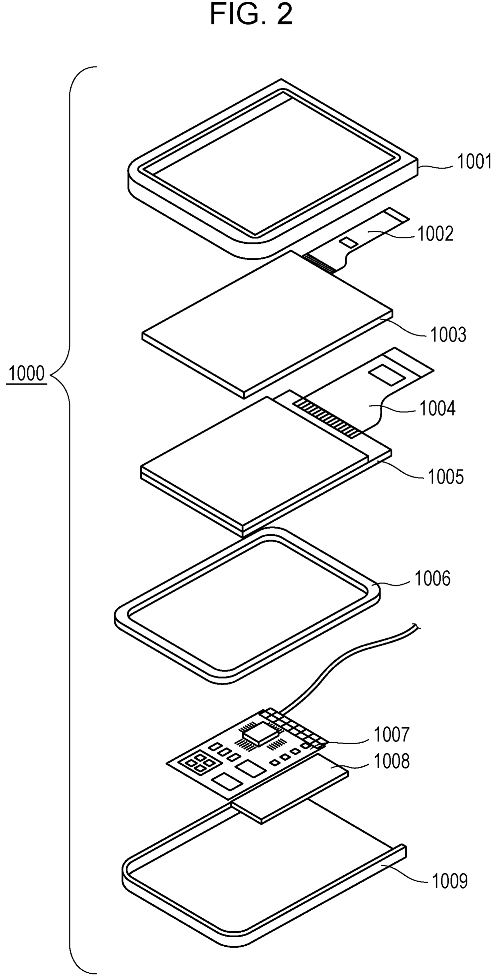

Fig. 2 is a schematic view showing an example of the display apparatus according to the present embodiment. A display apparatus 1000 may include an upper cover 1001, a lower cover 1009, and a touch panel 1003, a display panel 1005, a frame 1006, a circuit board 1007, and a battery 1008 disposed between the covers. The display panel 1005 may include the organic light-emitting element according to the present embodiment. Flexible print circuits (FPCs) 1002 and 1004 are connected to the touch panel 1003 and the display panel 1005, respectively. A transistor is printed on the circuit board 1007. The battery 1008 may be omitted if the display apparatus is not a mobile device. If the display apparatus is a mobile device, the battery 1008 may be disposed in another position.

The display apparatus according to the present embodiment may include red, green, and blue color filters. The red, green, and blue color filters may be disposed in the delta arrangement.

The display apparatus according to the present embodiment may be used as a display unit of a mobile terminal. In this case, the display apparatus may have both a display function and an operating function. Examples of the mobile terminal include cellular phones such as smart phones, tablets, and head-mounted displays.

The display apparatus according to the present embodiment may be used as a display unit of an image pickup apparatus that includes an image pickup element configured to receive light. The image pickup apparatus may include a display unit configured to display information acquired by the image pickup element. The display unit may be exposed to the outside of the image pickup apparatus or disposed in a viewfinder. The image pickup apparatus may be a digital camera or a digital camcorder.

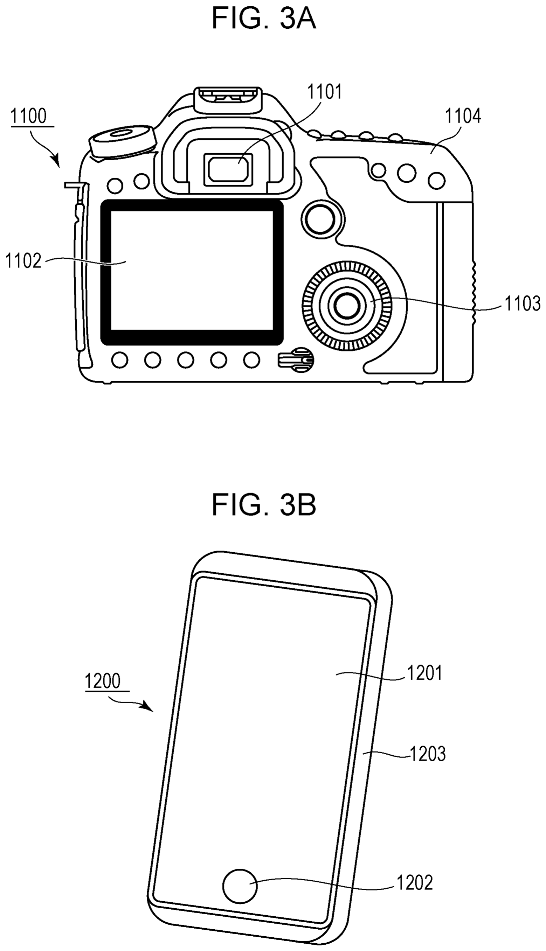

Fig. 3A is a schematic view showing an example of an image pickup apparatus according to the present embodiment. An image pickup apparatus 1100 may include a viewfinder 1101, a rear display 1102, an operation unit 1103, and a housing 1104. The viewfinder 1101 and the rear display 1102 may include the organic light-emitting element according to the present embodiment. In this case, the viewfinder 1101 and the rear display 1102 may display not only an image to be captured but also environmental information, image capture instructions, etc. The environmental information may be, for example, the intensity of external light, the direction of external light, the moving speed of a subject, or the possibility that the subject is hidden by an object.

Since the timing appropriate for capturing an image is only a moment, the information is desirably displayed as quickly as possible. Thus, the display apparatus including the organic light-emitting element according to the present embodiment is preferably used. This is because the organic light-emitting element has a high response speed.

The image pickup apparatus 1100 may further include an optical unit (not illustrated). The optical unit may have a single lens or a plurality of lenses and focuses an image on the image pickup element accommodated in the housing 1104. By adjusting the relative positions of the plurality of lenses, the focal point can be adjusted. This operation can also be performed automatically. The image pickup apparatus may be referred to as a photoelectric conversion apparatus. Instead of sequential imaging, the photoelectric conversion apparatus may involve, as an imaging method, detection of a difference from the previous image, extraction from continuously recorded images, or the like.

Fig. 3B is a schematic view showing an example of an electronic apparatus according to the present embodiment. An electronic apparatus 1200 includes a display unit 1201, an operation unit 1202, and a housing 1203. The housing 1203 may include a circuit, a printed board including the circuit, a battery, and a communication unit. The operation unit 1202 may be a button or a touch-sensitive response unit. The operation unit may be a biometric recognition unit that, for example, releases a lock upon recognition of fingerprints. An electronic apparatus including a communication unit can also be referred to as a communication apparatus. The electronic apparatus may further have a camera function by including lenses and an image pickup element. An image captured by the camera function is displayed on the display unit. Examples of the electronic apparatus include smartphones and notebook computers.

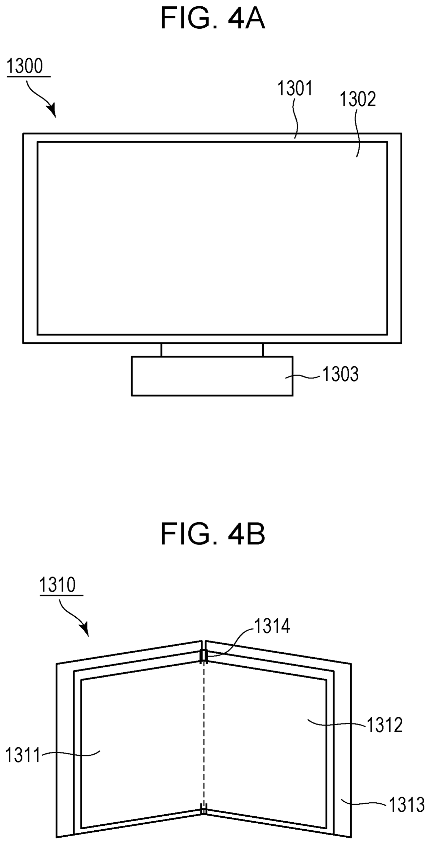

Fig. 4A and Fig. 4B show schematic views showing examples of the display apparatus according to the present embodiment. Fig. 4A is a display apparatus such as a television monitor or a PC monitor. A display apparatus 1300 includes a housing 1301 and a display unit 1302. The organic light-emitting element according to the present embodiment may be used in the display unit 1302.

The display apparatus 1300 may include a base 1303 that supports the housing 1301 and the display unit 1302. The base 1303 need not necessarily be in the form illustrated in Fig. 4A. The lower side of the housing 1301 may serve as a base.

The housing 1301 and the display unit 1302 may be curved. The radius of curvature may be 5000 mm or more and 6000 mm or less.

Fig. 4B is a schematic view showing another example of the display apparatus according to the present embodiment. A display apparatus 1310 in Fig. 4B is configured to be folded and is what is called a foldable display apparatus. The display apparatus 1310 includes a first display unit 1311, a second display unit 1312, a housing 1313, and a bending point 1314. The first display unit 1311 and the second display unit 1312 may include the organic light-emitting element according to the present embodiment. The first display unit 1311 and the second display unit 1312 may be a single seamless display apparatus. The first display unit 1311 and the second display unit 1312 can be divided by the bending point. The first display unit 1311 and the second display unit 1312 may display different images, or the first and second display units may together display a single image.

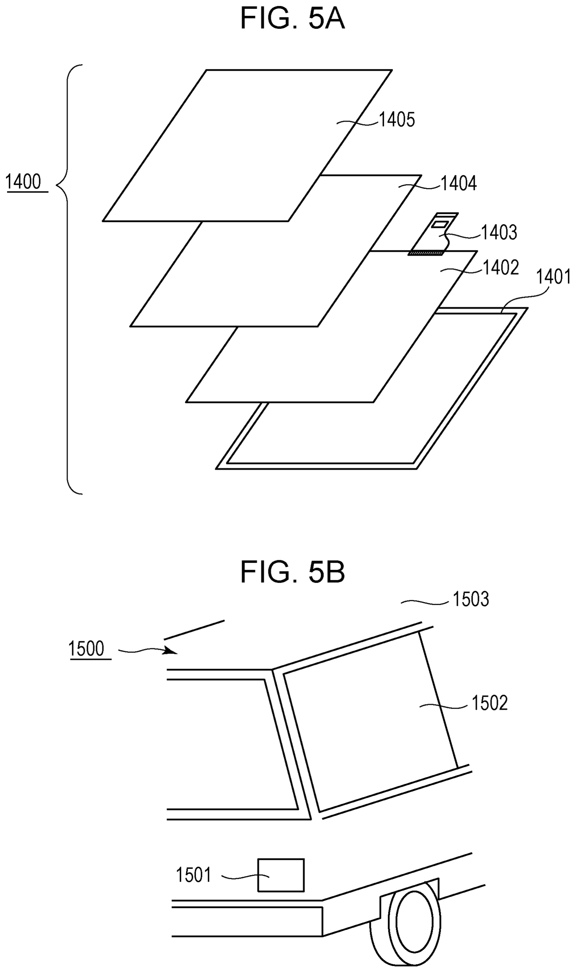

Fig. 5A is a schematic view showing an example of a lighting apparatus according to the present embodiment. A lighting apparatus 1400 may include a housing 1401, a light source 1402, and a circuit board 1403. The light source 1402 may include the organic light-emitting element according to the present embodiment. To improve the color rendering properties of the light source, the lighting apparatus 1400 may include an optical film 1404. To effectively diffuse light from the light source, the lighting apparatus 1400 may include a light diffusion unit 1405. The lighting apparatus 1400 including the light diffusion unit 1405 enables the light to reach a wide region. The optical film 1404 and the light diffusion unit 1405 may be disposed on the light-emitting side of the lighting apparatus. If necessary, a cover may be disposed at an outermost portion.

The lighting apparatus is, for example, an indoor lighting apparatus. The lighting apparatus may emit light of white, daylight white, or any other color from blue to red. The lighting apparatus according to the present embodiment may include a modulation circuit configured to modulate the light. The lighting apparatus according to the present embodiment may include a power supply circuit connected to the organic light-emitting element according to the present embodiment. The power supply circuit may be a circuit configured to convert AC voltage to DC voltage. White is a color with a color temperature of 4200 K, and daylight white is a color with a color temperature of 5000 K. The lighting apparatus according to the present embodiment may further include a color filter.

The lighting apparatus according to the present embodiment may also include a heat dissipation unit. The heat dissipation unit is configured to dissipate heat in the apparatus to the outside and may be, for example, a metal with high thermal conductivity or a ceramic.

Fig. 5B is a schematic view of an automobile that is an example of a moving object according to the present embodiment. The automobile includes a tail lamp that is an example of a lighting fixture. An automobile 1500 includes a tail lamp 1501 and a car body 1503, and the tail lamp may be configured to be turned on in response to, for example, brake operation. The car body 1503 can also be referred to as the body. The automobile 1500 may include a window 1502 attached to the car body 1503.

The tail lamp 1501 may include the organic light-emitting element according to the present embodiment. The tail lamp may include a protective member that protects the light source. The protective member may be formed of any material that has a certain degree of high strength and is transparent, but is preferably formed of a polycarbonate or the like. The polycarbonate may be mixed with a furandicarboxylic acid derivative, an acrylonitrile derivative, or the like.

The window 1502 may be a transparent display unless it is a window for checking the front and rear of the automobile. The transparent display may include the organic light-emitting element according to the present embodiment. In this case, components of the organic light-emitting element according to the present invention, such as electrodes, are constituted by transparent members.

The moving object according to the present embodiment includes one or both of a driving force generation unit configured to generate a driving force mainly used for movement of the moving object and a rotary body mainly used for movement of the moving object. The driving force generation unit may be an engine, a motor, or the like. The rotary body may be a tire, a wheel, a screw for a ship, a propeller for an air vehicle, or the like. Specifically, the moving object may be a bicycle, an automobile, a train, a ship, an aircraft, a drone, or the like. The moving object may include a body and a lighting fixture disposed on the body. The lighting fixture may emit light for allowing the position of the body to be recognized.

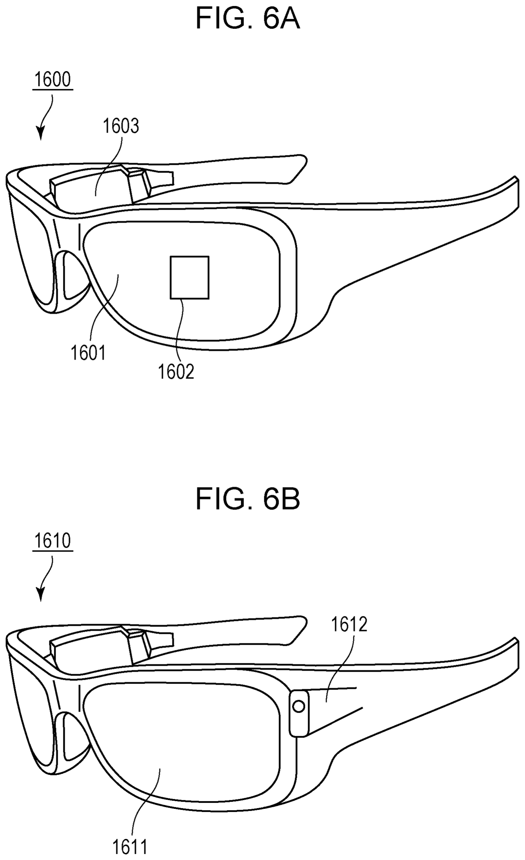

Application examples of the display apparatuses according to the above-described embodiments will be described with reference to Fig. 6A and Fig. 6B. The display apparatuses can be applied to systems that can be worn as wearable devices such as smart glasses, head-mounted displays, and smart contact lenses. A display apparatus usable for a wearable device may include an image pickup apparatus that can photoelectrically convert visible light and a display apparatus that can emit visible light.

Fig. 6A and Fig. 6B are schematic views illustrating examples of eyeglasses (smart glasses) according to the present embodiment. Eyeglasses 1600 (smart glasses) will be described with reference to Fig. 6A. The eyeglasses 1600 include a display unit on the rear side of a lens 1601. The display unit may include the organic light-emitting element according to the present invention. Furthermore, an image pickup apparatus 1602, such as a CMOS sensor or a SPAD, may be disposed on the front side of the lens 1601.

The eyeglasses 1600 further include a controller 1603. The controller 1603 functions as a power source for supplying electricity to the image pickup apparatus 1602 and the display unit. The controller 1603 controls the operation of the image pickup apparatus 1602 and the display unit. The lens 1601 is provided with an optical system for collecting light of the image pickup apparatus 1602 and the display unit.

Eyeglasses 1610 (smart glasses) will be described with reference to Fig. 6B. The eyeglasses 1610 include a controller 1612, and the controller 1612 is provided with a display apparatus including the organic light-emitting element according to the present invention. The controller 1612 may further include an image pickup apparatus corresponding to the image pickup apparatus 1602. A lens 1611 is provided with an optical system for projecting light emitted from the controller 1612, and an image is projected onto the lens 1611. The controller 1612 functions as a power source for supplying electricity to the image pickup apparatus and the display apparatus and also controls the operation of the image pickup apparatus and the display apparatus. The controller may include a gaze detection unit configured to detect the gaze of a wearer. The gaze may be detected using infrared radiation. An infrared light emission unit emits infrared light to an eyeball of a user gazing at a displayed image. Among emitted infrared light, reflected light from the eyeball is detected by an image pickup unit including a light-receiving element, whereby a captured image of the eyeball is obtained. Due to the presence of a reduction unit configured to reduce light from the infrared light emission unit to the display unit in plan view, degradation of image quality is reduced.

From the captured image of the eyeball obtained by infrared imaging, the controller 1612 detects the gaze of the user toward the displayed image. Any known method can be used for the gaze detection using the captured image of the eyeball. For example, a gaze detection method based on a Purkinje image formed by the reflection of irradiation light on a cornea can be used.

More specifically, a gaze detection process based on a pupil-corneal reflection method is performed. Using the pupil-corneal reflection method, a gaze vector representing the direction (rotation angle) of the eyeball is produced on the basis of a pupil image and a Purkinje image included in the captured image of the eyeball, whereby the gaze of the user is detected.

The display apparatus according to the present embodiment may include an image pickup apparatus including a light-receiving element and may control a displayed image on the display apparatus on the basis of the gaze information of the user from the image pickup apparatus.

Specifically, the display apparatus determines, on the basis of the gaze information, a first viewing region at which the user gazes and a second viewing region other than the first viewing region. The first viewing region and the second viewing region may be determined by the controller of the display apparatus, or may be determined by an external controller and sent therefrom. In a display region of the display apparatus, the display resolution of the first viewing region may be controlled to be higher than the display resolution in the second viewing region. That is, the resolution in the second viewing region may be set to be lower than that in the first viewing region.

The display region includes a first viewing region and a second viewing region different from the first viewing region, and a region of high priority is determined from the first viewing region and the second viewing region on the basis of the gaze information. The first viewing region and the second viewing region may be determined by the controller of the display apparatus, or may be determined by an external controller and sent therefrom. The resolution in the region of high priority may be controlled to be higher than the resolution in the region other than the region of high priority. That is, the resolution in a region of relatively low priority may be set to be lower.

AI may be used to determine the first viewing region or the viewing region of high priority. AI may be a model configured to use, as teaching data, an image of an eyeball and the actual gaze direction of the eyeball in the image and estimate, from the image of the eyeball, the angle of gaze and the distance to an object gazed. AI may be included in the display apparatus, the image pickup apparatus, or an external apparatus. When the external apparatus includes AI, it can be suitably used in smart glasses further including an image pickup apparatus configured to capture an external image. Smart glasses can display captured external information in real time.

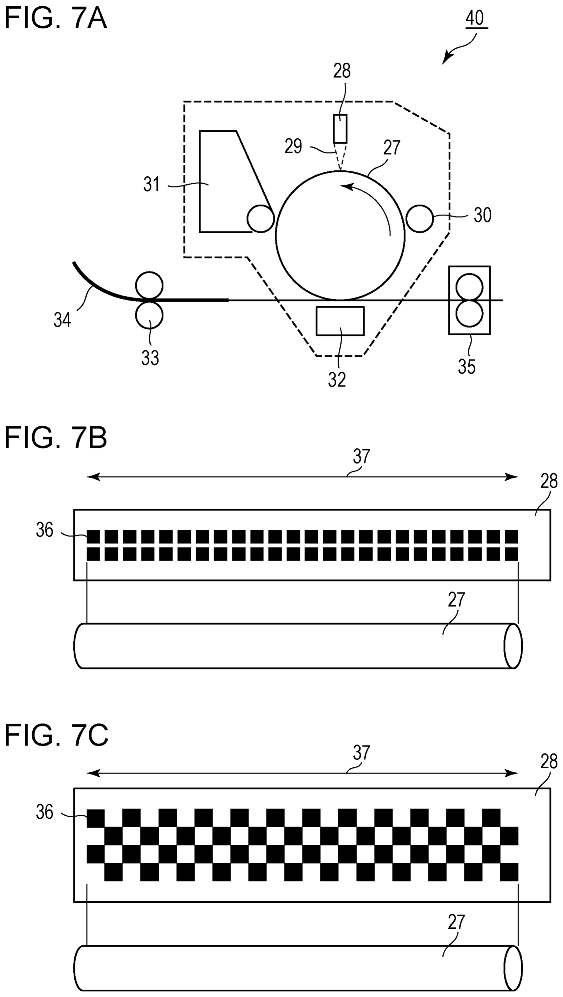

Fig. 7A is a schematic view showing an example of an image-forming apparatus according to the present embodiment. An image-forming apparatus 40 is an electrophotographic image-forming apparatus and includes a photoreceptor 27, an exposure light source 28, a charging unit 30, a developing unit 31, a transfer unit 32, a conveyer roller 33, and a fixing unit 35. The exposure light source 28 emits light 29, and an electrostatic latent image is formed on the surface of the photoreceptor 27. The exposure light source 28 may include the organic light-emitting element according to the present embodiment. The developing unit 31 contains a toner and the like. The charging unit 30 charges the photoreceptor 27. The transfer unit 32 transfers a developed image onto a recording medium 34. The conveyer roller 33 conveys the recording medium 34. The recording medium 34 is, for example, paper. The fixing unit 35 fixes an image formed on the recording medium 34.

Fig. 7B and Fig. 7C each illustrate the exposure light source 28 and each schematically illustrate how a plurality of light-emitting portions 36 are arranged on a long substrate. An arrow 37 indicates a row direction in which organic light-emitting elements are aligned. The row direction is the same as the direction of the rotation axis of the photoreceptor 27. This direction can also be referred to as the major-axis direction of the photoreceptor 27. In Fig. 7B, the light-emitting portions 36 are arranged along the major-axis direction of the photoreceptor 27. In Fig. 7C, unlike Fig. 7B, the light-emitting portions 36 are alternately arranged in the row direction in a first row and in a second row. The first row and the second row are located at different positions in the column direction. In the first row, the plurality of light-emitting portions 36 are arranged at intervals. In the second row, the light-emitting portions 36 are arranged at positions corresponding to the spaces between the light-emitting portions 36 in the first row. That is, the plurality of light-emitting portions 36 are arranged at intervals also in the column direction. The arrangement in Fig. 7C can be referred to as, for example, a lattice arrangement, a staggered arrangement, or a checkered pattern.

As described above, the use of an apparatus including the organic light-emitting element according to the present embodiment enables a stable display with good image quality over a long period of time.

### EXAMPLES

The present invention will now be described with reference to Examples. It should be noted that the present invention is not limited thereto.

### [Example 1]

### (1) Synthesis of Compound A-6

Compound A-6 was synthesized according to the following synthetic route.

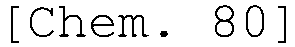

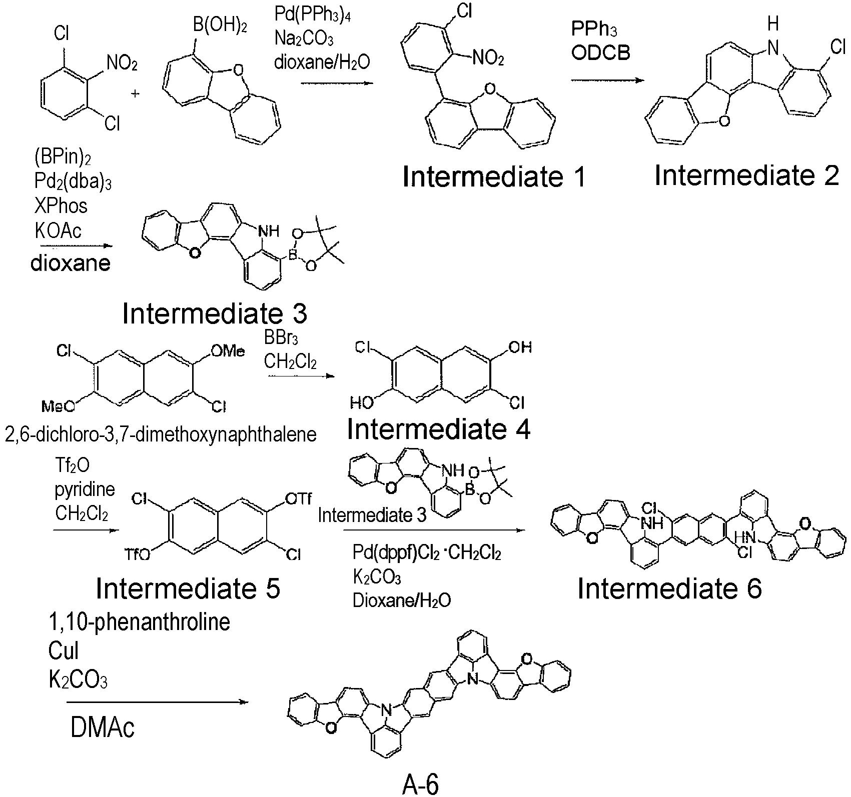

### (1-1) Synthesis of Intermediate 1

1,3-Dichloro-2-nitrobenzene (5 g), dibenzo[b,d]furan-4-ylboronic acid (5.6 g), tetrakis triphenylphosphine palladium (0.61 g), sodium carbonate (8.5 g), dioxane (40 mL), and water (10 mL) were added, and stirring was performed at 100°C for 10 hours. After extraction with dichloromethane, the resultant was concentrated. The concentrate was washed with methanol and filtered to obtain 6 g of intermediate 1 as a white solid.

### (1-2) Synthesis of Intermediate 2

Intermediate 1 (7.8 g), triphenylphosphine (14.15 g), and ortho-dichlorobenzene (180 mL) were added, and stirring was performed at 220°C for 120 hours. After the ortho-dichlorobenzene was distilled off, silica gel chromatography was performed at ethyl acetate:heptane = 1:4 to obtain 5.41 g of intermediate 2 as a white powder.

### (1-3) Synthesis of Intermediate 3

Intermediate 2 (3.4 g), bis(pinacolato)diboron (8.8 g), tris(dibenzylideneacetone)dipalladium(0) (0.33 g), XPhos (0.33 g), potassium acetate (3.41 g), and dioxane (100 mL) were added, and after Ar bubbling was performed, stirring was performed under nitrogen at 100°C for 8 hours. Water was added, and the mixture was extracted with toluene. After the toluene was distilled off, silica gel chromatography was performed at ethyl acetate:heptane = 1:10 to obtain 3.84 g of intermediate 3.

### (1-4) Synthesis of Intermediate 4

2,6-Dichloro-3,7-dimethoxynaphthalene(5 g) and dichloromethane (200 mL) were added, and after the mixture was cooled in an ice bath, boron tribromide (17% in Dichloromethane, ca. 1 mol/L) (97 ml) was added dropwise. The mixture was returned to room temperature and further stirred for 16 hours. Water was added, the mixture was extracted with ethyl acetate, and the ethyl acetate was distilled off to obtain 3.9 g of intermediate 4.

### (1-5) Synthesis of Intermediate 5

Intermediate 4 (1.83 g), dichloromethane (60 mL), and pyridine (2.58 mL) were added, and after the mixture was cooled in an ice bath, trifluoromethanesulfonic anhydride (3.37 ml) was added dropwise. The mixture was returned to room temperature and further stirred for 16 hours. Water was added, the mixture was extracted with dichloromethane, and the dichloromethane was distilled off. Silica gel chromatography was performed with dichloromethane to obtain 3.23 g of intermediate 5 as a white powder.

### (1-6) Synthesis of Intermediate 6

Intermediate 3 (0.766 g), intermediate 5 (2 g), tetrakis triphenylphosphine palladium (0.115 g), potassium carbonate (0.829 g), toluene (50 mL), ethanol (20 mL), and water (20 mL) were added, and stirring was performed at 100°C for 12 hours. The mixture was washed with methanol and filtered to obtain intermediate 6 in crude form. Intermediate 6 was directly used in the subsequent reaction without further purification.

### (1-7) Synthesis of Compound A-6

Intermediate 6, 1,10-phenanthroline (0.238 g), copper iodide (0.228 g), potassium carbonate (1.106 g), and dimethylacetamide (40 mL) were added, and stirring was performed at 160°C for 16 hours. After filtration with methanol/water, the filter cake was repeatedly dispersed and washed with toluene three times, and vacuum drying was performed at 140°C to obtain a green powder. The green powder was purified by sublimation to obtain 90 mg of compound A-6 as a yellow powder. THF was added to the yellow powder to form a slurry, and in this state, mass spectrometry was performed by APSAP (Atmospheric Pressure Solid Sample Probe)-MS for identification (m/z = 634.168, C₄₆H₃₂N₂O₂⁺).

### [Example 2]

### (2) Synthesis of Compound A-7

Compound A-7 was synthesized according to the following synthetic route. Intermediate 5 was synthesized by the method described in Example 1.

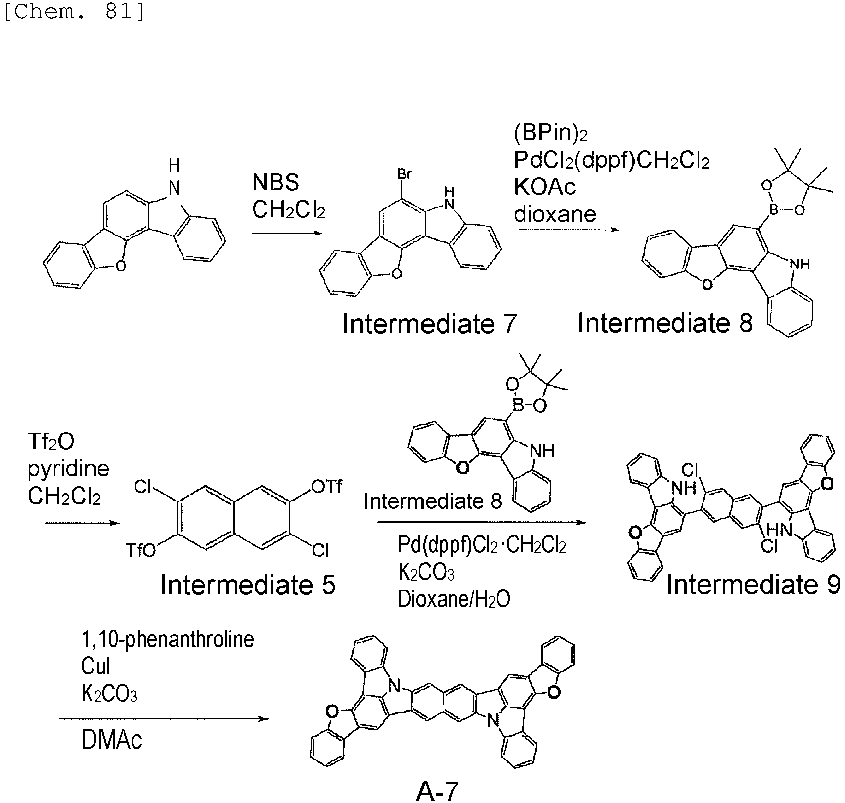

### (2-1) Synthesis of Intermediate 7

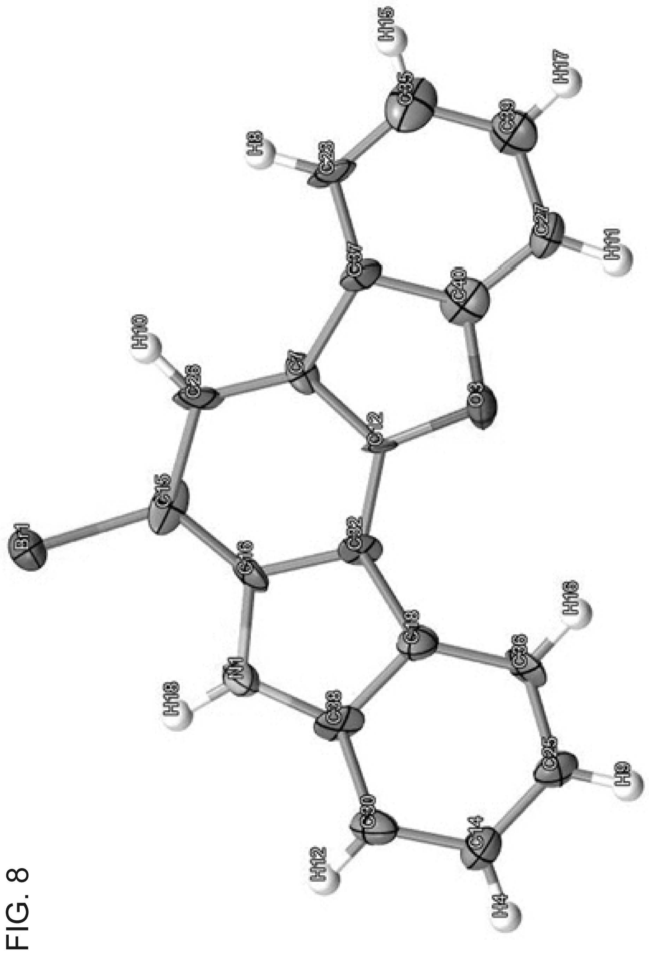

After 5H-benzofuro[3,2-c]carbazole (2.58 g) and dichloromethane (100 mL) were added and dissolved, N-bromosuccinimide (1.78 g) was added, and stirring was performed at room temperature for 20 hours. After extraction with dichloromethane, the resultant was concentrated. The concentrate was washed with ethanol and filtered to obtain 2.19 g of intermediate 7 as a white powder. Using chloroform and ethanol, the white powder was grown to obtain a single crystal (size: 0.3 mm × 0.06 mm × 0.06 mm). X-ray crystal structure analysis revealed that the position of bromination was the 6-position. Fig. 8 shows a crystal structure obtained by the single-crystal X-ray structure analysis. (Unit cell: a = 5.6801(8) Å, b = 15.309(2) Å, β = 96.405(7) o, c = 15.0889(19) Å, Z = 4, space group: P2₁)

### (2-2) Synthesis of Intermediate 8

Intermediate 7 (2.15 g), bis(pinacolato)diboron (1.79 g), a dichloromethane complex of [1,1'-bis(diphenylphosphino)ferrocene]dichloropalladium(II) (0.261 g), potassium acetate (1.88 g), and dioxane (80 mL) were added, and after Ar bubbling was performed, stirring was performed under nitrogen at 90°C for 12 hours. Water was added, and the mixture was extracted with dichloromethane. After the dichloromethane was distilled off, the resultant was washed with a small amount of methanol and heptane and filtered to obtain 1.6 g of intermediate 8.

### (2-3) Synthesis of Intermediate 9

Intermediate 8 (0.76 g), intermediate 5 (0.465 g), tetrakis triphenylphosphine palladium (0.031 g), potassium carbonate (0.521 g), dioxane (20 mL), and water (10 mL) were added, and after Ar bubbling was performed, stirring was performed under nitrogen at 90°C for 12 hours. The resultant was washed with methanol and filtered to obtain 0.368 g of intermediate 9 in crude form as a gray powder. Intermediate 9 was directly used in the subsequent reaction without further purification.

### (2-4) Synthesis of Compound A-7

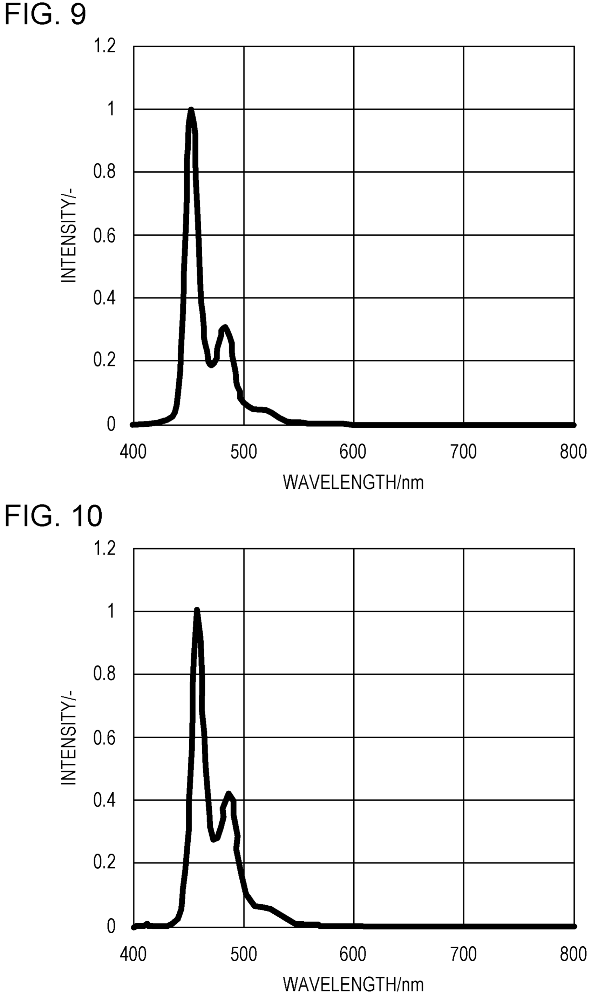

Intermediate 9 (0.368 g), 1,10-phenanthroline (0.069 g), copper iodide (0.066 g), potassium carbonate (0.321 g), and dimethylacetamide (20 mL) were added, and stirring was performed at 160°C for 3 hours. After filtration with methanol/water, the filter cake was repeatedly dispersed and washed with toluene three times, and vacuum drying was performed at 140°C. Thereafter, sublimation purification was performed to obtain 140 mg of compound A-7 as a yellow powder. THF was added to the yellow powder, and the resulting solution was subjected to mass spectrometry by UPLC (Ultra High Performance Liquid Chromatography)-MS for identification (m/z = 634.168, C₄₆H₃₂N₂O₂⁺). Fig. 9 shows a normalized emission spectrum of compound A-7 in a toluene solution.

### [Example 3]

### (3) Synthesis of Compound D-1

Compound D-1 was synthesized according to the following synthetic route.

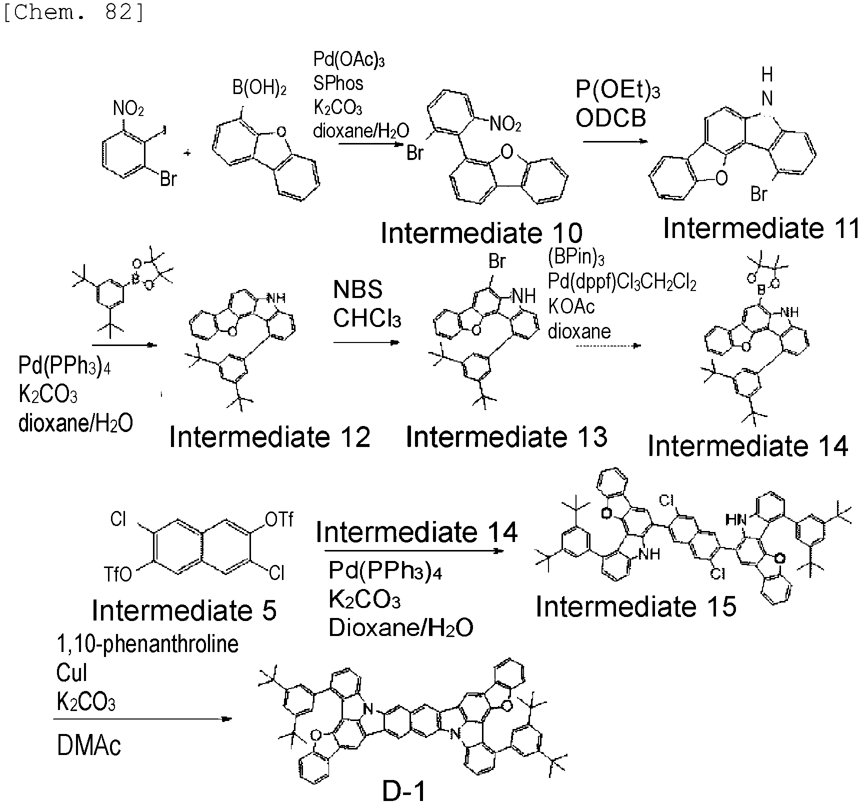

### (3-1) Synthesis of Intermediate 10

1-Bromo-2-iodo-3-nitrobenzene (8.89 g), dibenzo[b,d]furan-4-ylboronic acid (5.75 g), palladium acetate (0.122 g), dicyclohexyl(2',6'-dimethoxy-[1,1'-biphenyl]-2-yl)phosphine (0.443 g), potassium carbonate (11.2 g), dioxane (200 mL), and water (80 mL) were added, and stirring was performed at 80°C for 3 hours. After extraction with toluene, the resultant was concentrated. After the toluene was distilled off, silica gel chromatography was performed at chloroform:heptane = 1:2 to obtain 4.9 g of intermediate 10.

### (3-2) Synthesis of Intermediate 11

Intermediate 10 (1.5 g), triethyl phosphite (1.746 mL), and ortho-dichlorobenzene (80 mL) were added, and stirring was performed at 220°C for 72 hours. After the ortho-dichlorobenzene was distilled off, silica gel chromatography was performed at ethyl acetate:heptane = 1:4 to obtain 0.75 g of intermediate 10 as a white powder.

### (3-3) Synthesis of Intermediate 12

Intermediate 11 (0.75 g), 2-(3,5-di-tert-butylphenyl)-4,4,5,5-tetramethyl-1,3,2-dioxaborolane (0.78 g), triphenylphosphine palladium (55 mg), potassium carbonate (0.62 g), dioxane (80 mL), and water (40 ml) were added, and after Ar bubbling was performed, stirring was performed under nitrogen at 80°C for 8 hours. Water was added, and the mixture was extracted with toluene. After the toluene was distilled off, silica gel chromatography was performed at ethyl acetate:heptane = 1:3 to obtain 0.95 g of intermediate 12.

### (3-4) Synthesis of Intermediate 13

After intermediate 12 (0.92 g) and chloroform (80 mL) were added and dissolved, N-bromosuccinimide (0.374 g) was added, and stirring was performed at room temperature for 20 hours. After the chloroform was distilled off, silica gel chromatography was performed at ethyl acetate:heptane = 1:3 to obtain 0.76 g of intermediate 13 as a white powder.

### (3-5) Synthesis of Intermediate 14

Intermediate 13 (0.72 g), bis(pinacolato)diboron (0.453 g), a dichloromethane complex of [1,1'-bis(diphenylphosphino)ferrocene]dichloropalladium(II) (33 mg), potassium acetate (0.403 g), and dioxane (30 mL) were added, and after Ar bubbling was performed, stirring was performed under nitrogen at 90°C for 12 hours. Water was added, and the mixture was extracted with ethyl acetate. After the ethyl acetate was distilled off, silica gel chromatography was performed at ethyl acetate:heptane = 1:4 to obtain 0.351 g of intermediate 14.

### (3-6) Synthesis of Intermediate 15

Intermediate 14 (0.34 g), intermediate 5 (0.140 g), tetrakis triphenylphosphine palladium (16 mg), potassium carbonate (0.160 g), dioxane (20 mL), and water (10 mL) were added, and after Ar bubbling was performed, stirring was performed under nitrogen at 90°C for 12 hours. The resultant was washed with methanol and filtered to obtain 0.530 g of intermediate 15 in crude form, and silica gel chromatography was performed at ethyl acetate:heptane = 1:2 to obtain 0.271 g of intermediate 15.

### (3-7) Synthesis of Compound D-1

Intermediate 15 (0.271 g), 1,10-phenanthroline (0.054 g), copper iodide (0.051 g), potassium carbonate (0.240 g), and dimethylacetamide (10 mL) were added, and stirring was performed at 150°C for 10 hours. After filtration with water, chlorobenzene was added to the filter cake, and the mixture was melted by heating and suction-filtered through silica gel. After concentration, chlorobenzene was added again, the mixture was melted by heating and allowed to cool, and the precipitate was filtered. Vacuum drying was performed at 140°C to obtain 31 mg of compound D-1 as a yellow powder. Mass spectrometry by MALDI-MS was performed for identification (m/z = 1010.48, C₇₄H₆₂N₂O₂). Fig. 10 shows a normalized emission spectrum of compound D-1 in a toluene solution.

### [Example 4]

### (4) Synthesis of Compound D-2

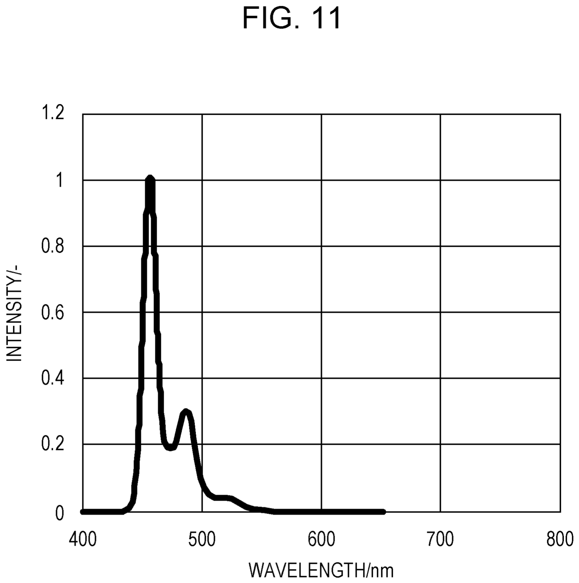

Compound D-2 was synthesized in the same manner as the synthesis of compound D-1 except that 2-([1,1': 3',1'': 3",1‴: 3‴,1ʺʺ-quinquephenyl]-5"-yl)-4,4,5,5-tetramethyl-1,3,2-dioxaborolane was used in place of 2-(3,5-di-tert-butylphenyl)-4,4,5,5-tetramethyl-1,3,2-dioxaborolane. Fig. 11 shows a normalized emission spectrum of compound D-2 in a toluene solution.

### [Example 5 (Fabrication and Evaluation of Organic Light-Emitting Element)]

An organic light-emitting element was fabricated in which an anode, a hole injection layer (HIL), a hole transport layer (HTL), an electron blocking layer (EBL), a light-emitting layer (EML), a hole blocking layer (HBL), an electron transport layer (ETL), and a cathode were sequentially formed on a substrate.

A glass substrate on which an ITO film as an anode was formed by sputtering so as to be 100 nm thick was used as a transparent conductive supporting substrate (ITO substrate). Next, the glass substrate having the ITO film was ultrasonically cleaned sequentially with acetone and isopropyl alcohol (IPA), cleaned by boiling in IPA, and then dried. Next, the glass substrate was subjected to UV/ozone cleaning. The glass substrate thus treated was used as a transparent conductive supporting substrate.

Next, a hole injection layer, a hole transport layer, an electron blocking layer, a light-emitting layer, a hole blocking layer, an electron transport layer, and metal electrode layers were continuously formed by performing vacuum deposition by resistance heating in a vacuum chamber at 10⁻⁵ Pa such that the electrode area was 3 mm². The layer structure is as shown below.

### [Table 6]

**Table 6**

| | Material | | | Thickness (nm) |
|---|---|---|---|---|
| Cathode | Metal electrode layer 2 | Al | | 100 |
| | Metal electrode layer 1 | Liq | | 0.5 |
| ETL | ET2 | | | 10 |
| HBL | ET12 | | | 20 |
| EML | Host | EM4 | Weight ratio A-6:EM4 = 1:99 | 25 |
| | Guest | A-6 | | |
| EBL | HT3 | | | 10 |
| HTL | HT2 | | | 15 |
| HIL | HT16 | | | 5 |

Next, the organic light-emitting element was covered with a protective glass plate in a dry air atmosphere and sealed with an acrylic resin adhesive to prevent the element from deteriorating due to moisture adsorption. In this manner, the organic light-emitting element was obtained.

For the obtained organic light-emitting element, the properties of the organic light-emitting element were measured and evaluated. The external quantum yield (EQE) of the organic light-emitting element at a current density of 10 mA/cm² was 7.1%.

Furthermore, a continuous operation test was performed at a current density of 20 mA/cm² to determine the time taken for the luminance to decrease by 20% from the initial luminance (LT80). When the time taken for the luminance to decrease by 20% in Comparative Example 1 was taken as 1.0, the relative value of LT80 in this Example was 2.24.

For the measurement apparatuses in this Example, specifically, the current-voltage characteristics were measured with a DC voltage and current source/monitor 6253 manufactured by ADC Corporation, and the emission luminance was measured with a spectroradiometer SR-LEDW manufactured by Topcon Corporation.

### [Example 6, Comparative Example 1 (Fabrication and Evaluation of Organic Light-Emitting Element)]

In Example 6, an organic light-emitting element was fabricated in the same manner as in Example 5 except that compound A-6 was changed to compound A-7. In Comparative Example 1, an organic light-emitting element was fabricated in the same manner as in Example 3 except that compound A-6 was changed to compound Ref-1. For the fabricated organic light-emitting elements, the properties of the organic light-emitting elements were measured and evaluated in the same manner as in Example 3. The results are shown in Table 7.

### [Table 7]

**Table 7**

| | Compound | EQE/% | LT80 (Relative value) |
|---|---|---|---|
| Example 5 | A-6 | 7.1 | 2.24 |
| Example 6 | A-7 | 6.1 | 1.45 |
| Comparative Example 1 | Ref-1 | 5.8 | 1.00 |

Table 7 shows that the relative values of LT80 of the organic light-emitting elements of Example 5 and Example 6 were 2.24 and 1.45, respectively. Therefore, the organic light-emitting elements including the organic compound according to the present invention have high durability. The EQE of the organic light-emitting element of Comparative Example 1 was 5.8%, whereas the EQEs of the organic light-emitting elements of Example 5 and Example 6 were 7.1% and 6.1%, respectively. Therefore, it was demonstrated that the organic light-emitting elements including the organic compound according to the present invention also have high luminous efficiency.

It follows from the foregoing that the organic compound according to the present invention is an organic compound having high thermal stability. The organic compound according to an embodiment of the present invention is an organic compound that exhibits particularly high oscillator strength. Therefore, the organic light-emitting element including the organic compound according to the present invention is an organic light-emitting element having high luminous efficiency. Furthermore, the organic light-emitting element including the organic compound according to the present invention is an organic light-emitting element having high element durability.

The present invention may also have the following configurations.

### (Configuration 1)

An organic compound represented by general formula (1-1) or (1-2).

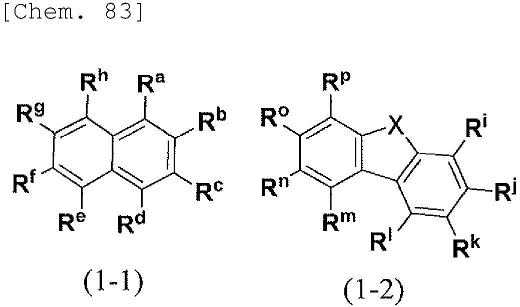

In general formula (1-1), at least four of bonding sites R^{a} to R^{h} are bonded to a skeleton selected from Structure A group. Among the bonding sites R^{a} to R^{h}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group.

In general formula (1-2), at least four of bonding sites Rⁱ to R^{p} are bonded to a skeleton selected from Structure A group. Among the bonding sites Rⁱ to R^{p}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

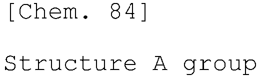

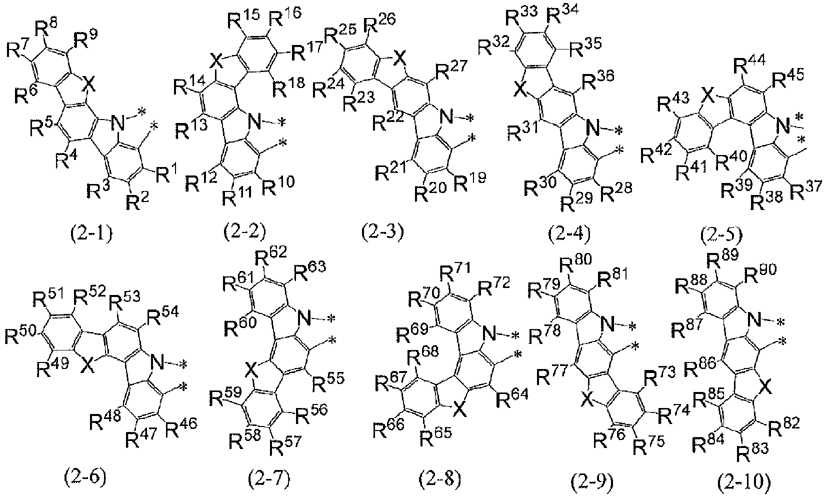

In general formulas (2-1) to (2-10), R¹ to R⁹⁰ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group. Bonding positions * represent positions of bonding to the bonding sites R^{a} to R^{p}.

### (Configuration 2)

The organic compound according to configuration 1, wherein in general formula (1-1), four of the bonding sites R^{a} to R^{h} are bonded to the skeleton selected from Structure A group, and in general formula (1-2), four of the bonding sites Rⁱ to R^{p} are bonded to the skeleton selected from Structure A group.

### (Configuration 3)

The organic compound according to configuration 1 or 2, wherein in the organic compound represented by general formula (1-1), the bonding sites R^{d}, R^{c}, R^{f}, and R^{g} are bonded to the skeleton selected from Structure A group.

### (Configuration 4)

The organic compound according to any one of configurations 1 to 3, wherein the organic compound represented by general formula (1-1) is bonded to general formula (2-1), (2-2), (2-4), (2-5), (2-6), (2-7), (2-8), (2-9), or (2-10).

### (Configuration 5)

The organic compound according to any one of configurations 1 to 4, wherein the organic compound represented by general formula (1-1) is bonded to general formula (2-1), (2-5), (2-6), (2-7), or (2-8).

### (Configuration 6)

The organic compound according to any one of configurations 1 to 5, wherein the organic compound represented by general formula (1-1) is represented by general formula (3-6) or (3-7).

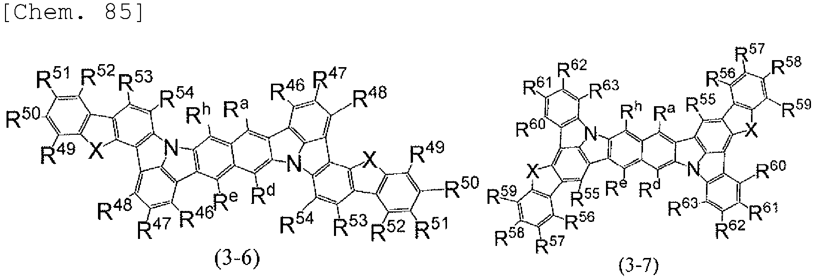

In general formulas (3-6) and (3-7), bonding sites R^{a}, R^{d}, R^{e}, and R^{h} are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. R⁴⁶ to R⁶³ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group.

X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

### (Configuration 7)

The organic compound according to configuration 6, wherein in general formulas (3-6) and (3-7), R⁴⁶ to R⁶³ are each independently selected from the group consisting of a hydrogen atom, a substituted or unsubstituted alkyl group having 1 to 20 carbon atoms, a substituted or unsubstituted aryl group having 6 to 20 carbon atoms, and a substituted or unsubstituted heterocyclic group having 3 to 24 carbon atoms.

### (Configuration 8)

The organic compound according to configuration 7, wherein in general formulas (3-6) and (3-7), R⁴⁶ to R⁶³ are hydrogen atoms.

### (Configuration 9)

The organic compound according to configuration 1 or 2, wherein in the organic compound represented by general formula (1-2), the bonding sites R^{j}, R^{k}, Rⁿ, and R^{o} are bonded to the skeleton selected from Structure A group.

### (Configuration 10)

The organic compound according to any one of configurations 1, 2, and 9, wherein the organic compound represented by general formula (1-2) is bonded to general formula (2-6).

### (Configuration 11)

The organic compound according to any one of configurations 1, 2, 9, and 10, wherein the organic compound represented by general formula (1-2) is represented by general formula (4-2).

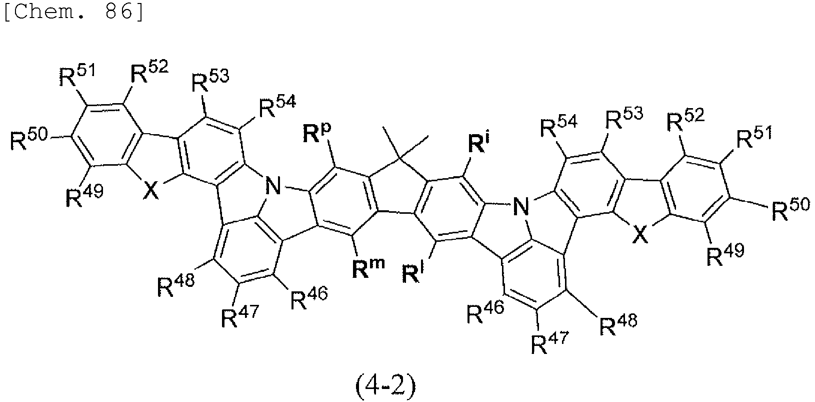

In general formula (4-2), bonding sites Rⁱ, R^{l}, R^{m}, and R^{p} are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. R⁴⁶ to R⁵⁴ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group. X is an oxygen atom, a sulfur atom, NR⁹¹, CR⁹²R⁹³, or SiR⁹⁴R⁹⁵. R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

### (Configuration 12)

The organic compound according to configuration 11, wherein in general formula (4-2), R⁴⁶ to R⁵⁴ are each independently selected from the group consisting of a substituted or unsubstituted alkyl group having 1 to 20 carbon atoms, a substituted or unsubstituted aryl group having 6 to 20 carbon atoms, and a substituted or unsubstituted heterocyclic group having 3 to 24 carbon atoms.

### (Configuration 13)

The organic compound according to configuration 11 or 12, wherein in general formula (4-2), R⁴⁶ to R⁵⁴ are hydrogen atoms.

### (Configuration 14)

An organic light-emitting element including:
a first electrode and a second electrode; and
an organic compound layer disposed between the first electrode and the second electrode,
wherein the organic compound layer contains the organic compound according to any one of configurations 1 to 13.

### (Configuration 15)

The organic light-emitting element according to configuration 14, wherein the organic compound layer includes a light-emitting layer, and
the light-emitting layer contains the organic compound.

### (Configuration 16)

The organic light-emitting element according to configuration 15, wherein the light-emitting layer further contains a first compound, and
a lowest excited singlet energy of the first compound is higher than a lowest excited singlet energy of the organic compound.

### (Configuration 17)

The organic light-emitting element according to configuration 16, wherein the first compound includes a fused polycyclic hydrocarbon compound.

### (Configuration 18)

The organic light-emitting element according to configuration 16 or 17, wherein the light-emitting layer further contains a second compound, and
a lowest excited singlet energy of the second compound is higher than the lowest excited singlet energy of the organic compound and lower than the lowest excited singlet energy of the first compound.

### (Configuration 19)

A display apparatus including a plurality of pixels, wherein at least one of the plurality of pixels includes the organic light-emitting element according to any one of configurations 14 to 18 and a transistor connected to the organic light-emitting element.

### (Configuration 20)

A photoelectric conversion apparatus including: an image pickup element configured to receive light; and a display unit configured to display an image captured by the image pickup element,
wherein the display unit includes the organic light-emitting element according to any one of configurations 14 to 18.

### (Configuration 21)

An image display apparatus including: a display unit including the organic light-emitting element according to any one of configurations 14 to 18; and a housing provided with the display unit.

### (Configuration 22)

An electronic apparatus including: a display unit including the organic light-emitting element according to any one of configurations 14 to 18; a housing provided with the display unit; and a communication unit provided in the housing and configured to communicate with an external unit.

### (Configuration 23)

A wearable device including: a display unit including the organic light-emitting element according to any one of configurations 14 to 18; an optical system configured to collect light of the display unit; and a controller configured to control display of the display unit.

### (Configuration 24)

A lighting apparatus including: a light source including the organic light-emitting element according to any one of configurations 14 to 18; and a housing provided with the light source.

### (Configuration 25)

A moving object including: a lighting fixture including the organic light-emitting element according to any one of configurations 14 to 18; and a body provided with the lighting fixture.

### (Configuration 26)

An image-forming apparatus including: a photoreceptor; and an exposure light source configured to expose the photoreceptor,
wherein the exposure light source includes the organic light-emitting element according to any one of configurations 14 to 18.

The present invention is not limited to the above embodiments, and various alterations and modifications can be made without departing from the spirit and scope of the present invention. Accordingly, the following claims are attached to make public the scope of the present invention.

This application claims priority to Japanese Patent Application No. 2023-112256 filed July 7, 2023 and Japanese Patent Application No. 2024-083867 filed May 23, 2024, which are hereby incorporated by reference herein in their entirety.

### Reference Signs List

- 1: interlayer insulating layer
- 2: reflective electrode
- 3: insulating layer
- 4: organic compound layer
- 5: transparent electrode
- 6: protective layer
- 7: color filter
- 10: subpixel
- 11: substrate
- 12: insulating layer
- 13: gate electrode
- 14: gate insulating film
- 15: semiconductor layer
- 16: drain electrode
- 17: source electrode
- 18: thin-film transistor
- 19: insulating film
- 20: contact hole
- 21: lower electrode
- 22: organic compound layer
- 23: upper electrode
- 24: first protective layer
- 25: second protective layer
- 26: organic light-emitting element
- 27: photoreceptor
- 28: exposure light source
- 29: light
- 30: charging unit
- 31: developing unit
- 32: transfer unit
- 33: conveying unit
- 34: recording medium
- 35: fixing unit
- 36: light-emitting portion
- 37: first direction parallel to major axis of photoreceptor
- 40: image-forming apparatus
- 100: display apparatus
- 1000: display apparatus
- 1001: upper cover
- 1002: flexible printed circuit
- 1003: touch panel
- 1004: flexible printed circuit
- 1005: display panel
- 1006: frame
- 1007: circuit board
- 1008: battery
- 1009: lower cover
- 1100: image pickup apparatus
- 1101: viewfinder
- 1102: rear display
- 1103: operation unit
- 1104: housing
- 1200: electronic apparatus
- 1201: display unit
- 1202: operation unit
- 1203: housing
- 1300: display apparatus
- 1301: frame
- 1302: display unit
- 1303: base
- 1310: display apparatus
- 1311: first display unit
- 1312: second display unit
- 1313: housing
- 1314: bending point
- 1400: lighting apparatus
- 1401: housing
- 1402: light source
- 1403: circuit board
- 1404: optical film
- 1405: light diffusion unit
- 1500: automobile
- 1501: tail lamp
- 1502: window
- 1503: car body
- 1600: smart glasses
- 1601: lens
- 1602: image pickup apparatus
- 1603: controller
- 1610: smart glasses
- 1611: lens
- 1612: controller

## Claims

1. An organic compound represented by general formula (1-1) or (1-2): 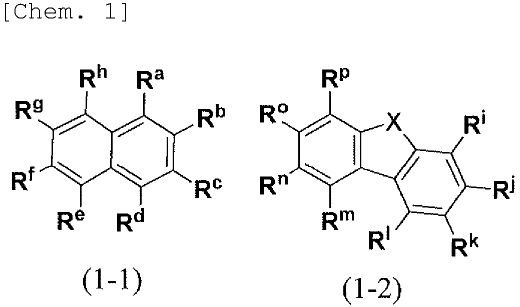
wherein in general formula (1-1), at least four of bonding sites R^{a} to R^{h} are bonded to a skeleton selected from Structure A group; and among the bonding sites R^{a} to R^{h}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group,
in general formula (1-2), at least four of bonding sites Rⁱ to R^{p} are bonded to a skeleton selected from Structure A group; among the bonding sites Rⁱ to R^{p}, bonding sites not bonded to Structure A group are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group; and X is an oxygen atom, a sulfur atom, NR⁹¹, CR⁹²R⁹³, or SiR⁹⁴R⁹⁵, where R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group, 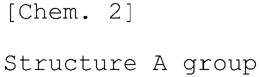 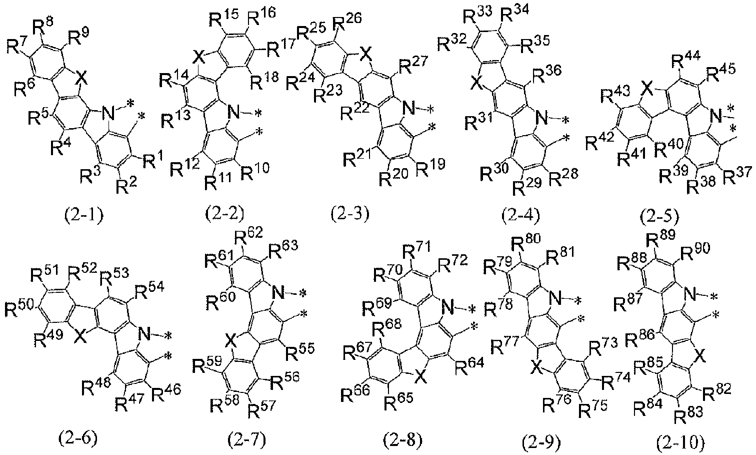
in general formulas (2-1) to (2-10), R¹ to R⁹⁰ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group; X is an oxygen atom, a sulfur atom, NR⁹¹, CR⁹²R⁹³, or SiR⁹⁴R⁹⁵, where R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group; and bonding positions * represent positions of bonding to the bonding sites R^{a} to R^{p}.

2. The organic compound according to claim 1, wherein in general formula (1-1), four of the bonding sites R^{a} to R^{h} are bonded to the skeleton selected from Structure A group, and in general formula (1-2), four of the bonding sites Rⁱ to R^{p} are bonded to the skeleton selected from Structure A group.

3. The organic compound according to claim 1, wherein in the organic compound represented by general formula (1-1), the bonding sites R^{d}, R^{c}, R^{f}, and R^{g} are bonded to the skeleton selected from Structure A group.

4. The organic compound according to claim 1, wherein the organic compound represented by general formula (1-1) is bonded to general formula (2-1), (2-2), (2-4), (2-5), (2-6), (2-7), (2-8), (2-9), or (2-10).

5. The organic compound according to claim 1, wherein the organic compound represented by general formula (1-1) is bonded to general formula (2-1), (2-5), (2-6), (2-7), or (2-8).

6. The organic compound according to claim 1, wherein the organic compound represented by general formula (1-1) is represented by general formula (3-6) or (3-7): 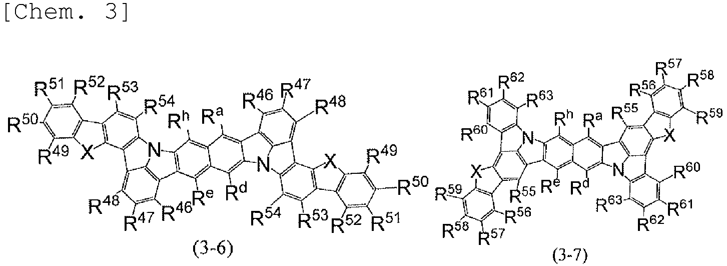
in general formulas (3-6) and (3-7), bonding sites R^{a}, R^{d}, R^{e}, and R^{h} are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group; R⁴⁶ to R⁶³ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group; and
X is an oxygen atom, a sulfur atom, NR⁹¹, CR⁹²R⁹³, or SiR⁹⁴R⁹⁵, where R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

7. The organic compound according to claim 6, wherein in general formulas (3-6) and (3-7), R⁴⁶ to R⁶³ are each independently selected from the group consisting of a hydrogen atom, a substituted or unsubstituted alkyl group having 1 to 20 carbon atoms, a substituted or unsubstituted aryl group having 6 to 20 carbon atoms, and a substituted or unsubstituted heterocyclic group having 3 to 24 carbon atoms.

8. The organic compound according to claim 7, wherein in general formulas (3-6) and (3-7), R⁴⁶ to R⁶³ are hydrogen atoms.

9. The organic compound according to claim 1, wherein in the organic compound represented by general formula (1-2), the bonding sites R^{j}, R^{k}, Rⁿ, and R^{o} are bonded to the skeleton selected from Structure A group.

10. The organic compound according to claim 1, wherein the organic compound represented by general formula (1-2) is bonded to general formula (2-6).

11. The organic compound according to claim 1, wherein the organic compound represented by general formula (1-2) is represented by general formula (4-2): 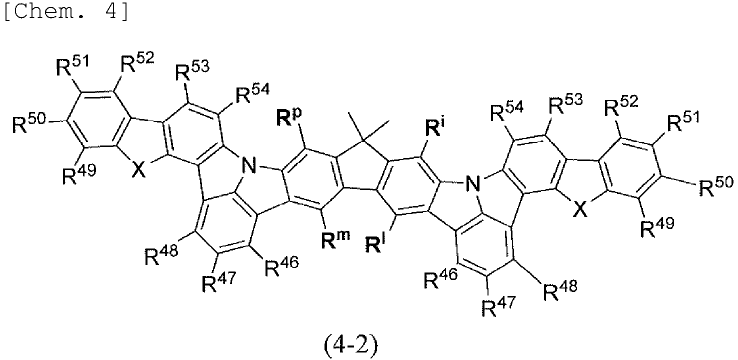 in general formula (4-2), bonding sites Rⁱ, R^{l}, R^{m}, and R^{p} are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group; R⁴⁶ to R⁵⁴ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, and a cyano group; and X is an oxygen atom, a sulfur atom, NR⁹¹, CR⁹²R⁹³, or SiR⁹⁴R⁹⁵, where R⁹¹ to R⁹⁵ are each independently selected from the group consisting of a hydrogen atom, a deuterium atom, a substituted or unsubstituted alkyl group, a substituted or unsubstituted aryl group, and a substituted or unsubstituted heterocyclic group.

12. The organic compound according to claim 11, wherein in general formula (4-2), R⁴⁶ to R⁵⁴ are each independently selected from the group consisting of a substituted or unsubstituted alkyl group having 1 to 20 carbon atoms, a substituted or unsubstituted aryl group having 6 to 20 carbon atoms, and a substituted or unsubstituted heterocyclic group having 3 to 24 carbon atoms.

13. The organic compound according to claim 11, wherein in general formula (4-2), R⁴⁶ to R⁵⁴ are hydrogen atoms.

14. An organic light-emitting element comprising:
a first electrode and a second electrode; and
an organic compound layer disposed between the first electrode and the second electrode,
wherein the organic compound layer contains the organic compound according to claim 1.

15. The organic light-emitting element according to claim 14, wherein the organic compound layer includes a light-emitting layer, and
the light-emitting layer contains the organic compound.

16. The organic light-emitting element according to claim 15, wherein the light-emitting layer further contains a first compound, and
a lowest excited singlet energy of the first compound is higher than a lowest excited singlet energy of the organic compound.

17. The organic light-emitting element according to claim 16, wherein the first compound includes a fused polycyclic hydrocarbon compound.

18. The organic light-emitting element according to claim 16, wherein the light-emitting layer further contains a second compound, and
a lowest excited singlet energy of the second compound is higher than the lowest excited singlet energy of the organic compound and lower than the lowest excited singlet energy of the first compound.

19. A display apparatus comprising a plurality of pixels, wherein at least one of the plurality of pixels includes the organic light-emitting element according to any one of claims 14 to 18 and a transistor connected to the organic light-emitting element.

20. A photoelectric conversion apparatus comprising: an image pickup element configured to receive light; and a display unit configured to display an image captured by the image pickup element,
wherein the display unit includes the organic light-emitting element according to any one of claims 14 to 18.

21. An image display apparatus comprising: a display unit including the organic light-emitting element according to any one of claims 14 to 18; and a housing provided with the display unit.

22. An electronic apparatus comprising: a display unit including the organic light-emitting element according to any one of claims 14 to 18; a housing provided with the display unit; and a communication unit provided in the housing and configured to communicate with an external unit.

23. A wearable device comprising: a display unit including the organic light-emitting element according to any one of claims 14 to 18; an optical system configured to collect light of the display unit; and a controller configured to control display of the display unit.

24. A lighting apparatus comprising: a light source including the organic light-emitting element according to any one of claims 14 to 18; and a housing provided with the light source.

25. A moving object comprising: a lighting fixture including the organic light-emitting element according to any one of claims 14 to 18; and a body provided with the lighting fixture.

26. An image-forming apparatus comprising: a photoreceptor; and an exposure light source configured to expose the photoreceptor,
wherein the exposure light source includes the organic light-emitting element according to any one of claims 14 to 18.
